(19)

Europäisches
Patentamt
European
Patent Office
Office européen
des brevets

(11) **EP 2 472 262 A2**

(12) **EUROPEAN PATENT APPLICATION**

(43) Date of publication:
**04.07.2012 Bulletin 2012/27**

(21) Application number: **12160346.8**

(22) Date of filing: **10.07.2008**

(51) Int Cl.:
*G01N 33/566* (2006.01)          *G01N 33/574* (2006.01)
*C12N 15/85* (2006.01)          *A61K 31/00* (2006.01)
*G01N 33/68* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **10.07.2007 US 959023 P**

(62) Document number(s) of the earlier application(s) in accordance with Art. 76 EPC:
**08781606.2 / 2 244 700**

(71) Applicant: **Immune Disease Institute, Inc.**
**Boston, MA 02115 (US)**

(72) Inventors:
• **Oh-Hora, Masatsugu**
**Boston, MA 02115 (US)**

• **Hogan, Patrick**
**Cambridge, MA 02140 (US)**
• **Feske, Stefan**
**New York, NY 10016 (US)**
• **Rao, Anjana**
**Cambridge, MA 02140 (US)**

(74) Representative: **Purdy, Hugh Barry**
**PurdyLucey**
**Intellectual Property**
**138-139 The Capel Building**
**Mary's Abbey**
**D7 Dublin (IE)**

Remarks:
This application was filed on 20-03-2012 as a divisional application to the application mentioned under INID code 62.

(54)     **Stromal interacting molecule knockout mouse and uses thereof**

(57)     This invention relates to knockout mice for the $Ca^{2+}$ sensor membrane protein STIM-1, STIM-2, or both, as well as cell lines from these knockout mice. Provided herein are various methods of use of isolated with knockout STIM-1 and/or STIM-2.

FIG. 2A

**EP 2 472 262 A2**

**Description**

**CROSS REFERENCE TO RELATED APPLICATION**

This application claims benefit under 35 U.S.C. § 119(e) of the U.S. provisional applications No. 60/959,023 filed July 10, 2007, the contents of which are herein incorporated by reference in its entirety.

**GOVERNMENT SUPPORT**

This invention was made with Government support under Grant Nos.: GM075256 (NIH/NIGMS), AI40127 (NIH/NIAID) and R01AI066128 (NIH/NIAID) awarded by the National Institutes of Health. The Government has certain rights in the invention.

**BACKGROUND OF INVENTION**

Inflammation is a general term for the local accumulation of fluid, plasma proteins, and white blood cells that is initiated when a group of cells or an organism is put under stress, by physical injury such as DNA damages, infection, or a local immune response. This is also known as an inflammatory response. The immune cells that invade tissues undergoing inflammatory responses are often called inflammatory cells or an inflammatory infiltrate and help cells or organisms to improve their conditions as a response to the stress. Inflammation can lead to death of cells in the organ or affected tissue. Inflammation is part of the normal immune response or the defense system in the body.
The activation, proliferation, and the differentiation of immune cells and the consequent inflammatory response are highly regulated in the body. The inflammatory response is elicited upon exposure to foreign materials such as pathogens and pathogen-derived compounds and is initiated and is sustained by cytokines (Carol, A., et. al., 1997, Frontiers in BioSciences, 2: 12-26). The cytokines involved include interleukin-1 (Il-1), Il-2, Il-3, Il-4, Il-5, Il-6, Il-10, Il-12, Il-13, IFN-y, TNF, TGF, lymphotoxin, and histamine. The inflammatory response should not be elicited by host- derived materials or nor should it be sustained by aberrant cytokine production. However, deregulation of inflammation can occur, provoking inflammatory diseases. Inflammation entails four well-known symptoms, including redness, heat, tenderness/pain, and swelling that characterize so many common diseases and conditions. Chronic inflammatory diseases, such as rheumatoid arthritis, inflammatory bowel disease, systemic lupus erythematosus, multiple sclerosis, and type 1 diabetes, affect almost half a billion of people. Many of these diseases are debilitating and are becoming increasingly common in our aging society.
Stimulation of immune cells causes depletion of $Ca^{2+}$ from endoplasmic reticulum (ER) stores, thereby triggering sustained $Ca^{2+}$ entry through store-operated $Ca^{2+}$ release-activated $Ca^{2+}$ (CRAC) channels, an essential signal for lymphocyte activation and proliferation. Recent evidence indicates that activation of CRAC current is initiated by STIM proteins, which sense ER $Ca^{2+}$ levels through an EF-hand located in the ER lumen and relocalize upon store depletion into puncta closely associated with the plasma membrane. The Drosophila Orai and human Orai1 (also called TMEM142A) are critical components of store-operated $Ca^{2+}$ entry downstream of STIM. Combined over-expression of Orai and Stim in Drosophila cells, or Orai1 and STIM1 in mammalian cells, leads to a marked increase in CRAC current.
Human and murine STIM1 were originally discovered as candidate growth regulators in tumors and in the bone marrow stroma, and the structurally related vertebrate family members, STIM 2 and the Drosophila homologue D-Stim, were subsequently identified. STIM proteins are ubiquitously expressed type I single-pass transmembrane proteins which have a unique combination of structural motifs within their polypeptide sequences. The extracellular regions contain an N-terminal unpaired EF-hand $Ca^{(2+)}$ binding motif adjacent to an unconventional glycosylated SAM domain, while the cytoplasmic regions contain alpha-helical coiled-coil domains within a region having homology to ERM domains adjacent to the transmembrane region, and phosphorylated proline-rich domains near the C-terminus. STIM1, STIM2 and D-Stim diverge significantly only in their structure C-terminal to the coiled-coil/ERM domains. The STIM structural domains were predicted to function in $Ca^{(2+)}$ binding as well as in mediating interactions between STIM proteins and other proteins, and homotypic STIM1-STIM1 and heterotypic STIM1-STIM2 interactions were demonstrated biochemically. However, the functional significance of the cellular localization of STIM1 and its domain structure only became evident after recent breakthrough research identified STIM1 as a key regulator of store-operated calcium (SOC) entry into cells. It is now clear that STIM1 is both a sensor of $Ca^{(2+)}$ depletion in the endoplasmic reticulum (ER) lumen and an activator of Orail-containing SOC channels in the plasma membrane.
Since cytokine production is crucial to maintaining an inflammatory response in chronic inflammation and the influx of $Ca^{2+}$ via the SOC channels is required to sustain the cytokine production in activated immune cells, discovery and development of new methods to control and modulate the $Ca^{2+}$ influxes into an immune cell and other cells in the body can lead to therapeutics for inflammatory, autoimmune, and $Ca^{2+}$ homeostasis-related diseases and disorders. Currently, there is still a need for tools that aid in such discoveries and development.

## SUMMARY OF THE INVENTION

Embodied in the invention is a transgenic mouse whose genome comprises a homozygous disruption of a STIM gene, the homozygous mouse exhibiting non-functional STIM protein.

Embodied in the invention is also a transgenic mouse whose genome comprises a heterozygous disruption of a STIM gene, wherein the mouse, by crossing with another transgenic mouse produces a mouse whose genome comprises a homozygous disruption of a STIM gene, the homozygous mouse exhibiting non-functional STIM protein.

The disrupted STIM gene can be STIM 1, STIM 2, or both STIM 1 and STIM 2. The STIM gene disruption can be constitutive or conditional where the disruption only occurs in thymocytes after normal development and during the double-positive (CD4$^+$ CD8$^+$) stage. Hence the invention provides a transgenic mouse whose genome comprises a homozygous disruption of a STIM gene in the CD4$^+$ CD8$^+$ thymocytes and developed T lymphocytes.

Encompassed in the invention are tissues, isolated cells, and cell lines derived from transgenic mice whose genome comprises a homozygous disruption of a STIM gene (ie. STIM1, STIM 2, or both STIM 1 and STIM 2). Alternately, tissues, isolated cells, and cell lines are derived from transgenic mice where the thymocytes' genome comprises a homozygous disruption of a STIM gene (ie. STIM 1, STIM 2, or both STIM 1 and STIM 2).

In one embodiment, the invention provides tissues, isolated cells, and cell lines derived from transgenic mice whose genome comprises a heterozygous disruption of a STIM gene (ie. STIM 1, STIM 2, or both STIM 1 and STIM 2).

In one embodiment, the isolated cells are T lymphocytes and mouse embryonic fibroblasts. Immortalized T lymphocytes and mouse embryonic fibroblasts cell lines that are STIM 1$^{-/-}$, STIM 2$^{-/-}$, or both STIM 1$^{-/-}$ and STIM 2$^{-/-}$ are provided.

In one embodiment, the invention provides a non-human transgenic animal having a cell type-specific conditionally targeted allele of Stim1 and/or Stim2. The non-human transgenic animal is a mouse. The cell type having conditionally targeted allele of Stim1 and/or Stim2 can be a T cell, a regulatory T cell, a neuronal cell, or an embryonic fibroblast cell. The conditionally targeted allele of Stim1 and/or Stim2 are conditionally deleted.

In one embodiment, provided herein are isolated cells derived from the non-human transgenic animal of having a cell type-specific conditionally targeted allele of Stim1 and/or Stim2, wherein the cell can be a T cell, a regulatory T cell, a neuronal cell, or an embryonic fibroblast cell, and the conditionally targeted allele of Stim1 and/or Stim2 are conditionally deleted.

In one embodiment, provided herein is a method for inhibiting Ca$^{2+}$-mediated cytokine expression in a cell without producing a profound reduction in store-operated Ca$^{2+}$ entry in the cell comprising contacting the cell with a selective Stim2 inhibitor in an amount effective to inhibit Ca$^{2+}$-mediated cytokine expression in the cell. The cell can be a lymphocyte, a T-cell or a regulatory T cell. The selective Stim2 inhibitor selectively inhibits Stim2 relative to Stim1. The cytokine is selected from IL-2, IL-4 and IFN-gamma.

In one embodiment, provided herein is a method for inhibiting Ca$^{2+}$-mediated cytokine expression in a cell and producing a profound reduction in store-operated Ca$^{2+}$ entry in the cell comprising contacting the cell with a selective Stim1 inhibitor in an amount effective to inhibit Ca$^{2+}$-mediated cytokine expression in a cell and produces a profound reduction in store-operated Ca$^{2+}$ entry in the cell. The cell can be a lymphocyte, a T-cell or a regulatory T cell. The selective Stim1 inhibitor selectively inhibits Stim1 relative to Stim2. The cytokine is selected from IL-2, IL-4 and IFN-gamma.

In one embodiment, provided herein is a method for inhibiting Ca$^{2+}$-mediated cytokine expression in a cell and producing a profound reduction in store-operated Ca$^{2+}$ entry in the cell comprising contacting the cell with a an inhibitor of Stim1 and an inhibitor of Stim2, wherein the amount of the inhibitors is effective to inhibit Ca$^{2+}$-mediated cytokine expression in the cell. The cell can be a lymphocyte, a T-cell or a regulatory T cell. The regulatory T cell is in a subject with a tumor and the amount of the inhibitors is effective for increasing an immune response against the tumor. The inhibitor of Stim1 and the inhibitor of Stim2 have the same chemical structure. The cytokine is selected from IL-2, IL-4 and IFN-gamma.

In one embodiment, provided herein is a method of identifying a test agent that inhibits Ca$^{2+}$-mediated cytokine expression in a cell without producing a profound reduction in store-operated Ca$^{2+}$ entry in the cell, comprising: (a) contacting at least one test agent with a recombinant cell that comprises a heterologous nucleic acid encoding a STIM2 protein or a functional fragment thereof, wherein the heterologous STIM2 protein or the functional fragment thereof comprises an amino acid sequence at least 80% identical to a human STIM2 protein; (b) measuring Ca$^{2+}$-mediated cytokine expression in the cell; and (c) measuring changes in ion fluxes or electrical current or membrane potential across the cell membrane, detecting changes in a fluorescence signal from the cell, detecting changes in a luminescence signal from the cell, or measuring changes in membrane potential of the cell.

In one embodiment, provided herein is a method of identifying a test agent that increases an immune response against a tumor in a subject, comprising: (a) contacting at least one test agent with a recombinant cell that comprises a heterologous nucleic acid encoding a STIM1 protein or a functional fragment thereof, and a STIM2 protein or a functional fragment thereof, wherein the heterologous STIM1 protein or functional fragment thereof comprises an amino acid

EP 2 472 262 A2

sequence at least 80% identical to a human STIM1 protein and the heterologous STIM2 protein or the functional fragment thereof comprises an amino acid sequence at least 80% identical to a human STIM2 protein; and measuring changes in ion fluxes or electrical current or membrane potential across the cell membrane, detecting changes in a fluorescence signal from the cell, detecting changes in a luminescence signal from the cell, or measuring changes in membrane potential of the cell; (b) contacting the test agent with an isolated form of the heterologous STIM1 protein; and measuring the binding of the test agent to the isolated heterologous STIM1 protein; and (c) contacting the test agent with an isolated form of the heterologous STIM2 protein; and measuring the binding of the test agent to the isolated heterologous STIM2 protein.

In one embodiment, the invention provides a method for screening for agents that modulate intracellular calcium fluxes comprising the use of a cell that is deficient in STIM protein. The agent can modulate the intracellular calcium fluxes by a mechanism that does not involves a STIM protein (ie. non STIM-dependent pathway or mechanism) or the agent can modulate the intracellular calcium fluxes by a mechanism that involves a STIM protein, thus involving a STIM-dependent pathway. The Stim-deficient cell used can be Stim1$^{-/-}$, Stim2$^{-/-}$, or both Stim1$^{-/-}$ Stim2$^{-/-}$.

In one embodiment, the invention provides a method for evaluating the mode of action of an agent that modulate intracellular calcium fluxes comprising the use of a cell that is deficient in a Stim protein. The agent can modulate the intracellular calcium fluxes by a mechanism that does not involves a STIM protein (ie. non STIM-dependent pathway or mechanism) or the agent can modulate the intracellular calcium fluxes by a mechanism that involves a STIM protein, thus involving a STIM-dependent pathway. The Stim-deficient cell used can be Stim1$^{-/-}$, Stim2$^{-/-}$, or both Stim1$^{-/-}$ Stim2$^{-/-}$.

In one embodiment, the invention provides a method for studying the cellular functions of a STIM protein or a mutant STIM protein in the absence of any other STIM homologues comprising the use of a cell that is deficient in both Stim1 and Stim 2.

In on embodiment, the invention provides a method of studying the effects and/or efficacy of a STIM inhibitor or a STIM modulator comprising the use of a cell that is deficient in a STIM protein. The Stim-deficient cell used can be Stim1$^{-/-}$, Stim2$^{-/-}$, or both Stim1$^{-/-}$ Stim2$^{-/-}$.

In one embodiment, the invention provides a method for screening a STIM inhibitor for side effects or toxicity resulting from the inhibitor's action on a target(s) other than STIM comprising the use of a transgenic mouse whose genome comprises a heterozygous or homozygous disruption of a STIM gene (ie. STIM 1, STIM 2, or both STIM 1 and STIM 2) or the use of cells that is deficient in a STIM protein. The Stim-deficient cell used can be Stim1$^{-/-}$, Stim2$^{-/-}$, or both Stim1$^{-/-}$ Stim2$^{-/-}$. Alternately, the use of transgenic mice where the thymocytes' genome comprises a homozygous disruption of a STIM gene (ie. STIM 1, STIM 2, or both STIM 1 and STIM 2) is also envisioned.

## DETAILED DESCRIPTION OF THE DRAWINGS

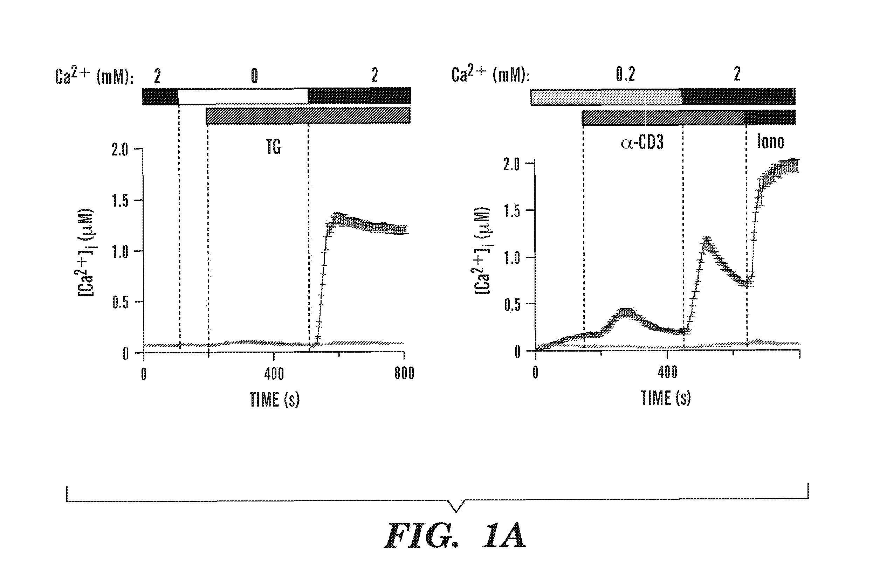

Fig. 1a. Store-operated Ca$^{2+}$ influx in littermate control (Stim1$^{+/+}$CD4-Cre or Stim1$^{fl/fl}$) (black) and Stim1$^{fl/fl}$CD4-Cre (grey) naive CD4$^+$ T cells in response to thapsigargin (TG, 1 $\mu$M) (left) or CD3 crosslinking followed by ionomycin (iono, 1 $\mu$M) (right), in the presence of 0.2 or 2 mM extracellular Ca$^{2+}$ as indicated.

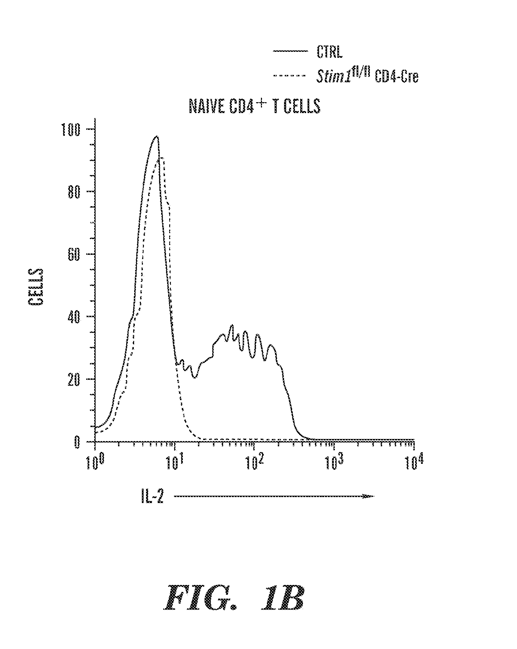

Fig. 1b. IL-2 production, as measured by intracellular cytokine staining. Naive CD4+ T cells were stimulated with PMA and ionomycin for 6 hours. Solid line: control (CTRL; Stim1$^{+/+}$CD4-Cre or Stim1$^{f1/fl}$), dashed line: Stim1$^{fl/fl}$CD4-Cre.

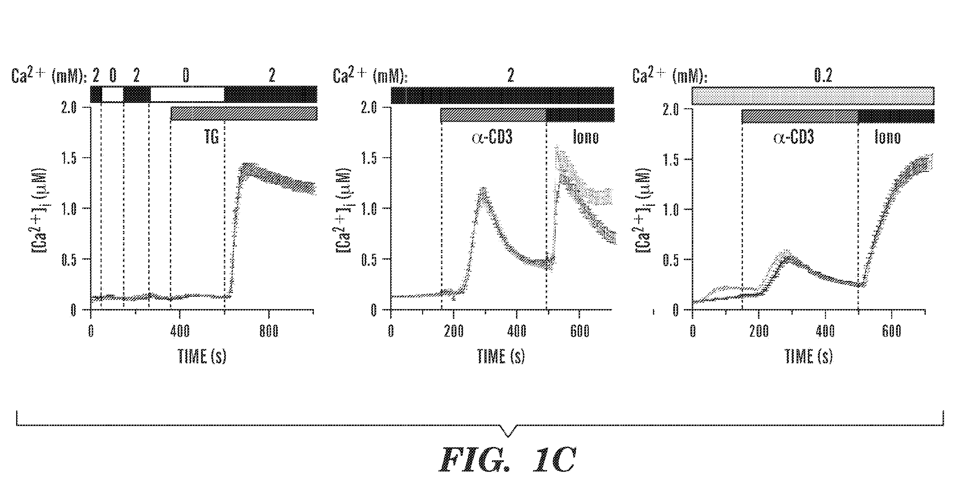

Fig. 1c. Store-operated Ca2+ entry in response to 1 $\mu$M TG (left) or CD3 crosslinking followed by 1 $\mu$M ionomycin (center and right) in naive CD4+ T cells from wild-type (WT, black) and Stim2$^{-/-}$ (grey) mice (both obtained by intercrossing Stim2$^{+/-}$-CMV-Cre- mice).

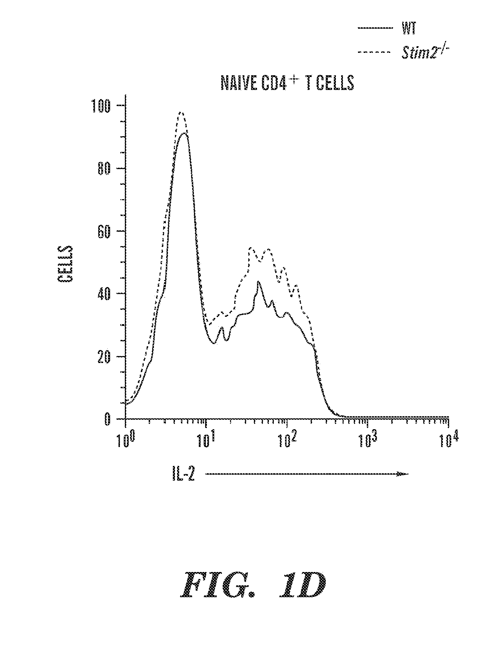

Fig. 1d. IL-2 production, measured by intracellular cytokine staining, by naive wild-type (WT, solid line) and Stim2$^{-/-}$ (dashed line) CD4$^+$ T cells stimulated for 6 h with PMA and ionomycin.

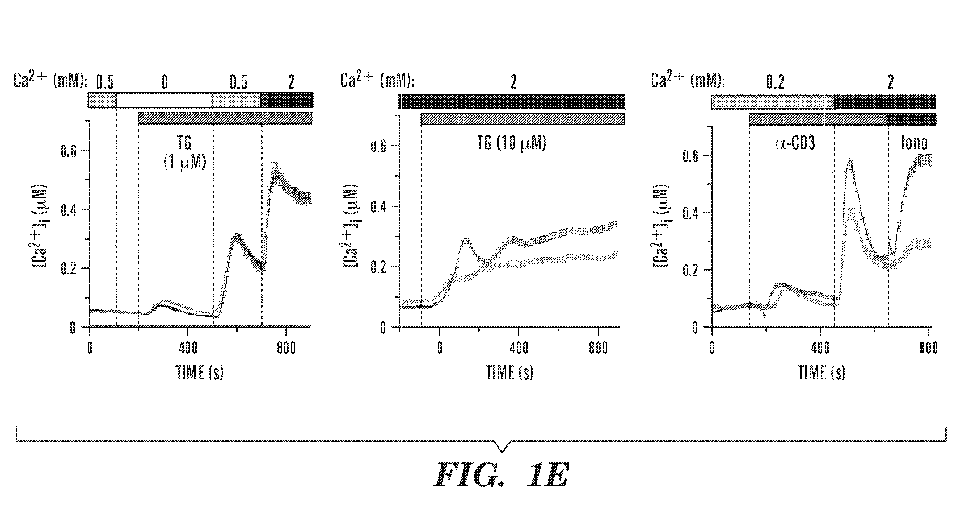

Fig. 1e. Representative [Ca$^{2+}$]i responses of control (Stim2$^{+/+}$CD4-Cre or Stim2$^{fl/fl}$) (black) and Stim2$^{-/-}$ (grey) THN cells differentiated for 7 d in vitro, in response to high (1 $\mu$M) or low (10 nM) TG or anti-CD3 followed by ionomycin as indicated.

Fig. If. IL-2 and IFN-$\gamma$ production by wild-type (WT, black) and Stim2$^{-/-}$ (grey) THN cells differentiated for 7 d in vitro, then restimulated for 6 h with PMA and ionomycin. Data are representative of at least three independent experiments. As controls, T cells from Stim$^{+/+}$CD4-Cre mice were compared with T cells from wildtype mice in initial experiments, to confirm that Cre expression had no toxic or other deteterious effect on Ca$^{2+}$ influx and cytokine expression. In subsequent experiments, Stim$^{+/+}$CD4-Cre and Stim$^{fl/fl}$ mice were used interchangeably as controls.

Fig. 2a. Left, Ca$^{2+}$ influx in wild-type (black), Stim1$^{-/-}$ (dark grey) and Stim2$^{-/-}$ (light grey) MEFs stimulated with 1 $\mu$M TG in Ringer solution containing 20 mM Ca$^{2+}$. Right, reconstitution of store-operated Ca$^{2+}$ entry by retroviral transduction with Myc-tagged STIM1 (black) or STIM2 (dark grey) into Stim1$^{-/-}$ MEFs. Empty vector, light grey. Expression vectors contained an IRES-GFP cassette and only GFP+ cells were analyzed.

Fig. 2b. Ca$^{2+}$ influx in response to treatment with 1 $\mu$M TG in control (CTRL; Stim1$^{+/+}$CD4-Cre or Stim1$^{fl/fl}$) and Stim1$^{fl/fl}$CD4-Cre THN cells transduced with empty retroviral vector or retroviral vectors encoding Myc-tagged STIM1 orSTIM2. Only GFP$^+$ cells were analyzed.

Fig. 2c. Representative dot plots (left) and averaged data (right) depicting cytokine production in cells of indicated genotypes (above dot plots) transduced with indicated retroviral vectors (right column). Only GFP$^+$ cells were analyzed. Error bars represent standard deviation. Data are representative of three independent experiments. As controls, Stim$^{+/+}$CD4-Cre mice were used initially, after which both Stim$^{+/+}$CD4-Cre and Stim$^{fl/fl}$ mice were used.

Fig. 3a. Averaged peak, steady-state (60 min after stimulation) [Ca$^{2+}$]$_i$ and initial rates of [Ca$^{2+}$]I in control (n=8) (CTRL; Stim2$^{+/+}$CD4-Cre or Stim2$^{fl/fl}$) or STIM2-deficient (n=7) (STIM2-KO; Stim2$^{fl/fl}$CD4-Cre) CD4$^+$ T cells differentiated under helper T non-polarizing conditions for 5 d, labeled with Fura-PE3, and stimulated with PMA and ionomycin (see Methods). Error bars, s.e.m. P-values were calculated using the unpaired student's t-test.

Fig. 3b Percent of CTRL or Stim1$^{+/+}$CD4-Cre cells with nuclear NFAT1 (mean $\pm$ s.d.) at indicated time points after stimulation. At least 300 cells/well were analyzed for each time point. Error bars, s.d.

Fig. 3c. Percent of CTRL or Stim2$^{+/+}$CD4-Cre with nuclear NFAT1 (mean $\pm$ s.d.) at indicated time points after stimulation. At least 300 cells/well were analyzed for each time point. Error bars, s.d.

Fig. 3d. IL-2 and IFN-$\gamma$ expression in CTRL or Stim1$^{+/+}$CD4-Cre cells. Data are representative of three independent experiments. As controls, Stim$^{+/+}$CD4-Cre mice were used initially, after which both Stim$^{+/+}$CD4-Cre and Stim$^{fl/fl}$ mice were used.

Fig. 3e. IL-2 and IFN-y expression in CTRL or Stim2+/+CD4-Cre cells. Data are representative of three independent experiments. As controls, Stim$^{+/+}$CD4-Cre mice were used initially, after which both Stim$^{+/+}$CD4-Cre and Stim$^{fl/fl}$ mice were used.

Fig. 4a. Current-voltage (I-V) relations recorded in extracellular 20 mM [Ca2$^+$]o and DVF solutions from differentiated CD4$^+$ T cells derived from indicated mice. Ca$^{2+}$ current (left panels) and monovalent cation current (right panels) elicited by TG (1 $\mu$M).

Fig. 4b. Single recordings of depotentiating Na$^+$ current elicited by replacement of 20 mM Ca$^{2+}$ Ringer solution (black bars) with divalent free (DVF) solution (open bars). Currents were measured during hyperpolarizing pulses to -100 mV applied every 1 s.

Fig. 4c. Summary of peak current densities recorded under the indicated conditions. Error bars represent s.e.m. n, number of cells analyzed. CTRL; Stim1$^{+/+}$CD4-Cre or Stim1$^{fl/fl}$ or Stim2$^{fl/fl}$ or Stim1$^{fl/fl}$Stim2$^{fl/fl}$, STIM1-KO; Stim1$^{fl/fl}$CD4-Cre, STIM2-KO; Stim2$^{fl/fl}$CD4-Cre, DKO; Stim1$^{fl/fl}$Stim2$^{fl/fl}$CD4-Cre. As controls, Stim$^{+/+}$CD4-Cre mice were used initially, after which both Stim$^{+/+}$CD4-Cre and Stim$^{fl/fl}$ mice were used.

Fig. 5a. Store-operated Ca$^{2+}$ influx. Left, naive CD4$^+$ T cells from littermate control (CTRL; Stim1$^{fl/fl}$Stim2$^{fl/fl}$, black) and DKO (Stim1$^{fl/fl}$Stim2$^{fl/fl}$CD4-Cre, grey) mice were stimulated with TG (top) or anti-CD3 followed by crosslinking with streptavidin (SA, bottom) in nominally Ca$^{2+}$-free Ringer solution followed by perfusion with 2 mM Ca$^{2+}$ Ringer solution to induce Ca$^{2+}$ influx. Middle, quantification of peak [Ca$^{2+}$]I in 2 mM Ca$^{2+}$ Ringer solution.

Fig.5b. IL-2 and TNF production by naive CD4+ T cells from control (CTRL; Stim1$^{+/+}$CD4-Cre or Stim1$^{fl/fl}$Stim2$^{fl/fl}$) or DKO mice stimulated for 6 h with PMA and ionomycin.

Fig. 5c. CD25 and CD69 expression on naive CD4$^+$ T cells from control (CTRL; Stim1$^{+/+}$CD4-Cre or Stim1$^{fl/fl}$Stim2$^{fl/fl}$) or DKO mice left unstimulated or stimulated for 16 h with anti-CD3 and anti-CD28.

Fig. 5d. Proliferation of naive CD4$^+$CD25$^-$ T cells from control (CTRL; Stim1$^{fl/fl}$Stim2$^{fl/fl}$) or DKO mice, as assessed by CFSE labeling. Cells were stimulated for 72 h with anti-CD3 and anti-CD28. Open histgrams, stimulated cells; shaded histograms, unstimulated cells.

Fig. 5e. Percentage of cells that underwent the indicated number of cell divisions (from data in Fig. 5d). Data are representative of two independent experiments. As controls, Stim$^{+/+}$CD4-Cre mice were used initially, after which both Stim$^{+/+}$CD4-Cre and Stim1$^{fl/fl}$Stim2$^{fl/fl}$ mice were used.

Fig. 6a. Thymocytes and splenocytes from 5-6 week old (left) or 3 month old (right) control (CTRL; Stim1$^{+/+}$CD4-Cre or Stim1$^{fl/fl}$Stim2$^{fl/fl}$) and DKO mice were stained for CD4, CD25 and Foxp3. Numbers above gates indicate percentage of cells within gate.

Fig. 6b. Numbers of total (left) and CD4$^+$CD25$^+$ T$_{reg}$ cells (right) in thymus and spleen of 5-6 week old control (CTRL; Stim1$^{+/+}$CD4-Cre or Stim1$^{fl/fl}$Stim2$^{fl/fl}$) or DKO mice (n=3; mean $\pm$ s.d.).

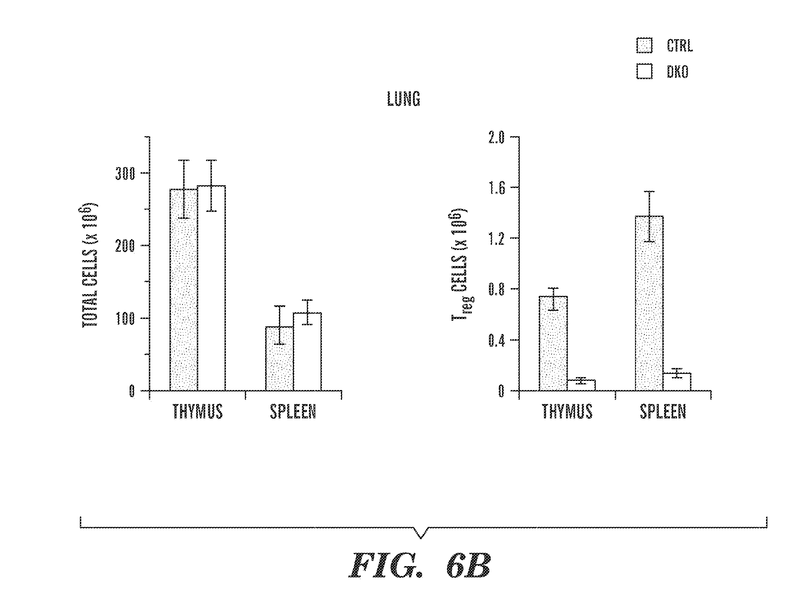

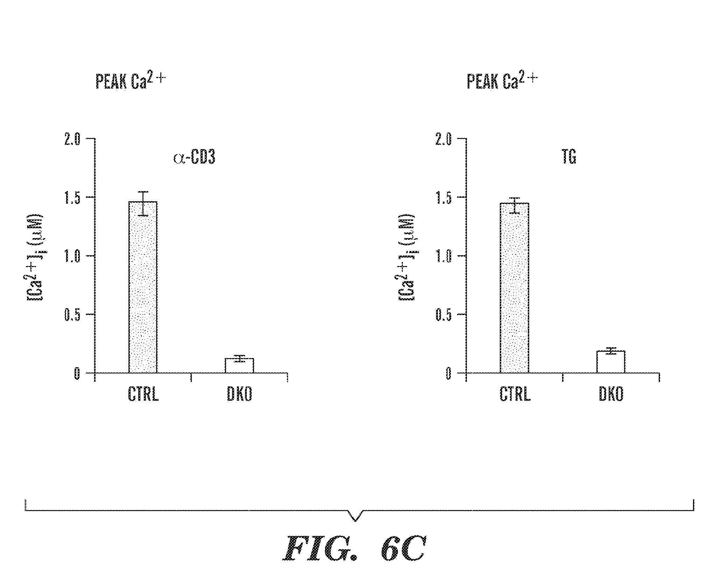

Fig. 6c. Store-operated Ca$^{2+}$ influx induced by CD3 crosslinking (left) or treatment with 1 $\mu$M TG (right) in CD4$^+$CD25$^+$ cells isolated from control (CTRL; Stim1$^{fl/fl}$Stim2$^{fl/fl}$) or DKO mice. Measurements were made in Ringer solution with 2 mM Ca$^{2+}$. Data are representative of three (anti-CD3) and two (TG) independent experiments for which 110 to 170 cells were analyzed each. Stim$^{+/+}$CD4-Cre mice were used as controls initially to confirm that Cre expression did not result in toxicity or other adverse effects. Subsequently, Stim1$^{fl/fl}$Stim2$^{fl/fl}$ mice were used to control for other factors, such as sex, age and breeding environment.

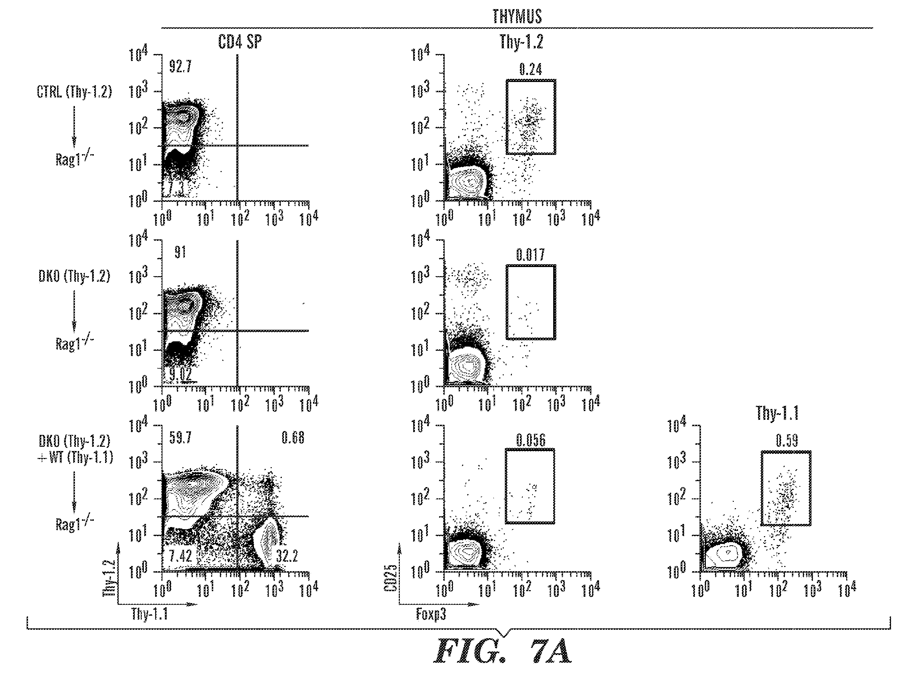

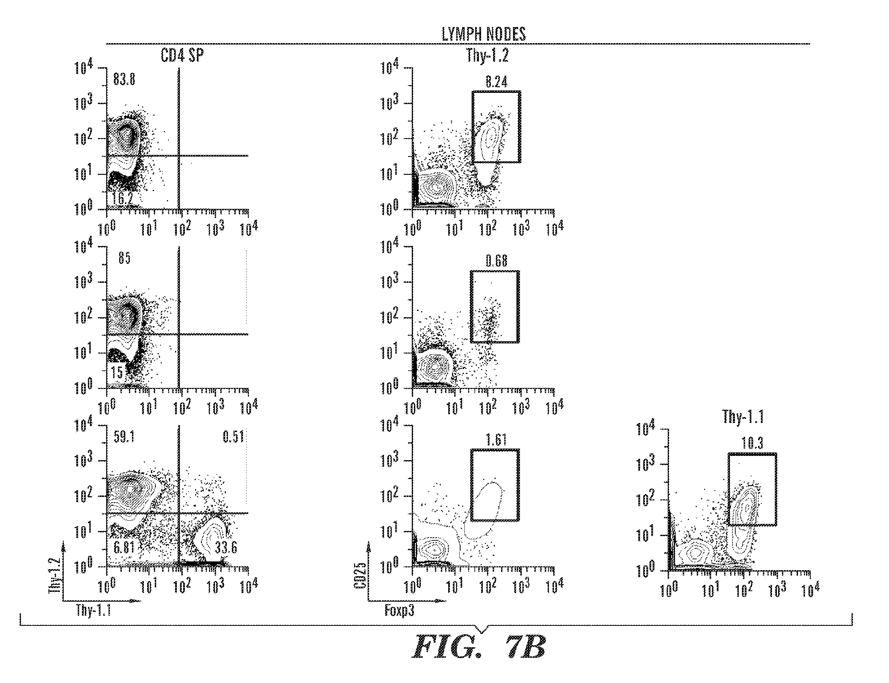

Fig.7a and 7b. Absence of STIM1 and STIM2 impairs Treg cells development. Sublethally-irradiated Rag1$^{-/-}$ mice were reconstituted with T cell-depleted bone marrow cells from Thy-1.2$^+$ control littermates (CTRL; Stim1$^{fl/fl}$Stim2$^{fl/fl}$) alone, Thy1.2$^+$ DKO mice (3 $\times$ 10$^6$ cells) alone, or from both Thy1.2$^+$ DKO (3 $\times$ 10$^6$ cells) and congenic B6 Thy1.1$^+$ wild-type mice (1.5 x 10$^6$ cells). 10-12 weeks after reconstitution, cells from thymus or lymph nodes were stained with anti-CD4, anti-Thy1.1, and anti-Thy1.2 together with anti-CD25 and anti-Foxp3. Numbers in quadrants indicate percentage of cells within that quadrant. Data are representative of results from three mixed chimeric mice from two independent experiments. As no Cre toxicity was observed in prior experiments, Stim1$^{f1/f1}$Stim2$^{f1/f1}$ mice were used as controls to adjust for other factors, such as sex, age and breeding environment.

Fig. 8a. Numbers of spleen and lymph node cells in recipient mice. Adoptive transfer of wild-type T$_{reg}$ cells suppresses the lymphoproliferative phenotype of DKO mice. $3 \times 10^5$ CD4$^+$CD25$^+$ or CD4$^+$CD25- Thy1.1$^+$ T cells from wild-type mice were transferred into 2 week old Thy1.2$^+$ DKO mice, and analysis was done 8 weeks after transfer. Error bars represent s.d. P-values were calculated using the paired student's t-test. Data are representative of results from three mice from two independent experiments.

Fig. 8b. Cells from spleen and lymph nodes were stained with indicated antibodies. Numbers above gates represent percentage of cells within gate. Data are representative of results from three mice from two independent experiments.

Fig. 8c. Donor cell engraftment. Cells were stained with antibodies against Thy1.1, Thy1.2, CD4, CD25, and Foxp3. Numbers of endogenous (Thy1.2$^+$, dark grey) and transferred (Thy1.1 light grey) T$_{reg}$ cells in mice that received CD25- or CD25$^+$ T cell are plotted. Error bars represent s.d.

Fig. 8d. Suppressive function of DKO T$_{reg}$ cells. CD4$^+$CD25$^+$ T cells were purified from control (CTRL; Stim1$^{f1/f1}$Stim2$^{f1/f1}$) or DKO mice and co-cultured with CFSE-labeled responder CD4$^+$CD25$^-$ T cells at a 1:1 ratio for 72 h in the presence of mitomycin C-treated T cell-depleted splenocytes and 0.3 $\mu$g/ml anti-CD3. Data are representative of at least three independent experiments. As no Cre toxicity was observed in prior experiments, Stim1$^{fl/fl}$Stim2$^{fl/fl}$ mice were used as controls to adjust for other factors, such as sex, age and breeding environment.

Fig 9a. Schematic diagram of the targeting strategy for conditional deletion of the *Stim1* gene. Exon 2, encoding the EF hand motif of the *Stim1* gene was flanked by loxP recombination sites (triangles). The neomycin resistance (neoR) gene was flanked by Frt recombination sites (hexagons). Deletion of these exons is predicted to result in each case in a frameshift which generates a premature stop codon in the next exon.

Fig 9b. Schematic diagram of the targeting strategy for conditional deletion of the Stim2 gene. Exon 3, encoding sequences C-terminal to the EF hand of the *Stim2* gene, was flanked by loxP recombination sites (triangles). The neomycin resistance (neoR) gene was flanked by Frt recombination sites (hexagons). Deletion of these exons is predicted to result in each case in a frameshift which generates a premature stop codon in the next exon.

Fig. 9c. Typical result of genotyping by PCR. Primers are indicated in a by arrows.

Fig. 9d. STIM1 and STIM2 expression in indicated cell types. For STIM1 detection, 25 $\mu$g protein was loaded in each lane of the SDS-polyacrylamide gel. For STIM2 detection, protein derived from 5 million cells (approximately 80 $\mu$g) was loaded in each lane. The band corresponding to STIM2 is indicated by the arrowhead. BMMC, bone marrow derived mast cells, MEF, mouse embryonic fibroblasts.

Fig. 10a. Expression of CD4 and TCRβ on Stim1$^{fl/fl}$CD4-Cre and control (CTRL; Stim1$^{+/+}$CD4-Cre or Stim1$^{fl/fl}$) naive CD4$^+$ T cells. Data are representative of two independent experiments.

Fig. 10b, Expression of CD25 and CD69 activation markers on Stim1$^{fl/fl}$CD4-Cre and control CD4$^+$ T cells 16 h after stimulation with anti-CD3 and anti-CD28.

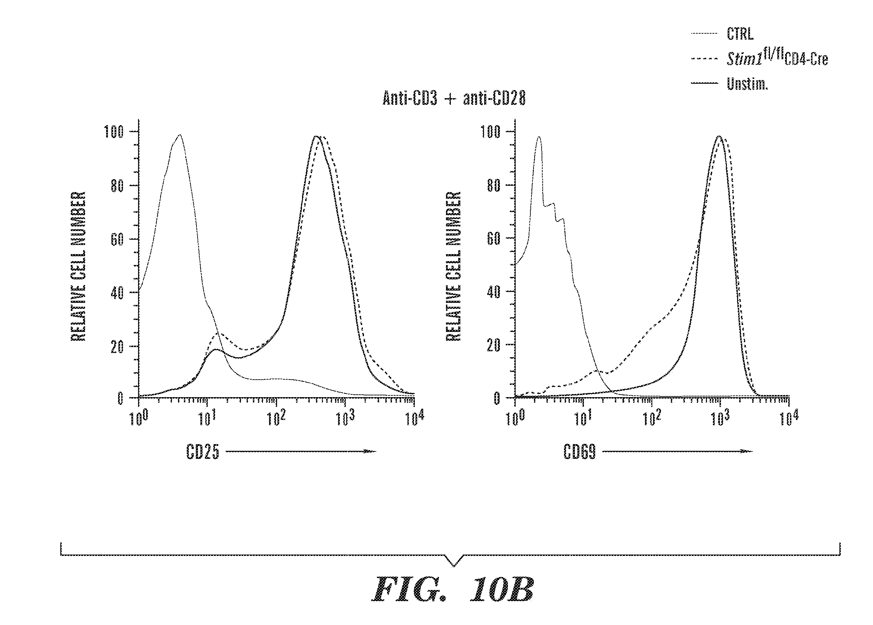

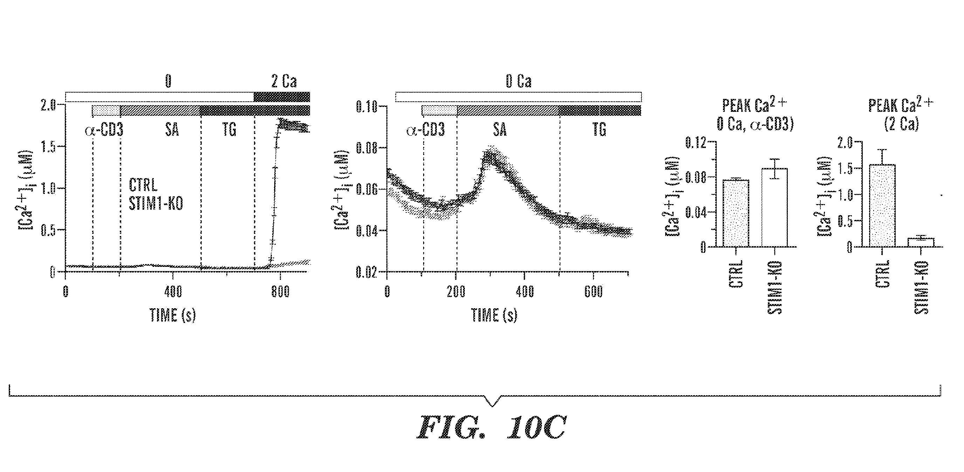

Fig. 10c, STIM1-deficient T cells show normal store depletion. Left, Naive CD4$^+$ T cells from control (CTRL) (black) and Stim1$^{fl/fl}$CD4-Cre (STIM1-KO, grey) littermates were sequentially perfused with biotinylated anti-CD3, streptavidin (SA) and thapsigargin (TG) in nominally Ca$^{2+}$-free Ringer solution followed by Ringer solution containing 2 mM Ca$^{2+}$ as indicated to induce Ca$^{2+}$ influx. Middle, as in left panel with y-axis enlarged to visualize store depletion. Right, quantification of peak [Ca$^{2+}$]I following store depletion (left) and influx (right) in Ringer solution containing 0 and 2 mM Ca$^{2+}$, respectively. Data are representative of two (CTRL; Stim1$^{fl/fl}$) and three (Stim1$^{fl/fl}$CD4-Cre) independent experiments.

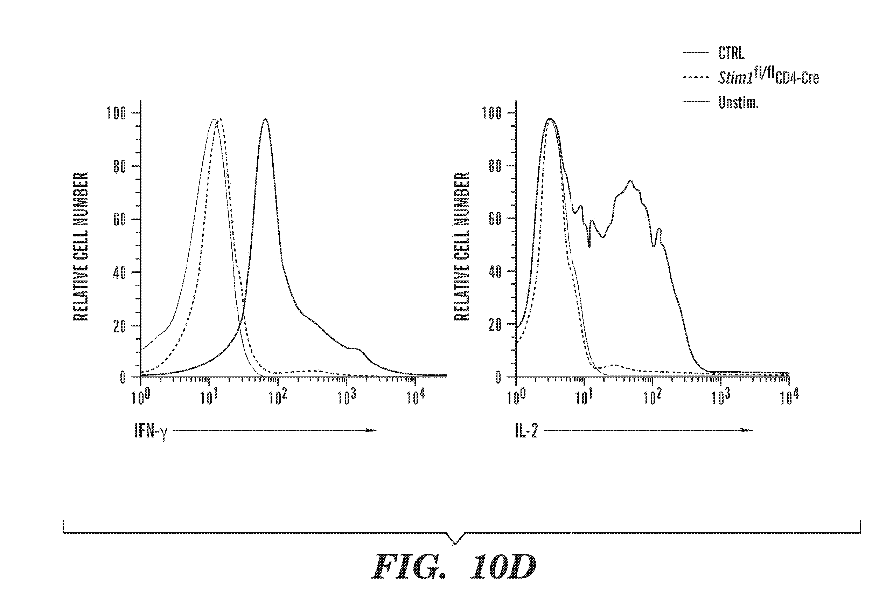

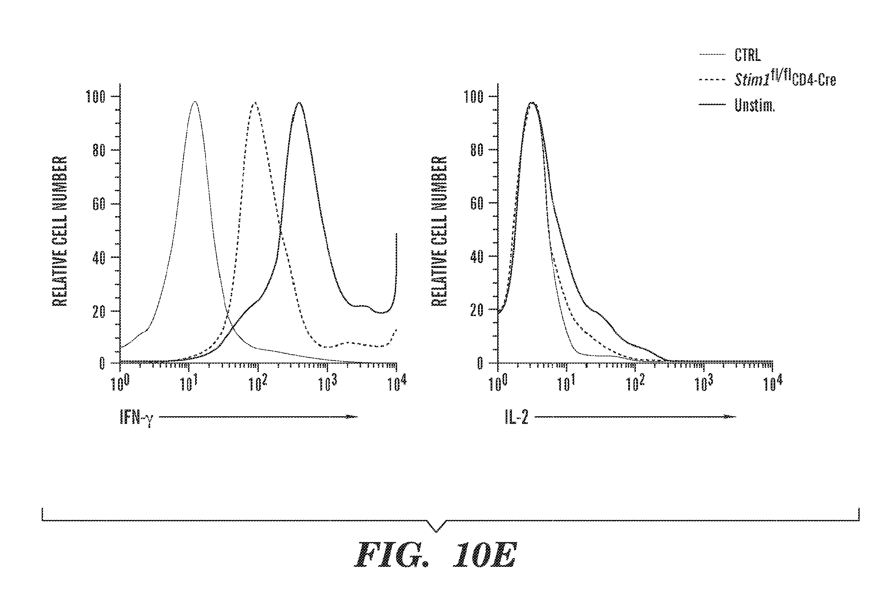

Fig. 10d and 10e, Cytokine production by CD4$^+$ helper T cells from indicated mice differentiated *in vitro* for 7 days and restimulated with anti-CD3 and anti-CD28 for 6 h. Data are representative of three independent experiments. As controls, T cells from Stim$^{+/+}$CD4-Cre mice were compared with T cells from wildtype mice in initial experiments, to confirm that Cre expression had no toxic or other deteterious effect on Ca$^{2+}$ influx and cytokine expression. In subsequent experiments, Stim$^{+/+}$CD4-Cre and Stim$^{fl/fl}$ mice were used interchangeably as controls. Data are representative of three independent experiments.

Fig. 11a. Upregulation of STIM2 protein level in activated T cells. Naive CD4$^+$ T cells from indicated mice were stimulated with anti-CD3 and anti-CD28 for 1, 2 or 3 days, or differentiated under T$_H$N, T$_H$1 or T$_H$2 conditions for 7 days. 25 $\mu$g of whole cell lysate was loaded in each lane. Arrowheads indicate the specific bands.

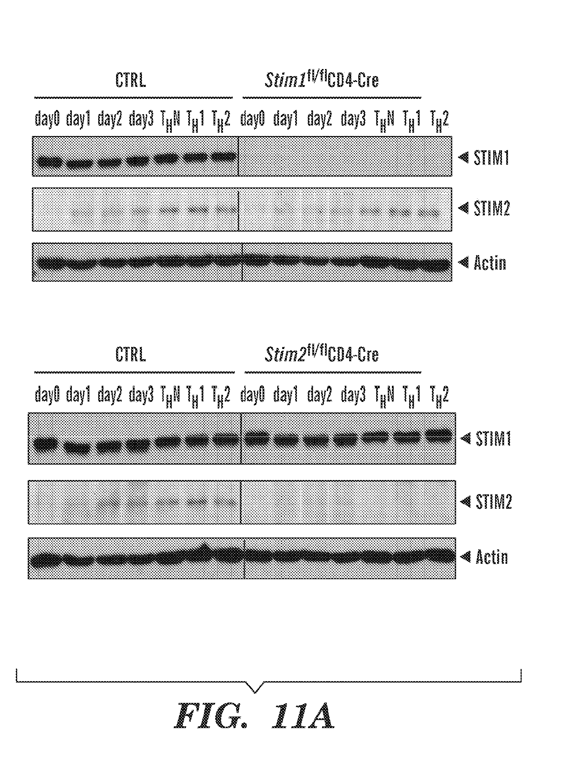

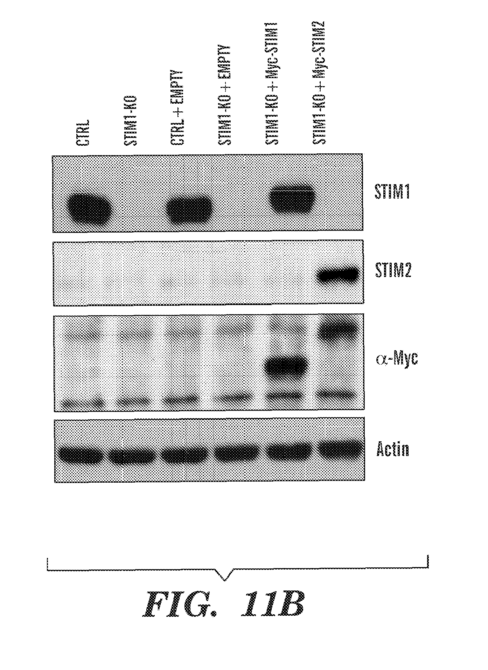

Fig. 11 b. Purified CD4$^+$ T cells from indicated mice were transduced with retroviral vectors containing an IRES-GFP cassette (empty or encoding Myc-tagged STIM1 or STIM2, as indicated in right column). The efficiencies of transduction were similar with all retroviruses (~40%) as judged by GFP expression (data not shown). Cells were lysed and subjected to immunoblotting as in a. CTRL; Stim1$^{+/+}$CD4-Cre or Stim1$^{fl/fl}$. Data are representative of three independent experiments. As controls, Stim$^{+/+}$CD4-Cre mice were used initially, after which both Stim$^{+/+}$CD4-Cre and Stim$^{fl/fl}$ mice were used.

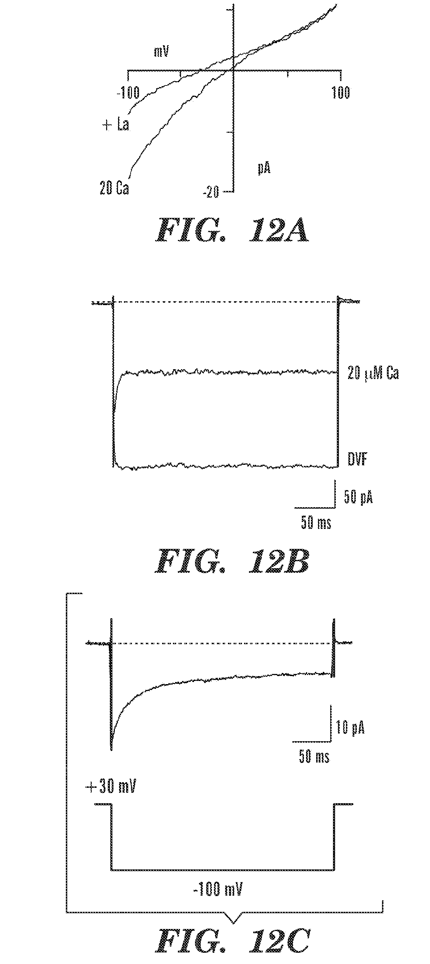

Fig. 12a. Electrophysiological raw currents in wild-type mouse T cells in extracellular 20 mM [Ca2+]o and following addition of 25 $\mu$M $La^{3+}$. The current in the presence of $La^{3+}$ was attributed to leak.

Fig. 12b. $Na^+$ CRAC currents in wild-type mouse T cells, blocked by ~50% with 20 $\mu$M $Ca^{2+}$, similar to the $IC_{50}$ of $Ca^{2+}$ inhibition in Jurkat T cells.

Fig. 12c. $I_{CRAC}$ in wild-type mouse T cells exhibiting fast inactivation following hyperpolarizing steps to -100 mV.

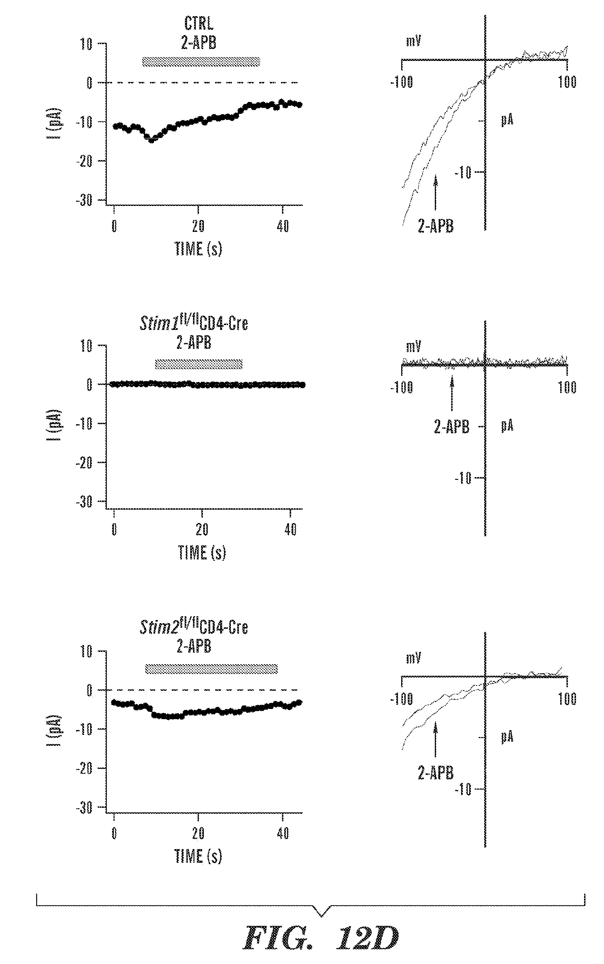

Fig. 12d. Potentiation and inhibition of $I_{CRAC}$ by 2-Aminoethoxydiphenyl borate (2-APB) in T cells derived from indicated mice. Thapsigargin-pretreated cells were exposed to 2-APB (40 $\mu$M, grey bar). Left, current at the end of a 100 ms step to -100 mV. Right, examples of I-V curves before and immediately following exposure to 2-APB (arrows). The increase in current amplitude following 2-APB application was 26$\pm$ 6% (n=4) in control T cells and 30$\pm$ 7% (n=5) in STIM2-deficient T cells. CTRL; $Stim1^{+/+}$CD4-Cre or $Stim1^{fl/fl}$. As controls, $Stim^{+/+}$CD4-Cre mice were used initially, after which both $Stim^{+/+}$CD4-Cre and $Stim^{fl/fl}$ mice were used.

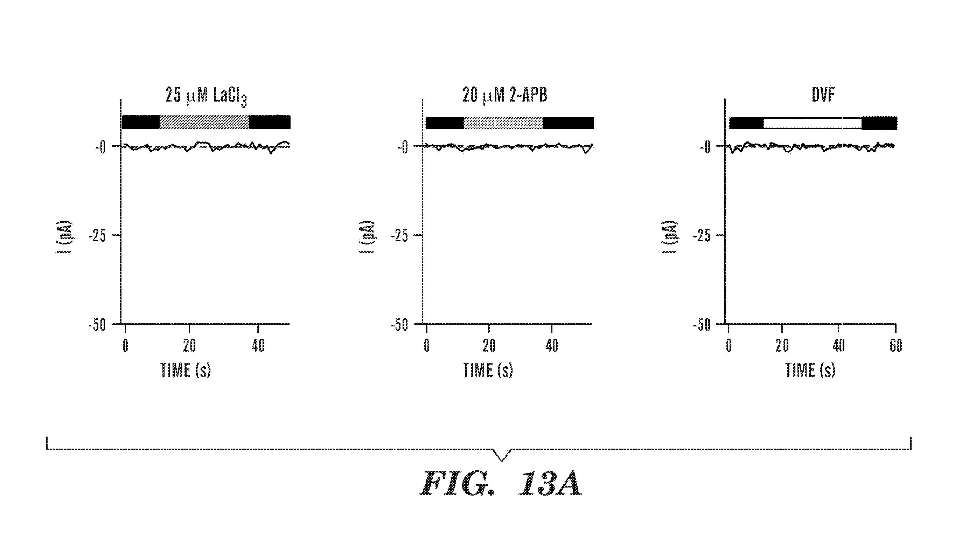

Fig. 13a. $I_{CRAC}$ in 20 mM $Ca^{2+}$ Ringer solution (black bars) or divalent free DVF solution (open bars) in DKO T cells. Currents were measured during hyperpolarizing pulses to-100 mV applied every 1 s.

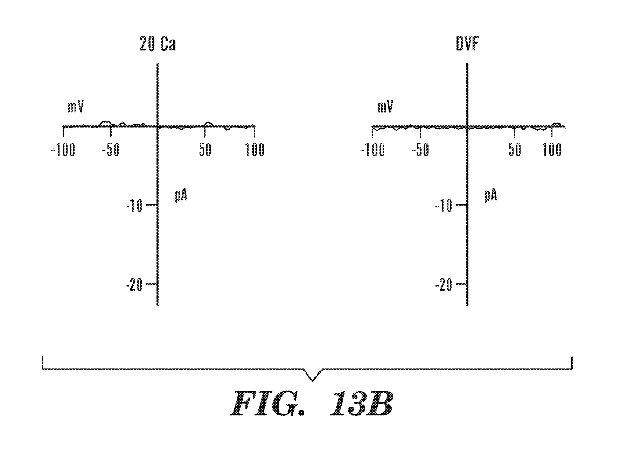

Fig. 13b. Current-voltage (I-V) relations recorded in extracellular 20 mM [Ca2+]o and DVF solutions from differentiated $CD4^+$ T cells derived from DKO mice. Store-depletion was induced by thapsigargin (1 $\mu$M). Left, $Ca^{2+}$ current; right, monovalent cation current.

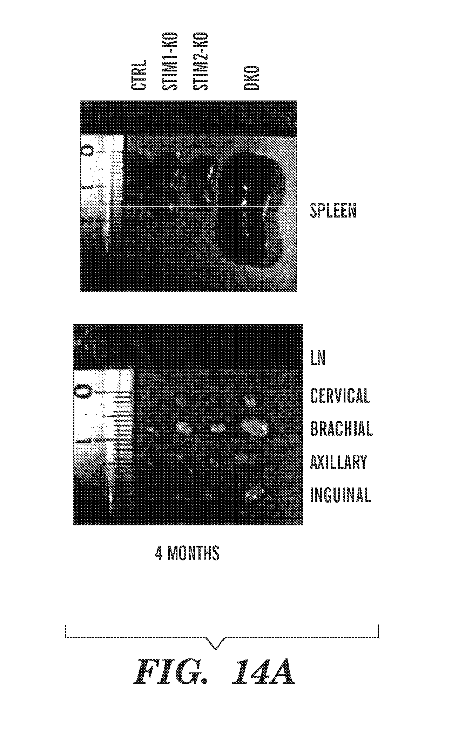

Fig. 14a. Phenotypic characterization of peripheral lymphoid cells derived from $Stim1^{fl/fl}Stim2^{fl/fl}$CD4-Cre mice. Representative spleens (top) and lymph nodes (bottom) from 4 month-old mice.

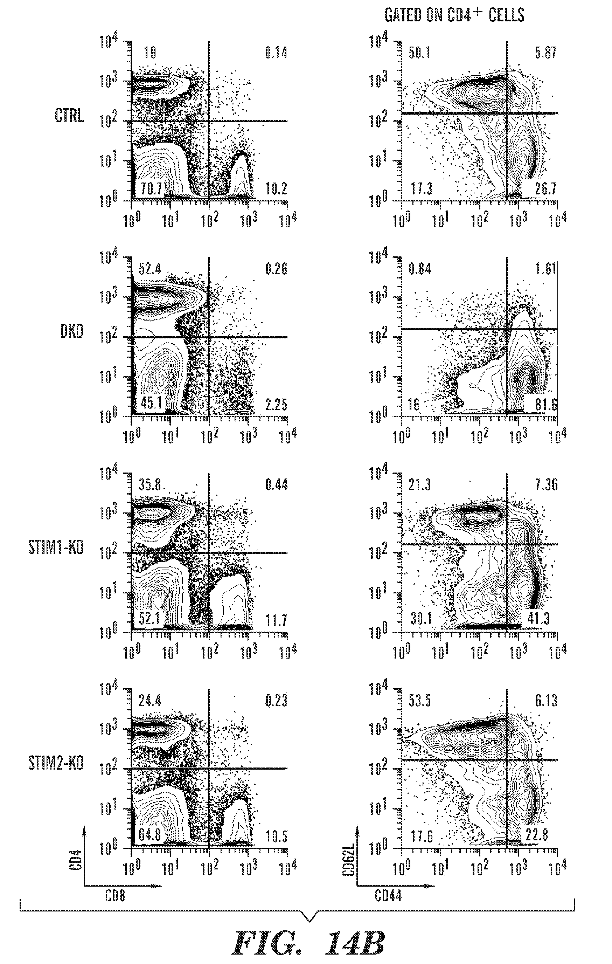

Fig. 14b. Splenocytes from indicated mice were analyzed for CD4 and CD8 expression by flow cytometry. CD4+ cells were gated and further analyzed for expression of CD44 and CD62L.

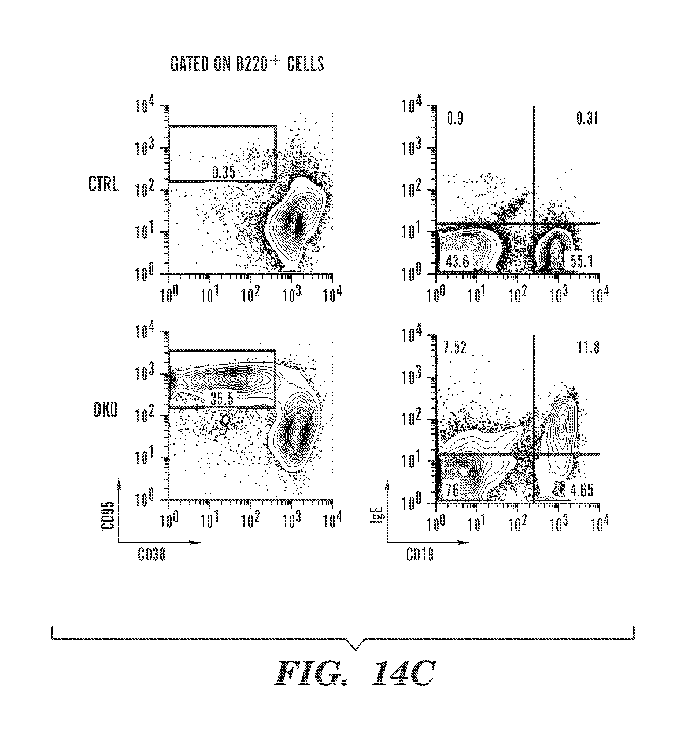

Fig. 14c. Germinal center B cells ($CD9S^+CD38^{low}$) and differentiated $CD19^+ IgE^+$ B cells among gated $B220^+$ B cells.

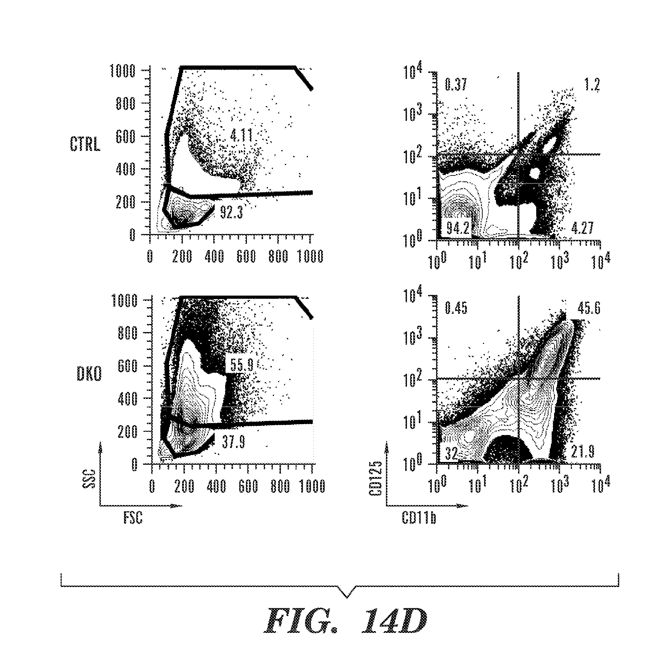

Fig. 14d. Right, splenocytes were stained for CD11b and CD125 (IL-5R). $CD11b^+$IL-5R+ positive cells correspond to eosinophils and basophils. Left large, activated or granulated cells are gated in the FCS/SSC plot. CTRL; $Stim1^{+/+}$CD4-Cre or $Stim1^{fl/fl}Stim2^{fl/fl}$, STIM1-KO; $Stim1^{fl/fl}$CD4-Cre, STIM2-KO; $Stim2^{fl/fl}$CD4-Cre, DKO; $Stim1^{fl/fl}Stim2^{fl/fl}$CD4-Cre. Data are representative of at least three independent experiments. $Stim^{+/+}$CD4-Cre mice were used as controls initially to confirm that Cre expression did not result in toxicity or other adverse effects. Subsequently, $Stim1^{fl/fl}Stim2^{fl/fl}$ mice were used to control for other factors, such as sex, age and breeding environment.

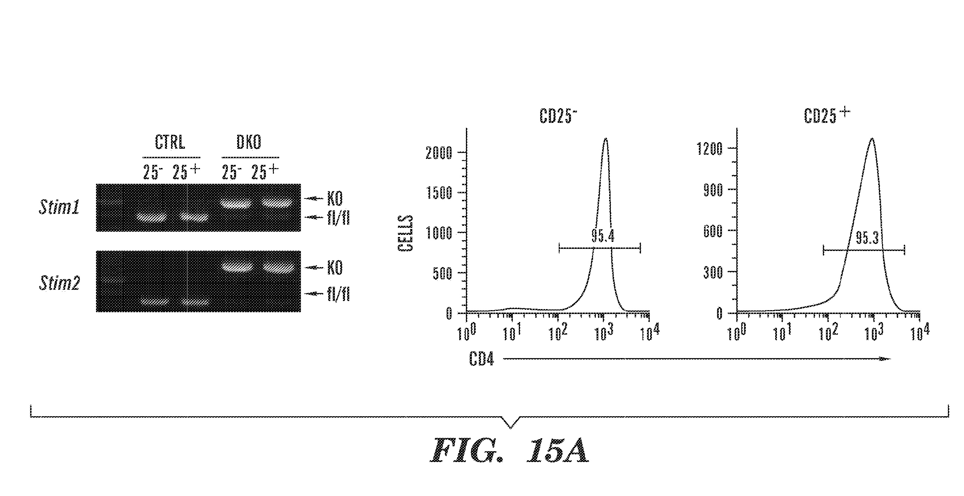

Fig. 15a. Genotyping of peripheral T cells derived from control (CTRL; $Stim1^{fl/fl}Stim2^{fl/fl}$) and DKO mice. Left, typical result of genotyping by PCR. Right, purity of $CD4^+$ T cells used for PCR ($\sim$ 95%). 25-, $CD4^+CD25^-$ conventional T cells; 25+, $CD4^+CD25^+$ regulatory T cells. Data are representative of two independent experiments.

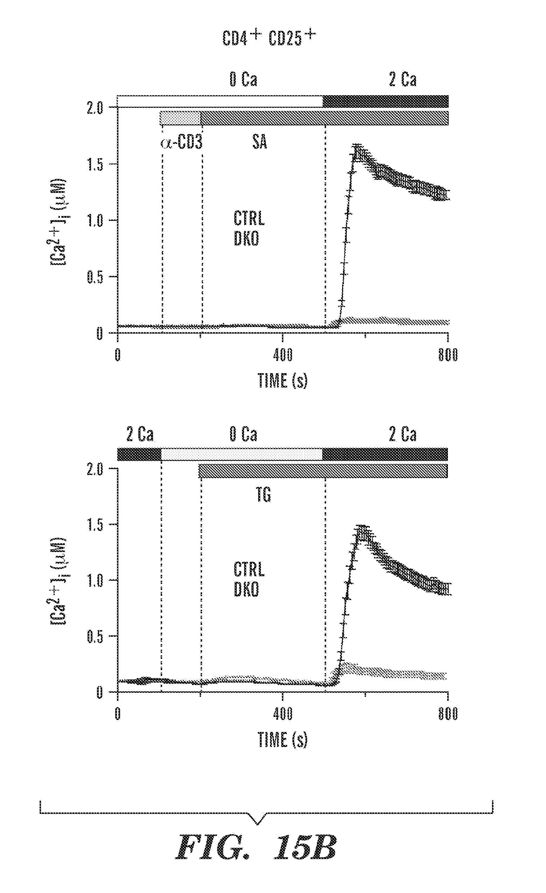

Fig. 15b. Impaired store-operated $Ca^{2+}$ influx in $CD4^+CD25^+$ $T_{reg}$ cells from DKO mice. $T_{reg}$ cells from control (CTRL, black) and DKO (grey) mice were stimulated either with anti-CD3 (crosslinked with streptavidin, SA) (top) or thapsigargin (TG, bottom) in nominally $Ca^{2+}$ free Ringer solution followed by perfusion with 2 mM $Ca^{2+}$ Ringer solution to induce $Ca^{2+}$ influx. Data are representative of three (anti-CD3) and two (TG) independent experiments for which 110 to 170 cells were analyzed each. As no Cre toxicity was observed in prior experiments, $Stim1^{fl/fl}Stim2^{fl/fl}$ mice were used as controls to adjust for other factors, such as sex, age and breeding environment.

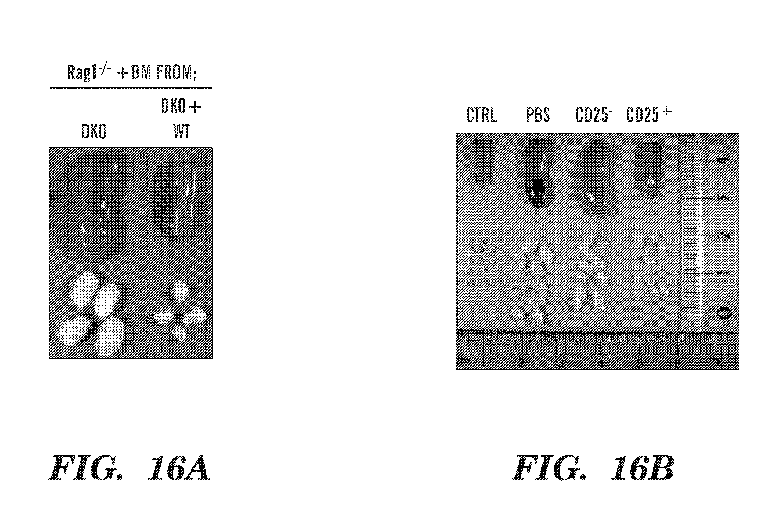

Fig. 16a. Suppression of lymphoproliferative phenotypes by wild-type $T_{reg}$ cells. Representative photographes of spleens (top) and lymph nodes (bottom) from Spleens (top) and lymph nodes (bottom) from sublethally-irradiated $Rag1^{-/-}$recipient mice reconstituted with T cell-depleted bone marrow cells from $Thy1.2^+$ DKO mice alone (left) or from both $Thy1.2^+$ DKO and congenic B6 $Thy1.1^+$ wild-type mice (right). Data are representative of results from three mixed chimeric mice from two independent experiments.

Fig. 16b. Representative photographes of spleens and lymph nodes from control mice and DKO mice injected with PBS, $CD4^+CD25^-$ and $CD4^+CD25^+$ T cells at 8 weeks after adoptive transfer of wild-type $T_{reg}$ cells to DKO mice. As no Cre toxicity was observed in prior experiments, we used $Stim1^{fl/fl}Stim2^{fl/fl}$ mice as controls to adjust for other factors, such as sex, age and breeding environment.

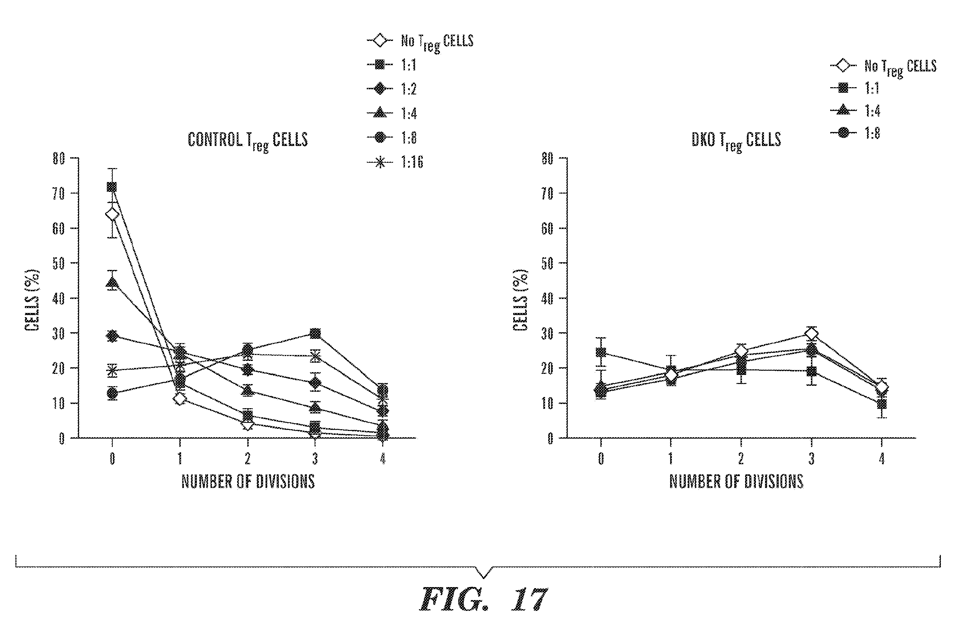

Fig. 17. Impaired suppressive activity of $Stim1^{fl/fl}Stim2^{fl/fl}$CD4-Cre Treg cells. $CD4^+CD25^+$ T cells purified from control (CTRL; $Stim1^{fl/fl}Stim2^{fl/fl}$, left) or DKO (right) mice were co-cultured with CFSE-labeled responder $CD4^+CD25^-$ T cells at indicated ratios for 72 h in the presence of mitomycin C-treated T cell-depleted splenocytes and 0.3 $\mu$g/ml anti-CD3. Error bars represent s.d. Data are average of at least three independent experiments. As no Cre toxicity was observed in prior experiments, we used Stim1fl/flStim2fl/fl mice as controls to adjust for other factors, such as sex, age and breeding environment.

## DETAILED DESCRIPTION OF THE INVENTION

Definitions

As used herein, the term "STIM protein" refers to a STIM 1 protein, a STIM 2 protein, or both STIM 1 and STIM 2 proteins. Examples of a STIM protein includes all mammalian STIM proteins, for example, human STIM 1 protein (Genbank Protein Accession Nos: Q13586, NP-003147, AAC51627), human STIM 2 (Genbank Protein Accession Nos: Q9P246, NP-065911, AAK82337), mouse STIM 1 (Genbank Protein Accession Nos: NP-033313) and mouse STIM 2 protein (Genbank Protein Accession Nos: P83093, NP-001074572, AAK82339, CAN36430).

As used herein, the term "STIM gene" refers to a nucleotide sequence encoding a STIM 1 protein or a STIM 2 protein. Examples of a STIM gene includes nucleotide sequences encoding all mammalian STIM proteins, for example, the human STIM 1 gene (Genbank Accession Nos.: NM_003156, gi2264345, gi2264346), the human STIM 2 gene (Genbank Accession Nos.: NM_020860, AF328905), the mouse STIM 1 gene (Genbank Accession No.: NM_009287,) and the mouse STIM 2 gene (Genbank Accession Nos.: NM_001081103, AM712359, AF328907).

In a knockout, preferably the target gene expression is undetectable or insignificant. A knock-out of a STIM gene means that the function of the respective STIM protein has been substantially decreased so that expression is not detectable or only present at insignificant levels. This may be achieved by a variety of mechanisms, including introduction of a disruption of the coding sequence, e.g. insertion of one or more stop codons, insertion of a DNA fragment, etc., deletion of coding sequence, substitution of stop codons for coding sequence, etc. In some cases the exogenous transgene sequences are ultimately deleted from the genome, leaving a net change to the native sequence. Different approaches may be used to achieve the "knock-out". A chromosomal deletion of all or part of the genomic gene may be induced, including deletions of the non-coding regions, particularly the promoter region, 3' regulatory sequences, enhancers, or deletions of gene that activate expression of STIM genes. A functional knock-out may also be achieved by the introduction of an anti-sense construct that blocks expression of the native genes (for example, see Li and Cohen (1996) Cell 85:319-329). "Knock-outs" also include conditional knock-outs, for example where alteration of the target gene occurs upon exposure of the animal to a substance that promotes target gene alteration, introduction of an enzyme that promotes recombination at the target gene site (e.g. Cre in the Cre-lox system), or other method for directing the target gene alteration postnatally.

As used herein, the term "deficient in the STIM 1 gene" or "STIM 1-deficient" means that no functional STIM 1 protein is produced due to the disruption of the STIM 1 gene. As used herein, the term "deficient in the STIM 2 gene" means that no functional STIM 2 protein is produced due to the disruption of the STIM 2 gene. As used herein, the term "deficient in the STIM1 and STIM 2 genes" or "deficient in the STIM proteins" mean that no functional STIM 1 and STIM 2 protein are produced due to the disruption of both the STIM 1 and STIM 2 gene.

The term "gene" means the nucleic acid sequence which is transcribed (DNA) to RNA in vitro or in vivo when operably linked to appropriate regulatory sequences. The gene may or may not include regions preceding and following the coding region, e.g. 5' untranslated (5'UTR) or "leader" sequences and 3' UTR or "trailer" sequences, as well as intervening sequences (introns) between individual coding segments (exons).

As used herein, the term "functional STIM protein" refers to a STIM protein that can sense the depletion in the $Ca^{2+}$ stores in the endoplasmic reticulum, triggers the $Ca^{2+}$ release-activated calcium channels in the plasma membrane to open, and allow an influx of $Ca^{2+}$ from the exterior into the cell.

As to the mice of the present invention, the term "tissue" includes any tissues, for example but not limited to, spleen, bone marrow, lymph nodes, endocrine tissues such as pancreatic islets, pituitary glands and exocrine tissues such as exocrine pancreas, gastric glands, small intestinal glands, Brunner's glands, salivary glands, mammary glands, etc., and their acini.

When a cell or animal has two identical or substantially similar alleles of a gene, it is said to be "homozygous." In contrast, when the cell or animal has two substantially different alleles it is said to be "heterozygous" for that gene.

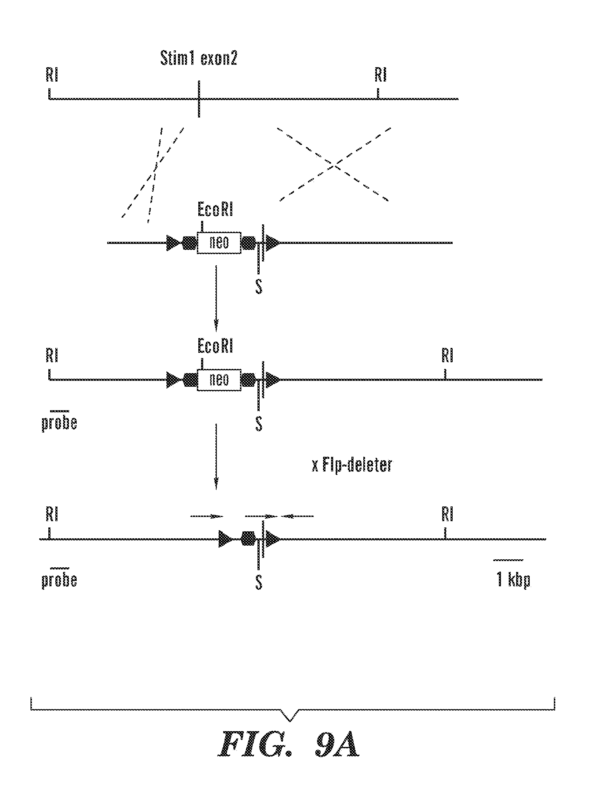

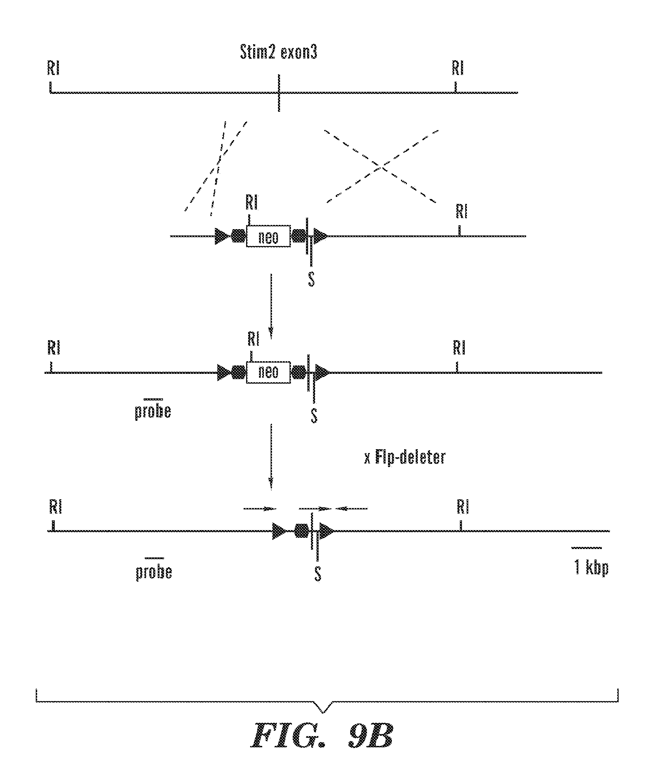

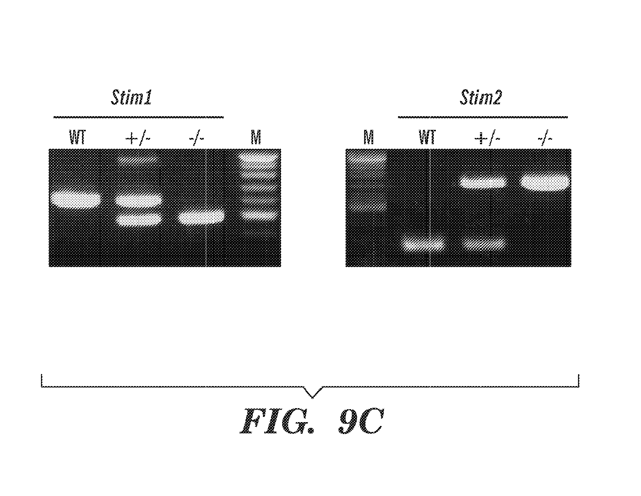

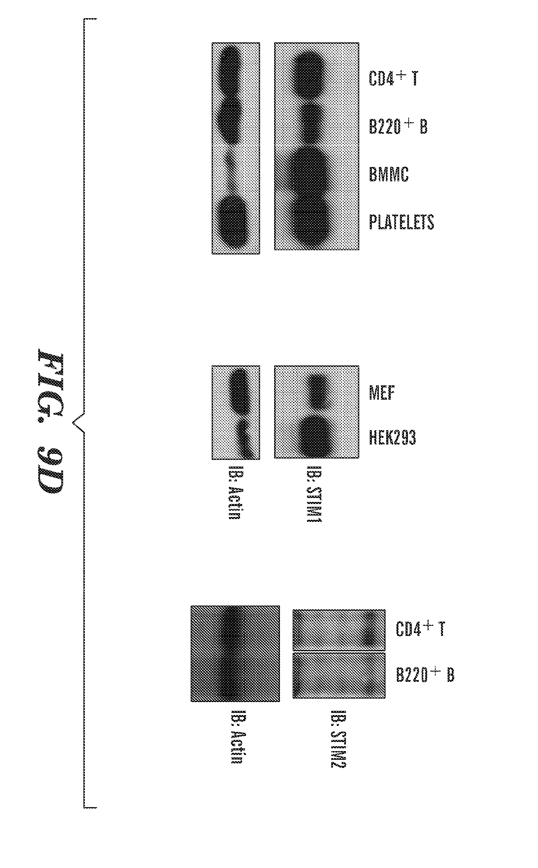

As used herein, the term "transgene" refers to a nucleic acid sequence which is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can be operably linked to one or more transcriptional regulatory sequences and any other nucleic acid, such as introns, that may be necessary for optimal expression of a selected nucleic acid. Exemplary transgenes of the present invention encode, for instance, a neomycine resistance gene fused with a STIM exon 2 and a Cre recombinase enzyme. Other exemplary transgenes are directed to disrupting a STIM gene by homologous recombination with genomic sequences of a STIM gene (See Figure 9).

As used herein, "modulation" with reference to STIM protein function refers to any alteration or adjustment in $Ca^{2+}$ binding, $Ca^{2+}$sensing, and/or activation of Orai1 that eventually leads to changes in calcium movements or fluxes into, out of and within cells. Modulation includes, for example, increases, up-regulation, induction, stimulation , relief of inhibition, reduction, inhibition, down-regulation and suppression.

As used herein, "modulation" with reference to intracellular calcium refers to any changes in the intracellular calcium including but not limited to alteration of calcium concentration in the cytoplasm and/or intracellular calcium storage organelles, e.g., endoplasmic reticulum, and alteration of the amplitude or kinetics of calcium movements or fluxes into, out of and within cells. Modulation includes, for example, increases, up-regulation, induction, stimulation, potentiation, relief of inhibition, reduction, inhibition, down-regulation and suppression.

As used herein, "agent" refers to any substance that can modulate intracellular calcium. Examples of agents include, but are not limited to, small organic molecules, large organic molecules, amino acids, peptides, polypeptides, nucleotides, nucleic acids (including DNA, cDNA, RNA, antisense RNA and any double- or single-stranded forms of nucleic acids), polynucleotides, carbohydrates, lipids, lipoproteins, glycoproteins, inorganic ions (including, for example, $Gd^{3+}$, lead and lanthinum).

As used herein, the term "comprising" means that other elements can also be present in addition to the defined elements presented. The use of "comprising" indicates inclusion rather than limitation.

As used herein, the term "heterologous nucleic acid" refers to nucleic acid sequences that are not naturally occurring in a cell. For example, when a Stim gene is inserted into the genome of a bacteria or virus, that Stim gene is heterologous to that recipient bacteria or virus because the bacteria and viral genome do not naturally have the Stim gene.

As used herein, the term "heterologous proteins" refers to proteins that are not naturally expressed in a cell. Such "heterologous proteins" are expressed from a "heterologous nucleic acid" incorporated into the cell as a "transgene".

As used herein, the term "functional fragment" refers to any subject polypeptide having an amino acid residue sequence shorter than that of a polypeptide whose amino acid residue sequence is described herein. A "functional fragment" of STIM 1 or STIM2 protein is shorten or truncated, yet is capable of sensing $Ca^{2+}$ fluxes, potentiaition changes, and/or the activation of CRAC current. The "functional fragment" polypeptide can have N-terminus or C-terminus truncations and/or also internal deletions.

As used herein, the term "subject" refers to a mammal, preferably a human.

As used herein, "identity" means the percentage of identical nucleotide or amino acid residues at corresponding positions in two or more sequences when the sequences are aligned to maximize sequence matching, i.e., taking into account gaps and insertions. Identity can be readily calculated by known methods, including but not limited to those described in (Computational Molecular Biology, Lesk, A. M., ea., Oxford University Press, New York, 1988; Biocomputing: Informatics and - 14 Genome Projects, Smith, D. W., ea., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988)). Methods to determine identity are designed to give the largest match between the sequences tested. Moreover, methods to determine identity are codified in publicly available computer programs such as BLASTP.

The terms "identical" or percent "identity", in the context of two or more nucleic acids or polypeptide sequences, refers to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region (e.g., nucleotide sequence encoding an antibody described herein or amino acid sequence of an antibody described herein), when compared and aligned for maximum correspondence over a comparison window or designated region) as measured using a BLAST or BLAST 2.0 sequence comparison algorithms with default parameters described below, or by manual alignment and visual inspection. Such sequences are then said to be "substantially identical." This term also refers to, or can be applied to, the compliment of a test sequence. The term also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence can be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2: 482, 1981, by the homology alignment algorithm of Needleman & Wunsch, J. Mol. Biol. 48: 443, 1970, by the search for similarity method of Pearson & Lipman, Proc. Nat'1. Acad. Sci. USA 85: 2444, 1988, by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Current Protocols in Molecular Biology, Ausubel et al., eds. 1995 supplement)).

Programs for searching for alignments are well known in the art, e.g., BLAST and the like. For example, if the target species is human, a source of such amino acid sequences or gene sequences (germline or rearranged antibody sequences) can be found in any suitable reference database such as Genbank, the NCBI protein databank (http://ncbi.nlm.nih.gov/BLAST/), VBASE, a database of human antibody genes (http://www.mrc-cpe.cam.ac.uk/imt-doc), and the Kabat database of immunoglobulins (http://www.immuno.bme.nwu.edu) or translated products thereof. If the alignments are done based on the nucleotide sequences, then the selected genes should be analyzed to determine which genes of that subset have the closest amino acid homology to the originating species antibody. It is contemplated that amino acid sequences or gene sequences which approach a higher degree homology as compared to other sequences in the database can be utilized and manipulated in accordance with the procedures described herein. Moreover, amino acid sequences or genes which have lesser homology can be utilized when they encode products which, when manipulated and selected in accordance with the procedures described herein, exhibit specificity for the predetermined target antigen. In certain embodiments, an acceptable range of homology is greater than about 50%. It should be understood that target species can be other than human.

An example of algorithm that is suitable for determining percent sequence identity and sequence similarity are the BLAST and BLAST 2.0 algorithms, which are described in Altschul et al., Nuc. Acids Res. 25: 3389-3402, 1977 and Altschul et al., J. Mol. Biol. 215: 403-410, 1990, respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.pov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always>0) and N (penalty score for mismatching residues; always<0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89: 10915, 1989) alignments (B) of 50, expectation (E) of 10, M=5, N=-4, and a comparison of both strands.

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

Other than in the operating examples, or where otherwise indicated, all numbers expressing quantities of ingredients or reaction conditions used herein should be understood as modified in all instances by the term "about." The term "about" when used in connection with percentages may mean $\pm 1\%$.

The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a nonlimiting example. Thus, the abbreviation "e.g." is synonymous with the term "for example."

All patents and other publications identified are expressly incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

Unless otherwise explained, all technical and scientific terms used herein have the same meaning as commonly understood by one of ordinary skill in the art to which this disclosure belongs. Definitions of common terms in immunlogy, transgenic mouse and molecular biology can be found in The Merck Manual of Diagnosis and Therapy, 18th Edition, published by Merck Research Laboratories, 2006 (ISBN 0-911910-18-2); Robert S. Porter et al. (eds.), The Encyclopedia of Molecular Biology, published by Blackwell Science Ltd., 1994 (ISBN 0-632-02182-9); and Robert A. Meyers (ed.),

Molecular Biology and Biotechnology: a Comprehensive Desk Reference, published by VCH Publishers, Inc., 1995 (ISBN 1-56081-569-8); The ELISA guidebook (Methods in molecular biology 149) by Crowther J. R. (2000); Fundamentals of RIA and Other Ligand Assays by Jeffrey Travis, 1979, Scientific Newsletters; Immunology by Werner Luttmann, published by Elsevier, 2006. Definitions of common terms in molecular biology may be found in Benjamin Lewin, Genes IX, published by Jones & Bartlett Publishing, 2007 (ISBN-13: 9780763740634); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

Unless otherwise stated, the present invention was performed using standard procedures, as described, for example in Maniatis et al., Molecular Cloning: A Laboratory Manual, Cold Spring Harbor Laboratory Press, Cold Spring Harbor, N.Y., USA (1982); Sambrook et al., Molecular Cloning: A Laboratory Manual (2 ed.), Cold Spring Harbor Laboratory Press, Cold Spring Harbor, N.Y., USA (1989); Davis et al., Basic Methods in Molecular Biology, Elsevier Science Publishing, Inc., New York, USA (1986); or Methods in Enzymology: Guide to Molecular Cloning Techniques Vol. 152, S. L. Berger and A. R. Kimmerl Eds., Academic Press Inc., San Diego, USA (1987), Current Protocols in Molecular Biology (CPMB) (Fred M. Ausubel, et al. ed., John Wiley and Sons, Inc.), Current Protocols in Protein Science (CPPS) (John E. Coligan, et. al., ed., John Wiley and Sons, Inc.) and Current Protocols in Immunology (CPI) (John E. Coligan, et. al., ed. John Wiley and Sons, Inc.), Current Protocols in Cell Biology (CPCB) (Juan S. Bonifacino et. al. ed., John Wiley and Sons, Inc.), Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998) which are all incorporated by reference herein in their entireties.

The present invention provides knockout transgenic mice that are lacking functional STIM 1, STIM 2, or STIM 1 and STIM 2 proteins. The STIM-deficient knockout mammal described herein provides a source of tissues, cells, and cell lines that are useful to practice (1) methods for the identification and/or evaluation of agents for their ability to affect $Ca^{2+}$ fluxes and $Ca^{2+}$ signaling in cells, such as T lymphocytes, in which $Ca^{2+}$ serves an important function in T cell proliferation, activation, and sustained immune response; (2) methods for evaluating the mode of action of an agent that modulate intracellular calcium fluxes; (3) methods for studying the cellular functions of a STIM protein or a mutant STIM protein in the absence of any other STIM homologues; (4) methods of studying the effects and/or efficacy of a STIM inhibitor or a STIM modulator; (5) methods for screening a STIM inhibitor for side effects or toxicity resulting from the inhibitor's action on a target(s) other than STIM; (6) methods for the identification of agents (e.g., therapeutic agents) which inhibit STIM protein activity; (7) methods for the identification of agents which mimic STIM protein activity; and (8) methods of treating diseases or conditions associated or regulated by $Ca^{2+}$ fluxes.

A STIM-deficient knockout transgenic mouse (Stim1$^{-/-}$; Stim2$^{-/-}$; Stim1$^{-/-}$, Stim2$^{-/-}$) encompassed in the invention can serve as tools for directly identifying the physiological roles of STIM 1 and STIM 2 protein in calcium homeostasis. A conditional STIM-deficient knockout mouse also provide a model animal useful in the study of the etiology of calcium homeostasis-related diseases such as Duchenne Muscular dystrophy, Polycystic kidney disease, autosomal dominant hypocalcemia, familial hypocalciuric hypercalcemia and neonatal severe hyperparathyroidism.

Thus, encompassed in the present invention are transgenic mice that are homozygous defective in the STIM 1 gene, in which mice thereby no functional STIM 1 is produced; mice that are homozygous deficient in the STIM 2 gene, in which mice thereby no functional STIM 2 is produced; and mice that are homozygous deficient in both the STIM 1 and STIM 2 genes, in which mice produce no functional STIM 1 and STIM 2 protein. Expression of STIM proteins are typically analyzed by Western Blot analysis and functional STIM protein as determined by fluorescence $Ca^{2+}$ measurements using Fura-2/AM or Fura-2 acetoxymethylester upon stimulation of ER $Ca^{2+}$ depletion, and these methods are well known in the art. The present invention further provides mice that are heterozygous defective in a STIM 1 gene, or a STIM 2 gene, or both STIM 1 and STIM 2 genes. The mice can be used as means for reproduction of mice that are homozygous for the defect of the STIM genes, through their cross-fertilization and examination of the presence/absence of the respective STIM gene product.

In one embodiment, the transgenic mice have a conditional deletion of the STIM gene in only the CD4$^+$, CD8$^+$, or CD4$^+$/CD8$^+$ T cells. Since the STIM 1 deficient mice showed a high percentage of perinatal lethality and the STIM 2 deficient mice showed high mortality rate shortly after birth, a conditional knock-out strategy was used. Organ and tissue specific knock-outs are generated by using promoters which express the CRE recombinase gene only in the tissue or organ of interest, for example, in the thymus or bone marrow, or in the myocytes. This is accomplished by using a promoter which is active only in the tissue or organ in which the knock out the gene is desired. Conditional disruption of a STIM gene using a Cre transgene under the control of the *Cd4* enhance/promoter/silencer (CD4-Cre) allows the disruption to occur only during the double-positive (CD4$^+$ CD 8$^+$) selection stage in the thymocytes, thus normal T cell development can occur. This conditional knock-out mice provide normal developed T cells with non-functional STIM proteins.

The present invention also provides tissues of mice that are homo- or heterozygous for the defect of the STIM gene or conditional homo- or heterozygous for the defect of the STIM gene. Such tissues, for example, spleens, lymph nodes, and including the embryonic tissues of the STIM-deficient fetuses, can be used to harvest STIM-deficient cells for *in vitro* cell culture studies and for the creation of cell lines. Immortal cell lines can be made by transfecting isolated cells with the SV40 large T antigen. Other methods of generating cell lines from cells isolated from tissues are described in United States Patent No. 4,950,598, 6,103,523, 6,458,593, and WO/2002/072768, and they are hereby incorporated by reference in their entirety.

The function of a STIM protein can be determined by measuring the changes in $Ca^{2+}$ fluxes in the cells. Intracellular $Ca^{2+}$ fluxes are detected using calcium binding dyes such as Fluo 3, Indo-1, and Fura to name a few. These dyes fluorescence when they bind the $Ca^{2+}$, thus functioning as a sensor for the amount of $Ca^{2+}$ in the surrounding. The fluorescence change is monitored over time or the cells can be analyzed by flow cytometry that is well known in the art. Other methods of calcium measurement are described in A Cushing, et. al, 1999, J Neurosci Methods 90: 33-6; S Vernino, et. al., 1994, Journal of Neuroscience, 14:5514-5524; and Lajos Gergely, et. al., 1997, Clin. Diag. Lab. Immunol. 4: 70-74, and US Patent Application US 2007/0031814, and they are hereby incorporated by reference. In particular embodiments of the methods for screening for or identifying agents and molecules that modulate intracellular calcium, the methods are conducted under conditions that permit store-operated calcium entry to occur. Such conditions are described herein and are known in the art. Test agents can be contacted with a cell deficient in STIM and assayed for any modulation of intracellular calcium in said cell.

For example, in one method for detecting or monitoring store-operated transport of calcium across the plasma membrane, cells may be treated to reduce the calcium levels of intracellular calcium stores and then analyzed for evidence of ion (particularly cation, e.g., calcium) influx in response thereto. Techniques for reducing calcium levels of intracellular stores and for analyzing cells for evidence of ion (particularly cation, e.g., calcium) influx are known in the art.

In other methods, diffusible signals can be used to activate store-operated calcium entry in methods of detecting and monitoring the same. One such signal is referred to as calcium influx factor (CIF) (see, e.g., Randriamam-pita and Tsien (1993) Nature 364:809-814; Parekh et al. (1993) Nature 364:814-818; Csutora et al. (1999) Proc. Natl. Acad. Sci. U.S.A. 96:121-126), which may be a small (~<500 D) phosphate-containing anion. A CIF activity from thapsigargin-treated Jurkat cells, as well as a similar activity from calcium pump-deficient yeast, can activate calcium influx in Xenopus oocytes and in Jurkat cells. When included in the patch pipette during whole-cell patch clamp of Jur-kat cells, the extracts activate an inward current resembling ICRAC.

In other methods, electrophysiological analysis of currents across a cell-detached plasma membrane patch or an outside-out membrane vesicle may be used to detect or monitor store-operated channel currents (e.g., ICRAC)

All patents and other publications identified are expressively incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

Stromal Interacting Molecule (STIM) Proteins

STIM proteins are type I transmembrane phospho-proteins with a single transmembrane segment separating the extracellular portion from the cytosolic domain. Vertebrates have two forms of the STIM protein, STIM1 and STIM2, while invertebrates have only one genetic form, e.g., D-STIM in *Drosophila melanogaster.* Comparison of the vertebrate and invertebrate STIM proteins demonstrates a conserved genomic organization, indicating that the two STIM genes found in vertebrates likely arose from a single ancestral gene (Williams et al. (2001) Biochem. J. 357: 673-685).

Each STIM protein contains a transmembrane domain located within the first one-third to one-half of the protein which is bounded on either side by N-terminal and C-terminal portions of the protein. The N-terminal portion of STIM forms the extracellular domain that is in the cytoplasm, whereas the C-terminal portion containing the $Ca^{2+}$-binding EF hands is found in the ER lumen. The depletion of the ER ca2+ store is sensed by the $Ca^{2+}$-binding EF hands and is then transduced to the cytoplasmic domain. This eventually leads to the activation of the Orai1 of the CRAC channel, the opening of the channel and the influx of $Ca^{2+}$ from the exterior.

STIM proteins undergo post-translational modification. STIM1 and STIM2 are modified by N-linked glycosylation and phosphorylation which occurs predominantly on serine residues. Differing levels of phosphorylation of STIM2 may account for two molecular mass isoforms (approximately 105 and 115 kDa) of the protein. In contrast, the molecular mass of STIM1 is approximately 90 kDa, which decreases to about 84 kDa when N-linked glycosylation is inhibited by tunicamycin. D-STIM (an approximately 65 kDa protein), like STIM1 and STIM2, is modified by N-linked glycosylation (Williams et al. (2001) Biochem. J. 357: 673-685) as evidenced by mobility shift experiments.

Production of knockout mouse

Detailed descriptions on the productions of knockout mouse and protocols of preparation and gene targeting of ES cells, electroporation, clonal selection and more are available in the following books: Hogan, B., Beddington, R., Costantini, F. and Lacy, E. (1994) Manipulating the Mouse Embryo: A Laboratory Manual, Cold Spring Harbor Laboratory;

Porter et al., Eur. J. Biochem., vol. 218, pp. 273-281 (1993); Bradley, A. (1991) "Modifying the mammalian genome by gene targeting" Current Opinion in Biotechnology 2: 823-829; Capecchi, M., "The New Mouse Genetics: Altering the Genome by Gene Targeting," Trends in Genetics, vol. 5, No. 3, 70-76 (1989); US Patents Nos.: 6100445, 6060642, 6365796, 6747187, and 7166764, and they are hereby explicitly incorporated by reference.

Uses of the Invention

Embodiments of the invention are cells that are deficient in STIM 1 protein, deficient in STIM 2 protein, or deficient in both STIM 1 and STIM 2 proteins. The use of Cre transgene under the control of the *Cd4* enhance/promoter/ silencer (CD4-Cre) allows the disruption of the STIM gene to occur only during the double-positive (CD4$^+$ CD 8$^+$) selection stage in the thymocytes, thus normal T cell development can occur. This is an example of a cell-type specific conditionally targeted allele of *Stim*1 and/of *Stim*2. Live mice with Stim1$^{-/-}$, Stim2$^{-/-}$, or both Stim1$^{-/-}$ Stim2$^{-/-}$ T lymphocytes can be obtained with this conditional knockout strategy. The Stim1$^{-/-}$, Stim2$^{-/-}$, or both Stim1$^{-/-}$ Stim2$^{-/-}$ T lymphocytes can be isolated from the spleen or lymph nodes as described herein and differentiated and/or activated, and then cultured for use in *in vitro* studies.

In another embodiment, the embryos of the Stim1$^{-/-}$, Stim2$^{-/-}$, or both Stim1$^{-/-}$ Stim2$^{-/-}$ non-conditional knockout mouse can be used to provide Stim1$^{-/-}$, Stim2$^{-/-}$, or both Stim1$^{-/-}$ Stim2$^{-/-}$ cells for *in vitro* cell culture studies. Mid to late developmental stage knockout mouse embryos (E13-E19) can be dissected for various tissue types (eg. muscle, heart, spleen, liver etc.) and the tissue are macerated, enyzme digested (eg., trypsin or collagenase), triturated, and single cells are isolated by filtration through a fine mesh. These isolated primary cells can then be selected for specific cell type based on cell type-specific surface markers that are known in the art. General method of isolating primary cells can be found at www.tissuedissociation.com/techniques.html. For example, the isolation of mouse embryonic fibroblast as described by Hertzog PJ. 2001, Methods Mol Biol. 158:205-15; Linda C. Samuelson and Joseph M. Metzger in CSH Protocols; 2006; doi:10.1101/pdb.prot4482, as are hereby incorporated by reference. The isolated cells should be at least 95% pure, that is, 95% of the cells are of the type of cells selected for. For example, an isolated sample of CD4$^+$ cells purified from the spleen or lymph nodes should comprise at least 95% of CD4$^+$ cells.

In one embodiment, the isolated cells, for example, mouse embryonic fibroblast or CD4$^+$ cells that are Stim1$^{-/-}$, Stim2$^{-/-}$, or both Stim1$^{-/-}$ Stim2$^{-/-}$, can be immortalized to create cell lines that are Stim1$^{-/-}$, Stim2$^{-/-}$, or both Stim1$^{-/-}$ Stim2$^{-/-}$ for use in *in vitro* cell studies. Methods of immortalizing cells are described herein.

Cell lines and CD4$^+$ cells that are Stim2$^{-/-}$ can be used to study the effects and/or efficacy of Stim1 inhibitors or Stim1 modulators. Similarly, cell lines and CD4$^+$ cells that are Stim1$^{-/-}$ can be used to study the effects and/or efficacy of Stim 2 inhibitors or Stim 2 modulators. Since both Stim 1 and Stim2 contribute to the Ca$^{2+}$ influx via the SOC CRAC channels, although at different levels, the deletion of one STIM protein allows experimentation and evaluation of agents that inhibits or modulate the other STIM protein. For example, if a compound X is known to inhibit the Ca$^{2+}$ influx in a cell, then the compound X can be tested using Stim2$^{-/-}$ or Stim1$^{-/-}$ cell to evaluate if the compound X inhibit Ca$^{2+}$ influx via inhibition of STIM 1 or STIM 2 protein. Alternatively, if a compound Y is known to inhibit STIM 1, then cells that are Stim1$^{-/-}$ can be used to evaluate if the compound Y would also inhibit STIM 2 protein. As used herein, the term "inhibit" refers to the blocking, stopping, diminishing, reducing, impeding the Ca$^{2+}$ sensing, Ca$^{2+}$ binding, Orai1 activating activity, Ca$^{2+}$ influx promoting activity of STIM protein and/or Ca$^{2+}$ mediated cytokine expression in a cell. Such cells can be a lymphocyte, a T-cell, a regulatory T cell, or an embryonic fibroblast. There should be at least a 5% reduction in the amount of Ca$^{2+}$ influx in the presence of an inhibitor compared to the Ca$^{2+}$ influx in the absence of any inhibitor. The method comprise contacting a STIM inhibitor with a STIM deficient cell, assessing the Ca$^{2+}$ influx said cell, determining that said STIM inhibitor is effective when there is a reduction in Ca$^{2+}$ influx observed in the treated cell.

Cell lines and CD4$^+$ cells that are Stim1$^{-/-}$, Stim2$^{-/-}$, or both Stim1$^{-/-}$ Stim2$^{-/-}$ can be used to study agents that modulate the intracellular Ca$^{2+}$ content. The agent can modulate the intracellular Ca$^{2+}$ content through the STIM protein, hence via a STIM-dependent pathway, or through some other mechanism or pathway. Cell lines and CD4$^+$ cells that are both Stim1$^{-/-}$ and Stim2$^{-/-}$ can be used to determine whether the agent is modulating Ca$^{2+}$ by affecting a STIM dependent pathway or some other pathway. The method comprise contacting an agent with a Stim 1$^{-/-}$ Stim 2$^{-/-}$ cell, assessing the Ca$^{2+}$ influx said cell, determining that said agent is modulating intracellular Ca$^{2+}$ through its effects on a STIM protein when there is no significant change in Ca$^{2+}$ influx observed in the treated and untreated Stim 1$^{-/-}$ Stim 2$^{-/-}$ cell.

Cell lines and CD4$^+$ cells that are both Stim1$^{-/-}$ and Stim2$^{-/-}$ can be used to study the cellular functions of individual STIM protein or mutant STIM protein, for example, mutants STIM can have defects in Ca$^{2+}$ binding, Ca$^{2+}$ sensing, or activating the CRAC channels. Construction of expression vectors and mutations of STIM genes are well known in the art. Genes encoding recombinant wild type STIM protein that are tagged or tagged mutant STIM proteins can be introduced into cells that are Stim1$^{-/-}$, Stim2$^{-/-}$, or both Stim1$^{-/-}$ Stim2$^{-/-}$, depending on the STIM protein to be studied. The functions of the individual domains of the STIM proteins can be studied on a STIM null background (ie. both Stim1$^{-/-}$ Stim2$^{-/-}$ cells) and assayed for Ca$^{2+}$ fluxes and/or interaction with Orai1. The tagging of a STIM protein, for example, by green fluorescent protein (GFP) or myc or HA, is useful for locating and detecting the STIM protein and aid in protein function analyses during the experiment.

Ca$^{2+}$-mediated cytokine expression in a cell is dependent on sustained Ca$^{2+}$ entry, and sustained Ca$^{2+}$ entry

is through store-operated $Ca^{2+}$ release-activated $Ca^{2+}$ (CRAC) channels, an essential signal for lymphocyte activation and proliferation. The activation of CRAC current is initiated by STIM proteins. Hence, the specific inhibition of Stim2 but not Stim1 does not profoundly change the store-operated $Ca^{2+}$ entry in the cell, whereas the specific inhibition of Stim1 but not Stim2 does.

In one embodiment, provided herein is a method for inhibiting $Ca^{2+}$-mediated cytokine expression in a cell without producing a profound reduction in store-operated $Ca^{2+}$ entry in the cell comprising contacting the cell with a selective Stim2 inhibitor in an amount effective to inhibit Ca2+-mediated cytokine expression in the cell. In one embodiment, the cell is a lymphocyte. In another embodiment, the cell is a T-cell. In another embodiment, the T cell is a regulatory T cell. In another embodiment, the selective Stim2 inhibitor selectively inhibits Stim2 relative to Stim1 in a cell that can be a lymphocyte, a T-cell, or a regulatory T cell. In one embodiment, the $Ca^{2+}$-mediated cytokine expressed in a cell cytokine where there is no profound reduction in store-operated $Ca^{2+}$ entry in the cell is selected from IL-2, IL-4 and IFN-gamma and the cell is a lymphocyte, a T-cell, or a regulatory T cell. In one embodiment, no profound reduction in store-operated $Ca^{2+}$ entry refers to no more than 2% reduction in store-operated $Ca^{2+}$ entry in the presence of the inhibitor compared to the control (in the absence of any inhibitor).

In one embodiment, provided herein as a method for inhibiting $Ca^{2+}$-mediated cytokine expression in a cell and producing a profound reduction in store-operated $Ca^{2+}$ entry in the cell comprising contacting the cell with a selective Stim1 inhibitor in an amount effective to inhibit $Ca^{2+}$-mediated cytokine expression in a cell and produces a profound reduction in store-operated $Ca^{2+}$ entry in the cell. This cell can be a lymphocyte, a T-cell, or a regulatory T cell. In one embodiment, the selective Stim1 inhibitor selectively inhibits Stim1 relative to Stim2 in a cell that can be a lymphocyte, a T-cell, or a regulatory T cell for the purpose of for inhibiting $Ca^{2+}$-mediated cytokine expression in a cell and producing a profound reduction in store-operated $Ca^{2+}$ entry in the cell. In one embodiment, the $Ca^{2+}$-mediated cytokine expressed in a cell where there is a profound reduction in store-operated $Ca^{2+}$ entry in the cell is selected from IL-2, IL-4 and IFN-gamma and the cell is a lymphocyte, a T-cell, or a regulatory T cell.

In one embodiment provided herein is a method for inhibiting $Ca^{2+}$-mediated cytokine expression in a cell and producing a profound reduction in store-operated $Ca^{2+}$ entry in the cell comprising contacting the cell with an inhibitor of Stim1 and an inhibitor of Stim2, wherein the amount of the inhibitors is effective to inhibit $Ca^{2+}$-mediated cytokine expression in the cell. This cell can be a lymphocyte, a T-cell, or a regulatory T cell. In one embodiment, the inhibitor of Stim1 and the inhibitor of Stim2 have the same chemical structure. In one embodiment, the $Ca^{2+}$-mediated cytokine expressed in a cell where there is a profound reduction in store-operated $Ca^{2+}$ entry in the cell is selected from IL-2, IL-4 and IFN-gamma and the cell is a lymphocyte, a T-cell, or a regulatory T cell.

In one embodiment provided herein is a method for inhibiting $Ca^{2+}$-mediated cytokine expression in a cell and producing a profound reduction in store-operated $Ca^{2+}$ entry in the cell comprising contacting the cell with an inhibitor of Stim1 and an inhibitor of Stim2, wherein the amount of the inhibitors is effective to inhibit $Ca^{2+}$-mediated cytokine expression in the cell, wherein the cell is regulatory T cell is in a subject with a tumor and the amount of the inhibitors is effective for increasing an immune response against the tumor.

Store-operated $Ca^{2+}$ entry can be determined by methods of measuring and monitoring $Ca^{2+}$ influxes described herein and other methods known in the art such as those described in U. S. Pat. Publication No. 2007/0031814 which is hereby incorporated by reference in its entirety.

Methods of determining $Ca^{2+}$-mediated cytokine expression such as IL-2, IL-4 and IFN-gamma include but are not limited to immunostaining with specific antibodies and FAC sorting as described herein, ELISA and quantitative real-time PCR.

In one embodiment, provided herein is a method of identifying a test agent that inhibits $Ca^{2+}$-mediated cytokine expression in a cell without producing a profound reduction in store-operated $Ca^{2+}$ entry in the cell, comprising: (a) contacting at least one test agent with a recombinant cell that comprises a heterologous nucleic acid encoding a STIM2 protein or a functional fragment thereof, wherein the heterologous STIM2 protein or the functional fragment thereof comprises an amino acid sequence at least 80% identical to a human STIM2 protein; (b) measuring $Ca^{2+}$-mediated cytokine expression in the cell; and, (c) measuring changes in ion fluxes or electrical current or membrane potential across the cell membrane, detecting changes in a fluorescence signal from the cell, detecting changes in a luminescence signal from the cell, or measuring changes in membrane potential of the cell. This cell used in the method of identifying a test agent described herein can be a lymphocyte, a T-cell, or a regulatory T cell. In one embodiment, the $Ca^{2+}$-mediated cytokine expressed in a cell is selected from IL-2, IL-4 and IFN-gamma and the cell is a lymphocyte, a T-cell, or a regulatory T cell.

Method of measuring ion fluxes electrical current or membrane potential across the cell membrane are known to one skilled in the art and are also described herein.

In another embodiment, provided herein is a method of identifying a test agent that increases an immune response against a tumor in a subject, comprising:

(a) contacting at least one test agent with a recombinant cell that comprises a heterologous nucleic acid encoding a STIM1 protein or a functional fragment thereof, and a STIM2 protein or a functional fragment thereof, wherein the heterologous STIM1 protein or functional fragment thereof comprises an amino acid sequence at least 80% identical to a human STIM1 protein and the heterologous STIM2 protein or the functional fragment thereof comprises an amino acid sequence at least 80% identical to a human STIM2 protein; and measuring changes in ion fluxes or electrical current or membrane potential across the cell membrane, detecting changes in a fluorescence signal from the cell, detecting changes in a luminescence signal from the cell, or measuring changes in membrane potential of the cell; (b) contacting the test agent with an isolated form of the heterologous STIM1 protein; and measuring the binding of the test agent to the isolated heterologous STIM1 protein; and (c) contacting the test agent with an isolated form of the heterologous STIM2 protein; and measuring the binding of the test agent to the isolated heterologous STIM2 protein. This cell used in the method of identifying a test agent that increases an immune response against a tumor described herein can be a lymphocyte, a T-cell, or a regulatory T cell.

As used herein, the term "a recombinant cell" refers to a cell that has a transgene incorporated into its naturally occurring genome. "A recombinant cell" also refers to an isolated cell derived from a non-human transgenic animal having a cell-type specific conditionally targeted allele of *Stim*1 and/or *Stim*2 as described herein. Such a "a recombinant cell" can be a lymphocyte, a T-cell, a regulatory T cell, an embryonic fibroblast, or an immortalized cell line thereof.

Recombinant cells expressing heterologous STIM protein or functional fragments thereof comprising an amino acid sequence of at least 80% identical to a human STIM protein, types of cells suitable for the cell-based assays, i.e. cells having components of signaling and messenger systems that can effect release if calcium ion from intracellular stores and/or expresses $Ca^{2+}$-mediated cytokines, and methods of measuring changes in ion fluxes or electrical current or membrane potential across the cell membrane, detecting changes in a fluorescence signal from the cell, detecting changes in a luminescence signal from the cell, or measuring changes in membrane potential of the cell are found and/or can be performed according to those described in U. S. Pat. Publication No. 2007/0031814 which is hereby incorporated by reference in its entirety.

In one embodiment, the invention provides a method for screening for agents modulating intracellular calcium fluxes that does not involved a STIM protein, the method comprising the use of a cell that is deficient in Stim protein. The method comprises contacting a Stim-deficient cell with an agent, assessing the effects of the agent on $Ca^{2+}$ influx in said Stim-deficient cell, identifying said agent as an agent that modulate intracellular $Ca^{2+}$ if it has an effect on the intracellular $Ca^{2+}$. An effect on the intracellular $Ca^{2+}$ can be any changes in the intracellular $Ca^{2+}$ content or fluxes. The Stim-deficient cell used can be Stim1$^{-/-}$, Stim2$^{-/-}$, or both Stim1$^{-/-}$ Stim2$^{-/-}$. An agent identified as such can be useful in the development of therapeutics for calcium homeostasis-related diseases.

In one embodiment, the invention provides a method for evaluating the mode of action of an agent that modulate intracellular calcium fluxes via a STIM-dependent pathway comprising the use of a cell that is deficient in a Stim protein. The Stim-deficient cell used can be Stim1$^{-/}$, Stim2$^{-/-}$, or both Stim1$^{-/-}$ Stim2$^{-/-}$. The method comprise contacting a Stim-deficient cell with an agent, assessing the effects of the agent on $Ca^{2+}$ influx in said Stim-deficient cell, comparing with the effects of said agent on the $Ca^{2+}$ influx in non Stim-deficient cells, and determining that said agent as an agent that modulate intracellular $Ca^{2+}$ via a Stim-dependent pathway if it has a reduction of at least 5% in the effects on the intracellular $Ca^{2+}$ on said Stim-deficient cell. For example, when Stim1 deficient cells are used to evaluated an agent Z, the agent Z is identified as an agent that modulates the $Ca^{2+}$ via the Stim1-dependent pathway when the effects of the $Ca^{2+}$ influx is reduced by 30% in the Stim1 deficient cells compared to cells that are expressing normal Stim1 levels. Similarly, when Stim2 deficient cells are used to evaluated an agent P, the agent P is identified as an agent that modulates the $Ca^{2+}$ influx via the Stim2-dependent pathway when the effects of the $Ca^{2+}$ influx is reduced by 30% in the Stim2 deficient cells compared to cells that are expressing normal Stim 2 levels.

In one embodiment, the transgenic animals and cells derived therefrom , also can be used to screen STIM inhibitors for side effects or toxicity resulting from the inhibitor's action on a target(s) other than STIM itself (e g, other STIM-like proteins). For example, an STIM inhibitor is administered to a conditional knockout mouse homozygous for STIM1, or STIM2 or STIM1/STIM2 and the resulting effects are monitored to evaluate side effects or toxicity of the inhibitor. Since the animal lacks the normal target of the respective STIM inhibitor in target tissues/cells, an effect observed upon administration of the inhibitor to the STIM1$^{-/-}$, STIM2$^{-/-}$, or STIM$^{-/-}$ and STIM2$^{-/-}$ mouse can be attributed to a side effect of the STIM inhibitor on another target(s) or to adverse effects of inhibiting STIM protein function in other tissue and cells. Similarly, cells deficient on STIM proteins can be used to test for side effects or toxicity of the STIM inhibitors on other cellular functions. Accordingly, the transgenic animals, tissues, cells, and cell lines of the invention are useful for distinguishing these side effects from the direct effects of the inhibitor on STIM activity.

In one embodiment, the invention provides a method for the identification of agents (e.g., therapeutic agents) which inhibit a specific STIM protein activity comprising the use of a cell deficient in STIM 1, or STIM 2 protein. A Stim 1$^{-/-}$ cell is useful for screening and identifying agents that inhibit STIM 2 specifically and therefore agents that modulate intracellular $Ca^{2+}$ fluxes via a STIM 2 -dependent pathway. Likewise, a Stim 2$^{-/-}$ cell is useful for screening and identifying agents that inhibit STIM 1 specifically and therefore agents that modulate intracellular $Ca^{2+}$ fluxes via a STIM 1-dependent pathway. The method comprises contacting a Stim-deficient cell with an agent, assessing the effects of the agent on $Ca^{2+}$ influx in the Stim-deficient cell, comparing with the effects of the agent on the $Ca^{2+}$ influx in non Stim-deficient cells, and determining that the agent is an agent that inhibit the respective STIM if it has a reduction of at least 5% in the effects on the intracellular $Ca^{2+}$ on the Stim-deficient cell.

In another embodiment, the invention provides a method for the identification of agents which mimic STIM protein activity comprising the use of a cell deficient in STIM protein. A Stim 1$^{-/-}$ cell is useful for screening and identifying agents that mimics STIM 1 specifically and a Stim 2$^{-/-}$ cell is useful for screening and identifying agents that inhibit STIM 2 specifically. The method comprises contacting a Stim-deficient cell with an agent, assessing the effects of the agent on $Ca^{2+}$ influx in the Stim-deficient cell, comparing with the effects of the agent on the $Ca^{2+}$ influx in control (no treated Stim-deficient cells), and determining that the agent mimics STIM activity if it cause an increase of at least 5% in the intracellular $Ca^{2+}$ in the Stim-deficient cell over that of control.

The test agents or inhibitors include, but are not limited to, biomolecules, including, but not limited to, amino acids, peptides, polypeptides, peptiomimetics, nucleotides, nucleic acids (including DNA, cDNA, RNA, antisense RNA and any double- or single-stranded forms of nucleic acids and derivatives and structural analogs thereof), polynucleotides, saccharides, fatty acids, steroids, carbohydrates, lipids, lipoproteins and glycoproteins. Such biomolecules can be substantially purified, or can be present in a mixture, such as a cell extract or supernate. Test agents further include synthetic or natural chemical compounds, such as simple or complex organic molecules, metal-containing compounds and inorganic ions. Also included are pharmacological compounds, which optionally can be subjected to directed or random chemical modifications, such as acylation, alkylation, esterification, amidation, etc., to produce structural analogs.

Test agents suitable for use in the methods can optionally be contained in compound libraries. Methods for producing compound libraries by random or directed synthesis of a wide variety of organic compounds and biomolecules are known in the art, and include expression of randomized oligonucleotides and oligopeptides. Methods of producing natural compounds in the form of bacterial, fungal, plant and animal extracts are also known in the art. Additionally, synthetically produced or natural compounds and compound libraries can be readily modified through conventional chemical, physical and biochemical means to produce combinatorial libraries. Compound libraries are also available from commercial sources.

Test agents identified using methods provided herein can also be used in connection with treatment of malignancies, including, but not limited to, malignancies of lymphoreticular origin, bladder cancer, breast cancer, colon cancer, endometrial cancer, head and neck cancer, lung cancer, melanoma, ovarian cancer, prostate cancer and rectal cancer. Store-operated calcium entry can play an important role in cell proliferation in cancer cells (Weiss et al. (2001) International Journal of Cancer 92 (6):877-882). T regulatory cells can limit the immune response against tumors. The experiments described herein show that T regulatory cells are more sensitive than other T cells to simultaneous loss (deletion) of STIM1 and STIM2. Test agents identified according to methods described herein can be useful in treating cancer by increasing the immune response against the tumor, by way of reducing the amount of regulatory T cells. As used herein, the term "tumor" means a mass of tissue growth resulting from an abnormal proliferation of tissues.

The present invention can be defined in any of the following numbered paragraphs:

**[A]** A method for inhibiting $Ca^{2+}$-mediated cytokine expression in a cell without producing a profound reduction in store-operated $Ca^{2+}$ entry in the cell comprising contacting the cell with a selective Stim2 inhibitor in an amount effective to inhibit $Ca^{2+}$-mediated cytokine expression in the cell.

**[B]** The method of paragraph [A], wherein the cell is a lymphocyte.

**[C]** The method of paragraph [B], wherein the cell is a T-cell.

**[D]** The method of paragraph [C], wherein the T cell is a regulatory T cell.

**[E]** The method of any of the paragraphs [A]-[D], wherein the selective Stim2 inhibitor selectively inhibits Stim2 relative to Stim1.

**[F]** The method of any of the paragraphs [A]-[E], wherein the cytokine is selected from IL-2, IL-4 and IFN-gamma.

**[G]** A method for inhibiting $Ca^{2+}$-mediated cytokine expression in a cell and producing a profound reduction in store-operated $Ca^{2+}$ entry in the cell comprising contacting the cell with a selective Stim1 inhibitor in an amount effective to inhibit $Ca^{2+}$-mediated cytokine expression in a cell and produces a profound reduction in store-operated $Ca^{2+}$ entry in the cell.

**[H]** The method of paragraph [G], wherein the cell is a lymphocyte.

**[I]** The method of paragraph [H], wherein the cell is a T-cell.

**[J]** The method of paragraph [I], wherein the T cell is a regulatory T cell.

**[K]** The method of any of the paragraphs [G]-[I], wherein the selective Stim1 inhibitor selectively inhibits Stim1 relative to Stim2.

**[L]** The method of any of paragraphs [G]-[K], wherein the cytokine is selected from IL-2, IL-4 and IFN-gamma.

**[M]** A method for inhibiting $Ca^{2+}$-mediated cytokine expression in a cell and producing a profound reduction in store-operated $Ca^{2+}$ entry in the cell comprising contacting the cell with a an inhibitor of Stim1 and an inhibitor of Stim2, wherein the amount of the inhibitors is effective to inhibit $Ca^{2+}$-mediated cytokine expression in the cell.

**[N]** The method of paragraph [M], wherein the cell is a lymphocyte.

**[O]** The method of paragraph [O], wherein the cell is a T-cell.

**[P]** The method of paragraph [P], wherein the T cell is a regulatory T cell.

**[Q]** The method of any of paragraphs [M]-[P], wherein the inhibitor of Stim1 and the inhibitor of Stim2 have the same chemical structure.

**[R]** The method of any of paragraphs [M]-[Q], wherein the cytokine is selected from IL-2, IL-4 and IFN-gamma.

**[S]** The method of paragraph [P], wherein the regulatory T cell is in a subject with a tumor and the amount of the inhibitors is effective for increasing an immune response against the tumor.

**[T]** A method of identifying a test agent that inhibits $Ca^{2+}$-mediated cytokine expression in a cell without producing a profound reduction in store-operated $Ca^{2+}$ entry in the cell, comprising: (a) contacting at least one test agent with a recombinant cell that comprises a heterologous nucleic acid encoding a STIM2 protein or a functional fragment thereof, wherein the heterologous STIM2 protein or the functional fragment thereof comprises an amino acid sequence at least 80% identical to a human STIM2 protein; (b) measuring $Ca^{2+}$-mediated cytokine expression in the cell; and (c) measuring changes in ion fluxes or electrical current or membrane potential across the cell membrane, detecting changes in a fluorescence signal from the cell, detecting changes in a luminescence signal from the cell, or measuring changes in membrane potential of the cell.

**[U]** The method of paragraph [T], wherein the cell is a T cell.

**[V]** The method of paragraph [T], wherein the cytokine is selected from IL-2, IL-4 and IFN-gamma.

**[W]** A method of identifying a test agent that increases an immune response against a tumor in a subject, comprising:

(a) contacting at least one test agent with a recombinant cell that comprises a heterologous nucleic acid encoding a STIM1 protein or a functional fragment thereof, and a STIM2 protein or a functional fragment thereof, wherein the heterologous STIM1 protein or functional fragment thereof comprises an amino acid sequence at least 80% identical to a human STIM1 protein and the heterologous STIM2 protein or the functional fragment thereof comprises an amino acid sequence at least 80% identical to a human STIM2 protein; and measuring changes in ion fluxes or electrical current or membrane potential across the cell membrane, detecting changes in a fluorescence signal from the cell, detecting changes in a luminescence signal from the cell, or measuring changes in membrane potential of the cell;

(b) contacting the test agent with an isolated form of the heterologous STIM1 protein; and measuring the binding of the test agent to the isolated heterologous STIM1 protein; and

(c) contacting the test agent with an isolated form of the heterologous STIM2 protein; and measuring the binding of the test agent to the isolated heterologous STIM2 protein.

**[X]** The method of paragraph [W], wherein the cell is a T cell..

**[Y]** A non-human transgenic animal having a cell type-specific conditionally targeted allele of Stim1 and/or Stim2.

**[Z]** The non-human transgenic animal of paragraph [Y], wherein the animal is a mouse.

**[AA]** The transgenic mouse of paragraph [Z], wherein the cell type is a T cell.

**[BB]** The transgenic mouse of paragraph [Z], wherein the cell type is a neuronal cell.

**[CC]** The transgenic mouse of paragraph [Z], wherein the cell type is a mouse embryonic fibroblast.

**[DD]** The non-human transgenic animal of any of paragraphs [Y]-[CC], wherein the targeted alleles are conditionally deleted.

**[EE]** Isolated cells derived from the non-human transgenic animal of any of paragraphs [Y]-[DD].

This invention is further illustrated by the following example which should not be construed as limiting. The contents of all references cited throughout this application, as well as the figures and table are incorporated herein by reference.

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

Other than in the operating examples, or where otherwise indicated, all numbers expressing quantities of ingredients or reaction conditions used herein should be understood as modified in all instances by the term "about." The term "about" when used in connection with percentages may mean $\pm 1\%$.

EXAMPLE

Introduction

Store-operated $Ca^{2+}$ entry via the $Ca^{2+}$ release-activated calcium (CRAC) channel is the predominant mechanism of intracellular $Ca^{2+}$ increase in stimulated immune cells (Parekh, A.B. & Putney, J.W., Physiol. Rev. 85, 757-810 (2005)). CRAC channels open after endoplasmic reticulum (ER) $Ca^{2+}$ stores are depleted by inositol trisphosphate (IP3) binding to IP3 receptors. Sustained $Ca^{2+}$ influx drives diverse functions of immune cells including T cell differentiation and cytokine expression (Lewis, R.S., Annu. Rev. Immunol. 19, 497-521 (2001); Feske, S.,et. al., Biochem. Biophys. Res. Commun. 311, 1117-1132 (2003); Gallo, E.M., et. al., Nature Immunol. 7, 25-32 (2006)). Genome-wide RNAi screens in *Drosophila* have identified two key molecules controlling CRAC channel activity, the ER $Ca^{2+}$ sensor Stim (Roos, J. et al., J. Cell Biol. 169, 435-445 (2005); Liou, J. et al., Curr. Biol. 15, 1235-1241 (2005)) and a pore subunit of the CRAC channel, Orai (Feske, S. et al., Nature 441, 179-185 (2006); Vig, M. et al., Science 312, 1220-1223 (2006); Zhang, S.L. et al.,. Proc. Natl Acad. Sci. U S A 103, 9357-9362 (2006)). *Drosophila Stim* and its mammalian homologues, *Stim1* and *Stim2* (Roos, J. et al., J. Cell Biol. 169, 435-445 (2005); Liou, J. et al., Curr. Biol. 15, 1235-1241 (2005)) are single-pass transmembrane proteins thought to sense ER $Ca^{2+}$ levels through $Ca^{2+}$-binding EF hands located in the ER lumen (Taylor, C.W., Trends Biochem. Sci. 31, 597-601 (2006); Lewis, R.S., Nature 446, 284-287 (2007); Putney, J.W., Jr., Cell Calcium (2007)). Stim1 is an established positive regulator of store-operated $Ca^{2+}$ entry (Zhang, S.L. et al., Nature 437, 902-905 (2005); Spassova, M.A. et al., Proc. Natl Acad. Sci. U S A 103, 4040-4045 (2006); Luik, R.M., et. al., J. Cell Biol. 174, 815-825 (2006); Wu, M.M., et. al., J. Cell Biol. 174, 803-813 (2006); Baba, Y. et al. Proc. Natl Acad. Sci. U S A 103, 16704-16709 (2006)), but the function of Stim2 is controversial (Roos, J. et al., J. Cell Biol. 169, 435-445 (2005); Liou, J. et al., Curr. Biol. 15, 1235-1241 (2005); Soboloff, J. et al., Curr. Biol. 16, 1465-1470 (2006); Soboloff, J. et al., J. Biol. Chem. 281, 20661-20665 (2006)). To investigate the physiological roles of *Stim1* and *Stim2,* mutant mice were generated with conditional deletion of the *Stim1* and *Stim2* genes. Here, the inventors show that Stim1 is a predominant effector of store-operated $Ca^{2+}$ entry in naive T cells and mouse embryonic fibroblasts (MEFs), and its deficiency severely impairs T cell cytokine expression. In contrast, *Stim2* has little effect on store-operated $Ca^{2+}$ entry in naive T cells, but contributes significantly to store-operated $Ca^{2+}$ entry in MEFs and to cytokine expression by differentiated T cells, in part by sustaining the late phase of NFAT nuclear localisation. Thus *Stim1* and *Stim2* are both positive regulators of $Ca^{2+}$-dependent cytokine expression in differentiated T cells; the more abundant Stim1 is essential for response initiation but modest amounts of *Stim2* have a crucial role in bolstering the function of Stim1.

Methods

Conditional gene targeting

Gene targeting of the *Stim1* and *Stim2* genes was performed by homologous recombination in Bruce-4 ES cells derived from C57BL/6 mice as previously described (Muljo, S.A. et al. J. Exp. Med. 202, 261-269 (2005)). Chimeric mice with targeted *Stim* alleles were generated by blastcyst injection of heterozygous Stim1[neo/+] or Stim2[neo/+] ES cell clones (see Fig. 9, neo = neomycin-resistance gene). Stim1-/- or Stim2-/- mice were generated by intercrossing the progeny of founder Stim$^{neo/+}$ mice after breeding to CMV-Cre (Cre deleter) transgenic mice (Schwenk, F., et. al. Nucleic Acids Res 23, 5080-5081 (1995)). To establish Stim1+/- or Stim2+/- mice without the Cre transgene, Stim+/-CMV-Cre+ mice were bred to C57BL/6 mice. To generate the conditional Stim1$^{fl/+}$ or Stim2$^{fl/+}$ alleles, founder Stim$^{neo/+}$ chimeric mice were bred to Flp deleter transgenic mice (Rodriguez, C.I. et al., Nat Genet 25, 139-140 (2000)) to remove the neomycin resistance cassette from the targeted Stim alleles. Rag1-/- and B6.Cg (Igh$^a$, Thy1.1 Gpil$^a$) mice were purchased from the Jackson laboratory. To generate mice with a T cell-specific disruption of Stim genes, CD4-Cre transgenic mice (Lee, P.P. et al. Immunity 15, 763-774 (2001)) were bred to each founder Stim$^{fl/+}$ mouse and progeny were intercrossed. All mice were maintained in specific pathogen-free barrier facilities at Harvard Medical School and were used in accordance with protocols approved by the Center for Animal Resources and Comparative Medicine of Harvard Medical School.

T cell differentiation, retroviral transductions and stimulation

T cell differentiation, retroviral transductions and stimulation

Purification of CD4$^+$ T cells from spleen and lymph nodes, induction of TH differentiation, stimulation with 10 nM PMA and 1 $\mu$M ionomycin or plate-bound anti-CD3 and anti-CD28, and assessment of cytokine production by intracellular staining and flow cytometric analysis were carried out as described previously (Ansel, K.M. et al., Nature Immunol. 5, 1251-1259 (2004)). Foxp3 expression was assessed by intracellular staining with an anti-Foxp3 (eBioscience) using the manufacturer's protocol and analyzed by flow cytometry. Retroviral transductions were performed as described previously (Wu, Y. et al., Cell 126, 375-387 (2006)). with KMV retroviral expression plasmids, either empty or containing *Stim1* or *Stim2* cDNAs followed by GFP cDNA under control of an internal ribosome entry site (IRES). Despite of their Ca$^{2+}$ influx defect, STIM1 deficient T cells upregulated CD25 (IL-2R-$\alpha$ chain) normally. Thus, because differentiating cultures were maintained in IL-2, almost equivalent cell numbers were recovered (the total cell number of differentiated STIM1-deficient cells at 7 days was 60-70% that of wild-type) and STIM-deficient cells could be retrovirally transduced with the same efficiency as wild-type cells.

Establishment of MEF cell lines

Stim1$^{-/-}$ and Stim2-/- mouse embryonic fibroblasts (MEFs) were established using standard protocols from E14.5 embryos obtained by intercrossing either Stim1+/- or Stim2+/- mice. MEFs were immortalized by retrovirally transducing them with SV40 large T antigen in a plasmid carrying the hygromycin resistance gene, followed by hygromycin selection.

Antibodies and western blotting

Cell extracts were prepared by resuspending cells in PBS, then lysing them in buffer containing 50 mM NaCl, 50 mM Tris-HCI pH 6.8, 2% SDS, 10% glycerol (final concentrations). Protein concentration was determined with the BCA Protein Reagent Kit (Pierce), after which 2-mercaptoethanol was added to a final concentration of 100 $\mu$M and the samples were boiled. Western blotting was performed according to standard protocols. STIM1 polyclonal antibodies were generated (Open Biosciences) against a C-terminal peptide of human STIM1 (CDNGSIGEETDSSPGRKKFPLKIFKKPLKK-COOH, (SEQ. ID. No. 1) where the cysteine at the N-terminus was introduced for the purpose of coupling the peptide with a carrier protein) and used at 1:2000. Affinity-purified polyclonal antibodies were generated against a C-terminal peptide of human STIM2 (CKPSKIKSLFKKKSK, (SEQ. ID. No. 2) where the cysteine at the N-terminus was introduced for the purpose of coupling the peptide with a carrier protein) and were used at 2 $\mu$g/ml. Polyclonal antibody against actin (I-19; SC-1616; Santa Cruz) was used at 1:500. Monoclonal antibody to the myc epitope tag was purified from supernatants of 9E10 hybridoma cell lines. All following fluorescent conjugated antibodies used in FACS analysis were purchased from eBioscience or BD Pharmingen. Pacific Blue-CD4 (RM4-5), FITC-CD8 (53-6.7), FITC-Thy1.1 (HIS51), FITC-IgE (R35-72), FTTC-CD11c (M1/70), PE-IL-2 (JES6-5HA), PE-IL-4 (11B11), PE-Foxp3 (FJK-16s), PE-CD19 (1D3), PE-CD125 (T21.2), PE-CD44 (IM7), PE-CD95 (Jo2), PerCP-B220 (RA3-6B2), PsrCPCy5.5-CD4 (RM4-5), PECy7-Thy1.2 (53-2.1), APC-IFN$\gamma$ (XMG1.2), APC-IL-10 (JES5-16E3), APC-TNF$\alpha$ (MP6-XT22), APC-CD25 (PC61.5), APC-TCRb (H57-597), APC-CD38 (90), APC-CD62L (MEL-14), bio-CD5 (53-7.3), bio-CD69 (H1.2F3) and PE-streptavidin.

Single cell [Ca$^{2+}$]i imaging

CD4+ T cells were isolated as described previously (Ansel, K.M. et al., Nature Immunol. 5, 1251-1259 (2004)), incubated overnight in loading medium (RPMI1640 10% FBS) and loaded with 1 $\mu$M fura-2/AM (Invitrogen) for 30 min at 22-25°C at a concentration of $1\times10^6$ cells/ml. Before measurements, T cells were attached to poly-L-lysine-coated coverslips for 15 min. For anti-CD3 stimulation, T cells were incubated with 5 $\mu$g/ml biotin conjugated anti-CD3 (clone 2C11, BD pharmingen) for 15 min at 22-25°C, and anti-CD3 crosslinking was achieved by perfusion of cells with 10 $\mu$g/ml streptavidin (Pierce). For long-term Ca$^{2+}$ imaging, differentiated CD4$^+$ T cells were loaded with 1 $\mu$M Fura-PE3, then stimulated with 10 nM PMA and 0.5 $\mu$M ionomycin, in Ringer solutions containing 2 mM Ca$^{2+}$ supplemented with 2% fetal calf serum. During image acquisition, cells were constantly perfused with buffer warmed to 37°C. Measurements of [Ca2$^+$]i were carried out and analyzed as described previously (Feske, S. et al., Nature 441, 179-185 (2006)). Ca$^{2+}$ influx rates were inferred from the maximal rate of the initial rise in intracellular Ca$^{2+}$ concentrations (d[Ca$^{2+}$]$_i$/ dt) in 0.2 - 2 mM extracellular Ca$^{2+}$, expressed as the ration 'd[Ca2+]$_i$/dt, where d[Ca2+]$_i$ is the maximum difference in [Ca$^{2+}$]$_i$ over a 20-second time interval (dt) between the re-adddition of extracellular Ca$^{2+}$ and the peak of the Ca$^{2+}$ influx response.

For each experiment, 100-150 individual T cells cells or at least 30 individual MEFs were analyzed for 340/380 ratio using Igor Pro analysis software (Wavemetrics).

NFAT1 nuclear translocation assay

CD4+ T cells were cultured in non-polarizing conditions and harvested at day 5, and then stimulated for various times with 10 nM PMA plus 1 μM ionomycin at $1 \times 10^5$ cells/well in 200 μl in 96-well plates for the indicated times, attached to poly-L-lysine-coated wells in 384-well plates (5000-8000 cells/well; 3 wells/sample) by centrifugation at 149 x G for 3 min. Cells were fixed with 3% (vol/vol) paraformaldehyde and then stained with anti-NFAT1 (purified rabbit polyclonal antibody to the 67.1 peptide of NFAT1) Ho, A.M.,et. al., J. Biol. Chem. 269, 28181-28186 (1994) and indocarbocyanine-conjugated anti-rabbit secondary antibody, and counterstained with the DNA-intercalating dye DAPI (4,6-diamidino-2-phenylindole). Images were acquired using the ImageXpress Micro automated imaging system (Molecular Devices) using a 20x objective and analyzed using the translocation application module of MetaXpress software version 6.1 (Molecular Devices). Cytoplasmic to nuclear translocation was assessed by calculating a correlation of intensity between anti-NFAT1-Cy3 staining with DAPI; T cells were scored as having nuclear NFAT1 when >90% of NFAT1-indocarbocyanine staining coincided with the fluorescence signal from the DNA intercalating dye DAPI. Each data point represents an average of at least 300 individual cells per well.

siRNA-mediated knockdown in HEK293 cells

$0.6 \times 10^6$ HEK293 cells were plated in a 6-well plate overnight and transfected the next day with siRNAs (Dharmacon, Inc., Lafayette, CO) against human STIM1 and STIM2 using lipofectamine 2000 transfection reagent (Invitrogen, Carlsbad, CA) according to the manufacturer's protocol. The transfection procedure was repeated after 48 h to increase the efficiency of knockdown. Cells were harvested for immunoblot analysis or measurements of [Ca2+]i 72 h later. To monitor the degree of knockdown, immunoblotting for STIM1 was performed 72 h after the second transfection using a polyclonal antibody against human STIM1 (a kind gift of M. Dziadek (U Auckland, New Zealand)) at a 1:1000 dilution. STIM2 transcript levels were measured by real-time RT-PCR in cells transfected with scrambled control siRNA (siC2), siSTIM1 and siSTIM2. Threshold cycles ($C_T$) for STIM2 were normalized to GAPDH housekeeping gene expression levels ($\Delta C_T$) and plotted as $0.5^{\Delta Ct} \times 10^6$ (arbitrary units). The siRNA sequences used were: STIM1: AGGUGGAGGUGCAAUAUUA (SEQ. ID. No. 3) ; STIM2#1: UAAACCUCCUGGAUCAUUA (SEQ. ID. No. 4); STIM2#2: CUUUAAGCCUCGAGAUAUA (SEQ. ID. No. 5).

Patch-clamp measurements

CD4+ T cells were cultured in non-polarizing conditions and harvested at day 5. Patch-clamp recordings were performed using an Axopatch 200 amplifier (Axon Instruments) interfaced to an ITC-18 input/output board (Instrutech) and an iMac G5 computer. Currents were filtered at 1 kHz with a 4-pole Bessel filter and sampled at 5 kHz. Recording electrodes were pulled from 100-μl pipettes, coated with Sylgard, and fire-polished to a final resistance of 2-5 MΩ. Stimulation and data acquisition and analysis were performed using in-house routines developed on the Igor Pro platform (Wavemetrics). All data were corrected for the liquid junction potential of the pipette solution relative to Ringer's in the bath (10 mV) and for leak currents collected in 20 mM [Ca2+]o + 25 μM La3+. The standard extracellular Ringer solution contained (in mM): 130 NaCl, 4.5 KCl, 20 CaCl2, 1 MgCl2, 10 D-glucose, and 5 Na-Hepes (pH 7.4). In some experiments, 2 mM CaCl2 was used in the standard extracellular solution and the NaCl concentration was raised to 150 mM. The standard divalent-free (DVF) Ringer solutions contained (in mM) 150 NaCl, 10mM tetraacetic acid, 1mM EDTA and 10 mM Hepes (pH 7.4). 25 nM charybdotoxin (Sigma) was added to all extracellular solutions to eliminate contamination from Kv1.3 channels. The standard internal solution contained (in mM) 145 mM Cs aspartate, 8 mM MgCl2, 10 BAPTA (1,2-bis(o-aminophenoxy)ethane-N,N,N',N'-tetraacetic acid) and 10 mM Cs-Hepes (pH 7.2). Averaged results are presented as the mean value ± s.e.m. Curve fitting was done by least-squares methods using built-in functions in Igor Pro 5.0. The permeability of Cs+ relative to that of Na+ was calculated from the bionic reversal potential using the relation:

$$\frac{P_{Cs}}{P_{Na}} = \frac{[Na]_o}{[Cs]_i} e^{-E_{rev}F/RT}$$

where $P_{Cs}$ and $P_{Na}$ are the permeabilities of Cs (the test ion) and Na+ respectively; [Cs]i and [Na]o are the ionic concentrations; $E_{rev}$ is the reversal potential; R is the gas constant (8.314 J K-1 mol-1), T is the absolute temperature and F is the Faraday constant (9648 C mol-1).

Hematoxylin and eosin staining

Tissues were harvested from 3-4 months old mice and fixed by 10% formalin. Hematoxylin and eosin staining was performed by standard procedure.

Purification and adoptive transfer of T_reg cells

CD4+CD25+ and CD4+CD25- T cells derived from Thy1.1+ congenic mice were sorted by FACSVantage after purification of CD4+ T cells by Dynabeads Mouse CD4 and DETACHaBEAD mouse CD4 (Invitrogen). wild-type Thy1.1+,

CD4+CD25+ or CD4+CD25- T cells (3 × 10^5) were injected intraperitoneally. into 2 week old mice. Injected mice were analyzed at 8 weeks after adoptive transfer.

Mixed bone marrow transfer

T cell-depleted bone marrow cells from Thy1.2+ DKO mice (3 × 10^6 cells) were mixed with those of Thy1.1+ congenic wild-type mice (1.5 × 10^6 cells) and injected via the retro-orbital sinus into sublethally irradiated Rag1-/- mice (450 Rads). Reconstituted mice were analyzed 10-12 weeks after bone marrow transfer.

Proliferation and in vitro suppression assays

CD4+CD25- or CD4+CD25+ T cells were positively selected with MACS CD25 microbeads (Miltenyi Biotec) after purification of CD4+ T cells. Purified CD4+CD25- T cells (2 × 10^7/ml) were incubated for 10 min at 37°C with CFSE (carboxyfluorescein diacetate succinimidyl diester) (1.25 μM). Cells were stimulated for 72 h with anti-CD3 and anti-CD28 and the number of cell divisions was assessed by flow cytometory. In vitro suppression assays were performed by co-culture of CFSE-labeled CD4+CD25- T cells (5 × 10^4) with the indicated ratios of CD4+CD25+ T cells purified from control littermates, or DKO mice, in the presence of mitomycin C-treated T cell-depleted splenocytes (5 x 10^4) and 0.3 μg/ml anti-CD3 (2C11) in round-bottom plates for 72 h at 37°C.

Example 1. Conditional ablation of Stim1 or Stim2.

As both STIM1 and STIM2 are ubiquitously expressed in vivo (Williams, R.T. et al., Biochem. J. 357, 673-685 (2001)), mice bearing loxP-flanked alleles of Stim1 and Stim2 were generated (Fig. 9). These mice were bred to a CMV-Cre deleter strain (Schwenk, F., Baron, U. & Rajewsky, K. Nucleic Acids Res 23, 5080-5081 (1995)) to examine the effects of deleting Stim1 and Stim2 in all tissues. STIM1-deficient mice on the C57BL/6 background were alive at the expected mendelian ratios at E18.5 but showed perinatal lethality, with 75% of pups born dead and most of the remaining pups dying within two days; in contrast, mice lacking STIM2 survived until 4 weeks postpartum, but showed slight growth retardation and died at 4-5 weeks of age (Table 1a and 1b). To 'rescue' the perinatal lethality of STIM1-deficient mice, these mice were crossed to the outbred ICR mouse strain. Although perinatal lethality was still high (38%), about half of the outbred STIM1-deficient offspring survived past day two with severe growth retardation, and died of unknown causes within the next 2 weeks (Table 1c).

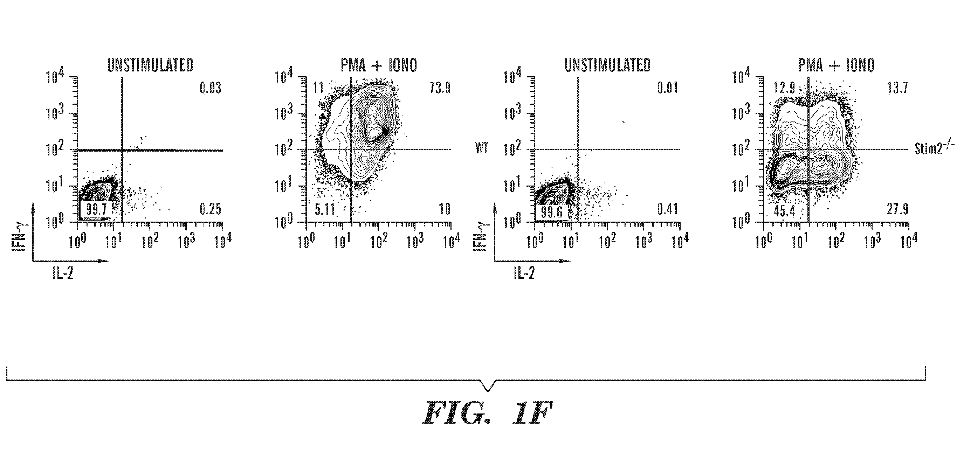

To examine the effect of STIM deficiency in T cells, mice were crossed with loxP-flanked Stim1 or Stim2 to mice expressing a Cre transgene under control of a Cd4 enhancer-promoter-silencer cassette (CD4-Cre) that causes deletion at the double-positive (CD4+CD8+) stage of thymocyte development (Lee, P.P. et al., Immunity 15, 763-774 (2001)). Thymic cellularity and T cell development appeared normal in Stim1fl/fl CD4-Cre+ and Stim2fl/fl CD4-Cre+ mice (data not shown), permitting analysis of peripheral CD4+ and CD8+ T cells (Fig. 1). Unless otherwise indicated, the T cell experiments in this paper were performed using T cells from mice in which Stim1 and/or Stim2 were conditionally deleted with CD4-Cre, whereas the fibroblast experiments were performed with mouse embryonic fibroblasts (MEFs) from Stim1fl/fl CMV-Cre+ and Stim2fl/fl CMV-Cre+ mice.

Example 2. STIM proteins regulate store-operated Ca^{2+} influx.

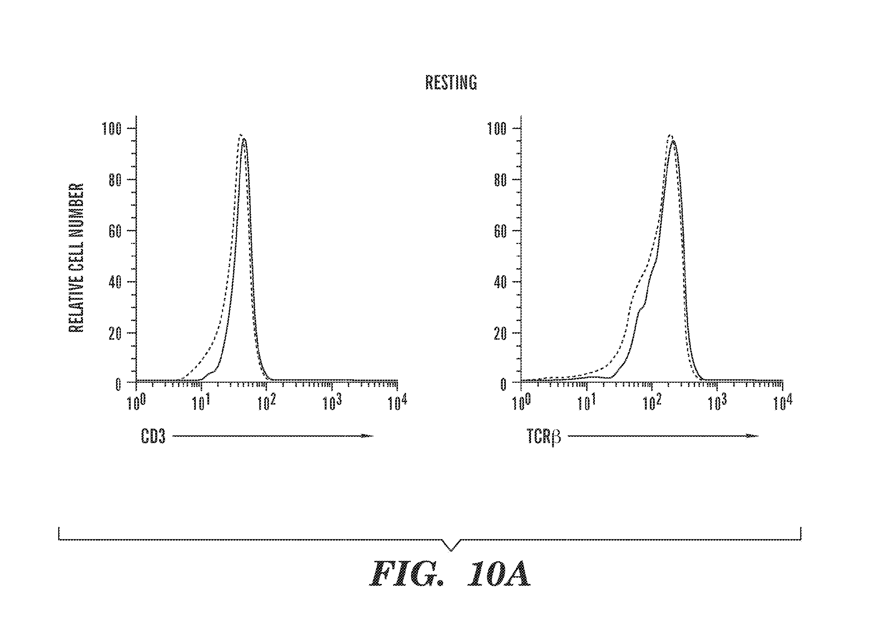

STIM1-deficient CD4+ T cells displayed almost no Ca^{2+} influx after passive depletion of ER Ca^{2+} stores with thapsigargin (TG), an inhibitor of the sarcoplasmic-endoplasmic reticulum ATPase (SERCA) pump, or after TCR crosslinking with anti-CD3 (Fig. 1a). Resting control and STIM1-deficient T cells showed similar expression of surface CD3 and TCRβ and exhibited similar depletion of ER Ca^{2+} stores and expression of the activation markers CD25 and CD69 upon stimulation with anti-CD3 or anti-CD3 and anti-CD28 (Fig. 10a-c). STIM1-deficient CD4+ T cells failed to produce IL-2 after stimulation with PMA and ionomycin (Fig. 1b) or anti-CD3 and anti-CD28 (Fig. 10d). Collectively, these results provide the first genetic evidence that STIM1 controls store-operated Ca^{2+} influx and Ca^{2+}-dependent cytokine production in primary murine T cells.

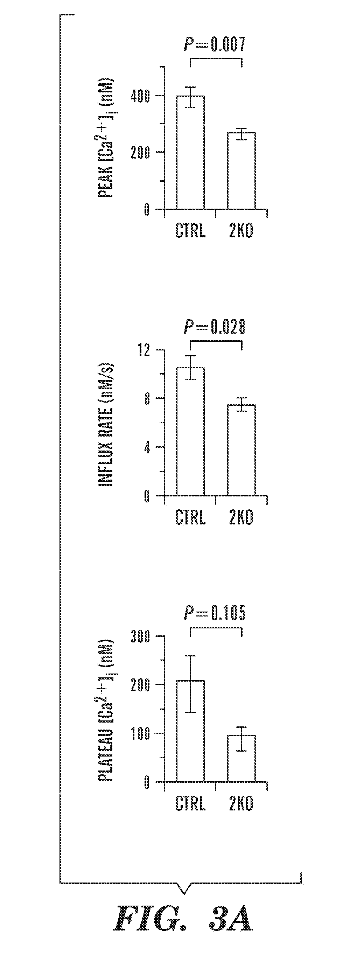

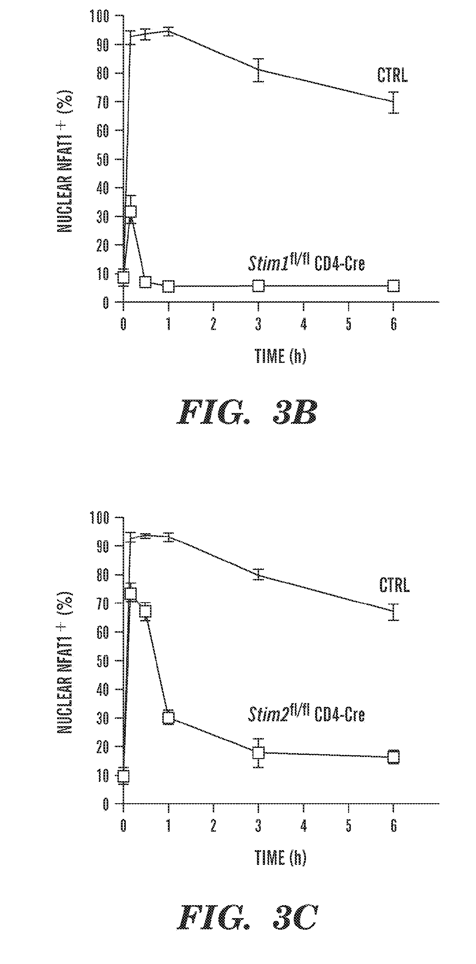

In contrast, STIM2-deficient primary CD4+ T cells obtained from Stim2fl/flCMV-Cre+ mice showed little or no impairment in Ca^{2+} influx or IL-2 production relative to control CD4+ T cells in response to treatment with thapsigargin, ionomycin or anti-CD3 (Fig. 1c,d). A possible explanation for this discrepancy between STIM1 and STIM2 is that STIM2 is expressed in much lower amounts than STIM1 in naive CD4+ T cells (Fig. 11a). Although T cell activation led to a substantial increase in STIM2 expression, which was maintained after 3-7 days of T helper type 1 (TH1) or TH2 differentiation, this increased quantity of STIM2 nevertheless amounted to only a small proportion (3-10%) of total STIM protein (Supplementary Fig. 3a,b, online). Consistent with the fact that even in differentiated T cells STIM2 constitutes a minor fraction of total STIM protein, STIM2-deficient T cells differentiated for 1 week under non-polarizing (THN) conditions showed a mild decrease in Ca^{2+} influx in response to acute low-dose thapsigargin or anti-CD3 stimulation (Fig. 1e). However, these cells displayed a considerable decrease in the ability to produce IL-2 and IFN-γ upon sustained stimulation with PMA and ionomycin (Fig. If) or anti-CD3 and anti-CD28 (Fig. 11e). Likewise, STIM2-deficient Th1 and Th2 cells showed decreased IL-2, IL-4 and IFNγ production (data not shown).

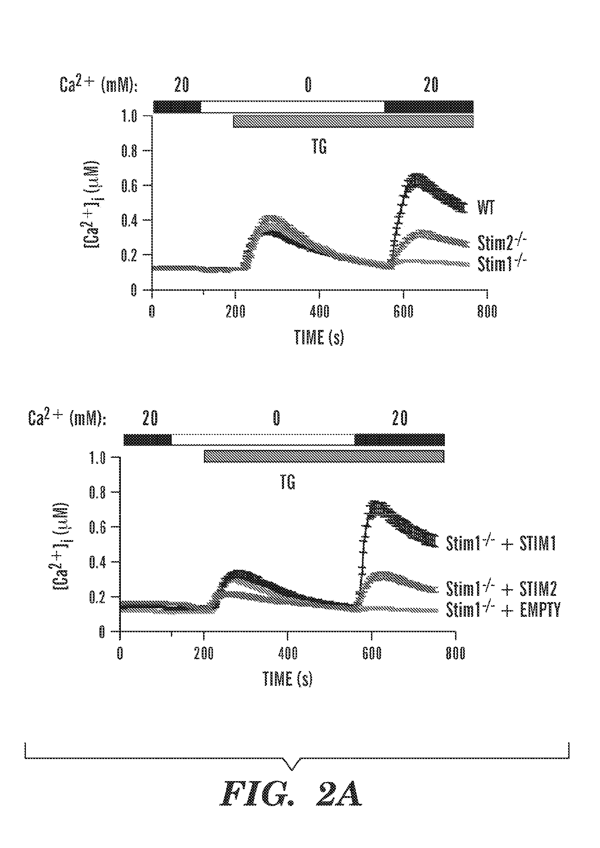

In addition, store-operated Ca^{2+} influx in MEFs from Stim1fl/fl CMV-Cre+ and Stim2fl/fl CMV-Cre+ mice were examined. It was confirmed that Ca^{2+} influx in wild-type MEFs with thapsigargin treatment was due to ER Ca^{2+} store depletion, since no Ca^{2+} influx was observed in the absence of thapsigargin treatment (data not shown). As in T cells, STIM1 deficiency abrogated Ca^{2+} influx; moreover, in MEFs STIM2 deficiency also clearly reduced Ca^{2+} influx (Fig. 2a).

Together these experiments show that STIM1 deficiency results in a complete loss of store-operated $Ca^{2+}$ entry in T cells and fibroblasts, whereas STIM2 deficiency had a lesser effect.

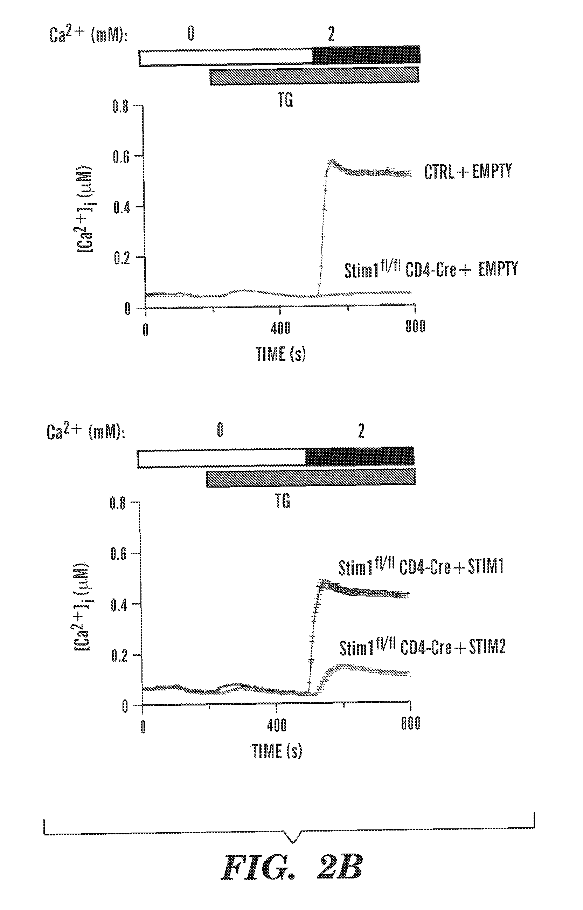

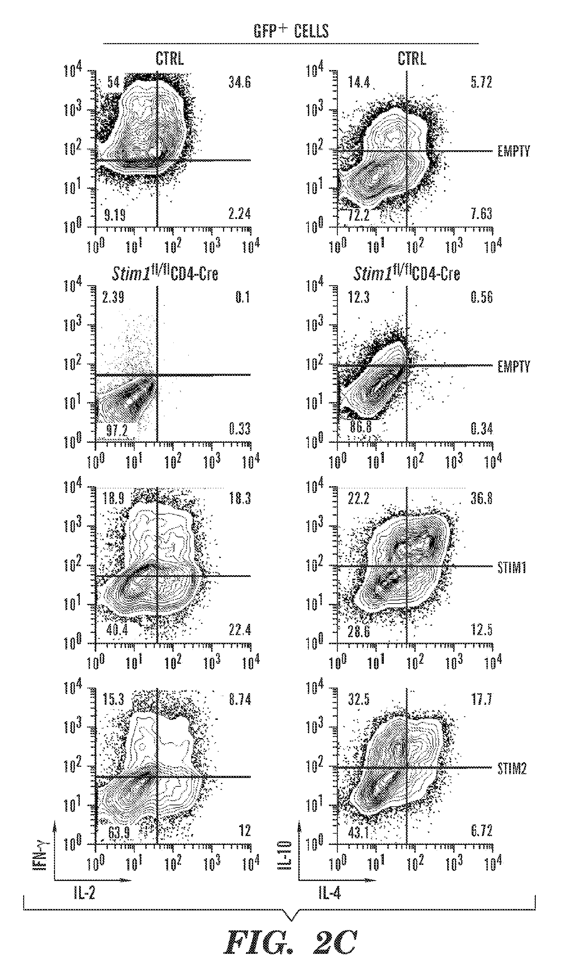

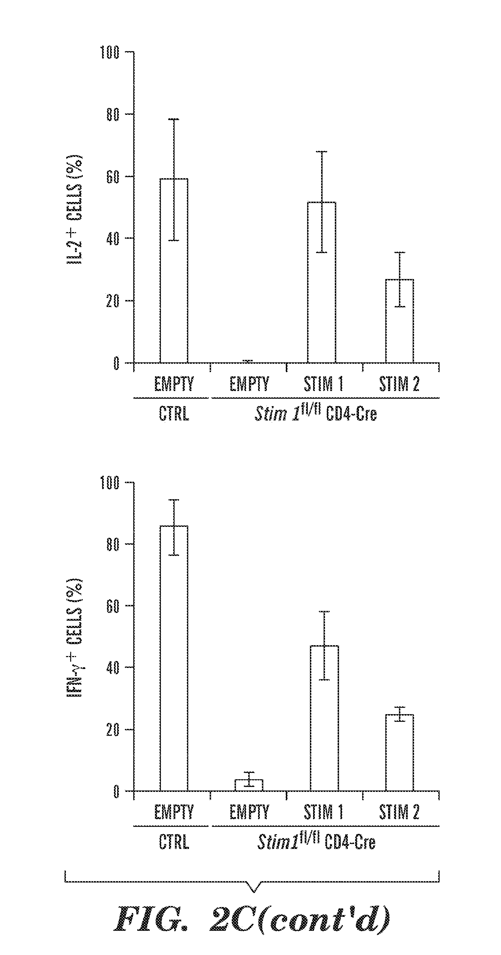

To confirm that STIM2 was a functional ER $Ca^{2+}$ sensor, STIM1-deficient T cells and MEFs were reconstituted with Myc-tagged STIM1 or STIM2, then tested $Ca^{2+}$ influx and cytokine production (Fig. 2a, b). The retroviral vectors used permitting stable low protein expression were used to avoid artifacts resulting from overexpression. The introduced STIM1 and STIM2 were expressed in similar amounts in both cell types, as shown by immunoblot analysis with anti-Myc (Fig. 11b and data not shown). STIM1 robustly reconstituted store-operated $Ca^{2+}$ influx in STIM1-deficient MEFs (Fig. 2a) and STIM1-deficient helper T cells differentiated for 1 week in nono-polarizing conditions and treated with thapsigargin (Fig. 2b); in contrast, weaker $Ca^{2+}$ influx was observed in cells reconstituted with STIM2 (Fig. 2a, b). Nevertheless, STIM2 was surprisingly effective in restoring cytokine expression to STIM1-deficient T cells stimulated with PMA and ionomycin (Fig. 2c). Together these results indicate that endogenous STIM2 is a positive regulator of $Ca^{2+}$ signaling in these two cell types, rather than an inhibitor of STIM1 function as was proposed by another study (Soboloff, J. et al., Curr. Biol. 16, 1465-1470 (2006)).

Example 3. Aborted Ca2+ entry and NFAT nuclear transport.

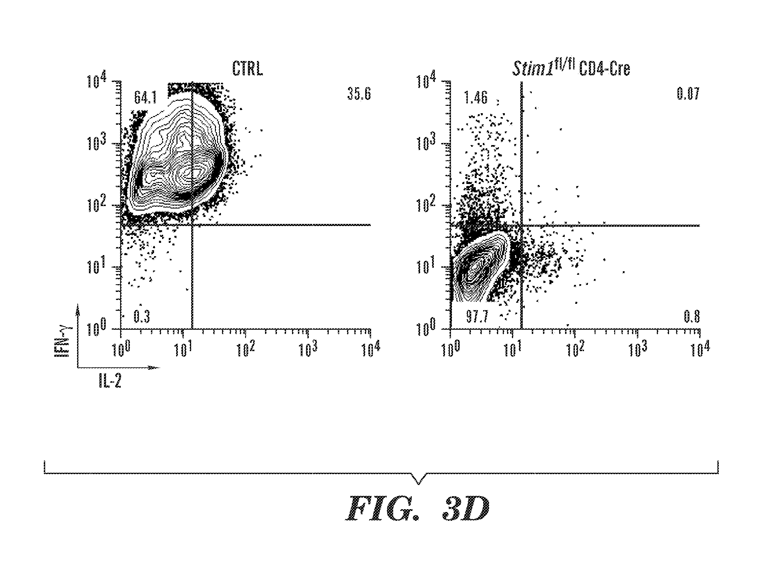

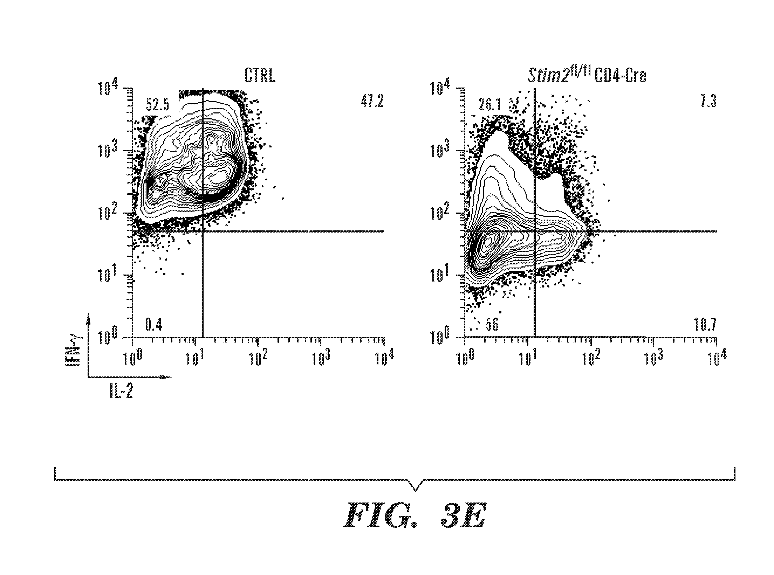

To reconcile the minor decrease in store-operated $Ca^{2+}$ influx with the relatively much lower cytokine expression observed in STIM2-deficient T cells, $Ca^{2+}$ influx were examined on a longer time-scale by loading the cells with Fura PE-3, a calcium indicator that is well retained in the cytoplasm (Vorndran, C., et. al., Biophys J 69, 2112-2124 (1995)). STIM2-deficient T cells showed decreased store-operated $Ca^{2+}$ entry compared to wild-type T cells, and attained a lower plateau of sustained intracellular free $Ca^{2+}$ concentration ($[Ca^{2+}]i$) after 20 minutes (Fig. 3a). To confirm that $Ca^{2+}$ signaling is reduced in STIM2-deficient cells, the nuclear translocation of the $Ca^{2+}$-dependent transcription factor NFAT18 were monitored. The nuclear translocation was quantified using the MetaXpress programme (data not shown) and differentiated helper T cells from wild-type, STIM1-deficient and STIM2-deficient mice for 1 week under non-polarizing conditions and then under stimulated with PMA and ionomycin in the same conditions stimulation used for the cytokine assay, so that NFAT1 nuclear translocation (Fig. 3b, c) and cytokine expression (Fig. 3d, e) could be directly compared.

The results showed unambiguously that under physiological conditions, both STIM1 and STIM2 contribute to the sustained $Ca^{2+}$ influx and NFAT nuclear translocation required for high expression of cytokine genes (Hogan, P.G., et. al., Genes Dev. 17, 2205-2232 (2003)). In STIM1-deficient helper T cells differentiated for 1 week under non-polarizing conditions, NFAT was transiently imported into the nucleus, presumably because of the transient elevation in $Ca^{2+}$ ($[Ca^{2+}]i$) that accompanies ER $Ca^{2+}$ store depletion--but NFAT was imported into the nucleus only in a fraction of cells and was rapidly re-exported (Fig. 3b). In contrast, almost as many STIM2-deficient as control cells (70-75% versus 85-95%) showed nuclear NFAT1 at 10 min, but this response was sustained in control but not STIM2-deficient cells (Fig. 3c). These results point to a major role for STIM2 in T cell signaling, and explain the substantial reduction of cytokine expression in STIM2-deficient T cells (Fig. 1f, 3e).

Example 4. CRAC current is impaired in STIM1-deficient cells.

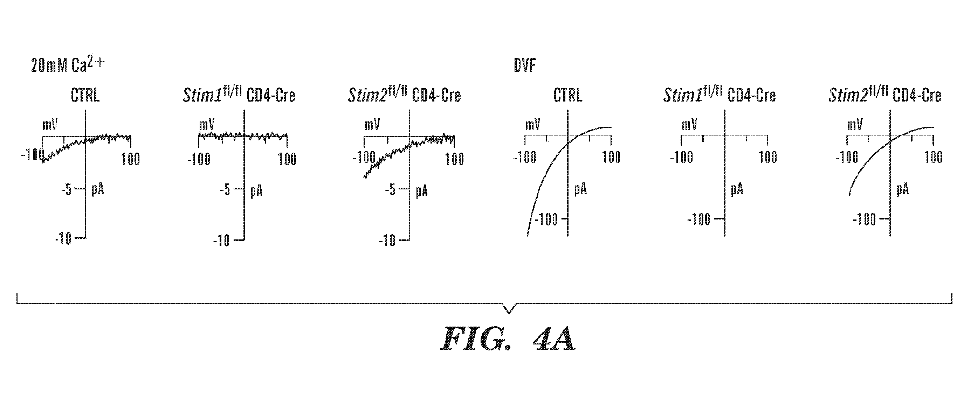

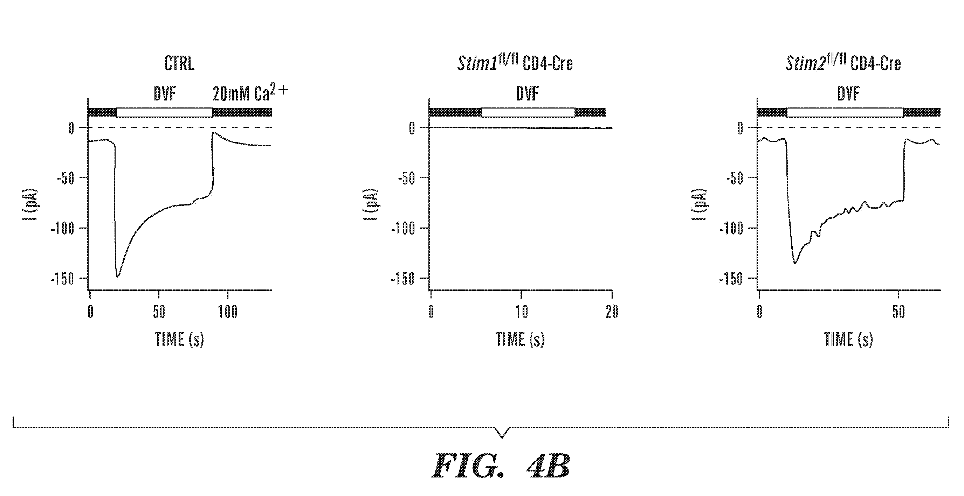

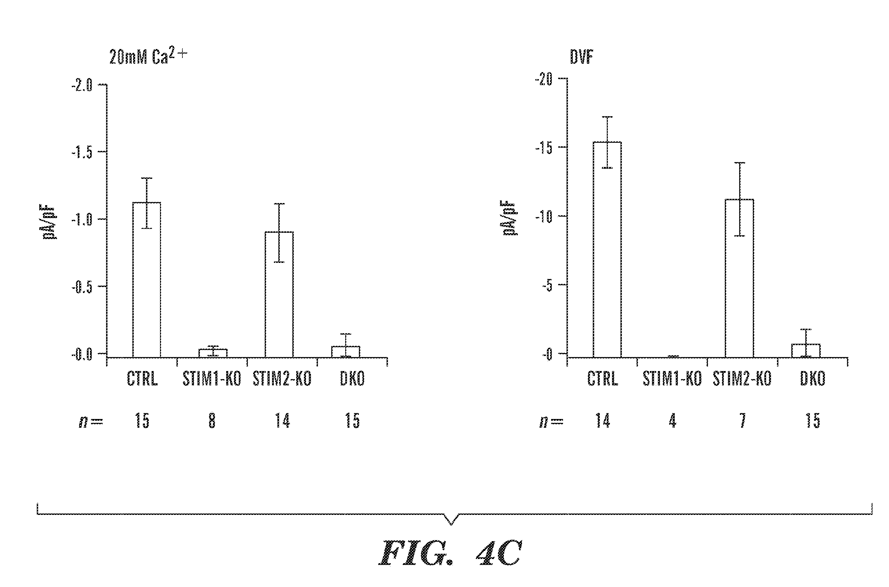

Whole cell patch clamp recordings were used to determine whether deletion of STIM1 and STIM2 affected the CRAC current ($I_{CRAC}$). In response to ER $Ca^{2+}$ store depletion by thapsigargin (TG), control mouse CD4+ T cells displayed $Ca^{2+}$ currents with properties similar to those of $I_{CRAC}$ in human T cells (Fig. 4). These properties included an inwardly-rectifying current-voltage relationship with a very positive reversal potential in the presence of 20 mM $Ca^{2+}$ (Fig. 4a), fast inactivation in 20 mM $Ca^{2+}$ (Fig. 12c), depotentiation of the $Na^+$ current in divalent cation-free (DVF) solutions (Fig. 4b), a low $Cs^+$ permeability (Permeability of $Cs^+$/Permability of $Na^+$=0.2$\pm$0.04; n=14 samples), blockade of the $Na^+$ current by micromolar concentrations of extracellular $Ca^{2+}$ (Fig. 12b), and potentiation and inhibition by low and high concentrations of 2-aminoehtoxydiphenyl borate (Prakriya, M. & Lewis, R.S., J. Physiol. 536, 3-19 (2001)) (although potentiation by 2-aminoehtoxydiphenyl borate in mouse cells did not appear as robust as in Jurkat or human T cells; Fig. 12d, and data not shown). Further, inclusion of 10 mM calcium-specific BAPTA in the patch pipette caused the slow development of an inward current in 20 mM $[Ca^{2+}]o$ following whole-cell break-in, reminiscent of the development of $I_{CRAC}$ in response to store depletion (data not shown). Collectively, these results indicated that mouse T cells have a $Ca^{2+}$ current with properties indistinguishable from those of $I_{CRAC}$.

Consistent with the $Ca^{2+}$ imaging results, STIM1-deficient T cells displayed no detectable $I_{CRAC}$ in either 20 mM $Ca^{2+}$ or divalent cation-free (DVF) media; in contrast, STIM2-deficient cells showed a slight decrease in the magnitude of $I_{CRAC}$, which did not, however, reach statistical significance with the number of cells examined (Fig. 4c). The properties of $I_{CRAC}$ were unaltered in STIM2-deficient T cells in terms of $Ca^{2+}$ and $Cs^+$ selectivity, fast inactivation, depotentiation, and responsiveness to high and low concentrations of 2-aminoehtoxydiphenyl borate (Fig. 12 and data not shown). These results indicate that endogenous STIM1 is required for $I_{CRAC}$ in primary CD4+ T cells, but endogenous STIM2 makes little or no contribution to the recorded $I_{CRAC}$ under the same conditions, possibly because it constitutes a very low fraction of total STIM protein even in differentiated T cells (Fig. 11b).

Example 5. Complex phenotype of double knockouts.

To analyze the consequences of combined deletion of *Stim1* and *Stim2* in T cells *in vivo*, *Stim1*fl/fl *Stim2*fl/fl CD4-Cre+ "double knockout" (abbreviated DKO) mice were generated. These mice showed no defect in conventional thymic development, as assessed by thymic cellularity and numbers and proportions of CD4- CD8- double-negative (DN), CD4+CD8+ double-positive (DP), and CD4+ and CD8+ single-positive (SP) cells (data not shown). Two possible explanations are that STIM proteins are long-lived, thus residual STIM1 and STIM2 protein can be present and functional well after gene deletion has occurred at the DP stage, or that thymocytes use STIM-independent $Ca^{2+}$ influx mechanisms that differ from those utilized by peripheral T cells. The roles of STIM proteins in T cell development and thymic selection are being explored as part of a separate study, using mice in which Cre expression is initiated early during T cell or haematopoietic cell development.

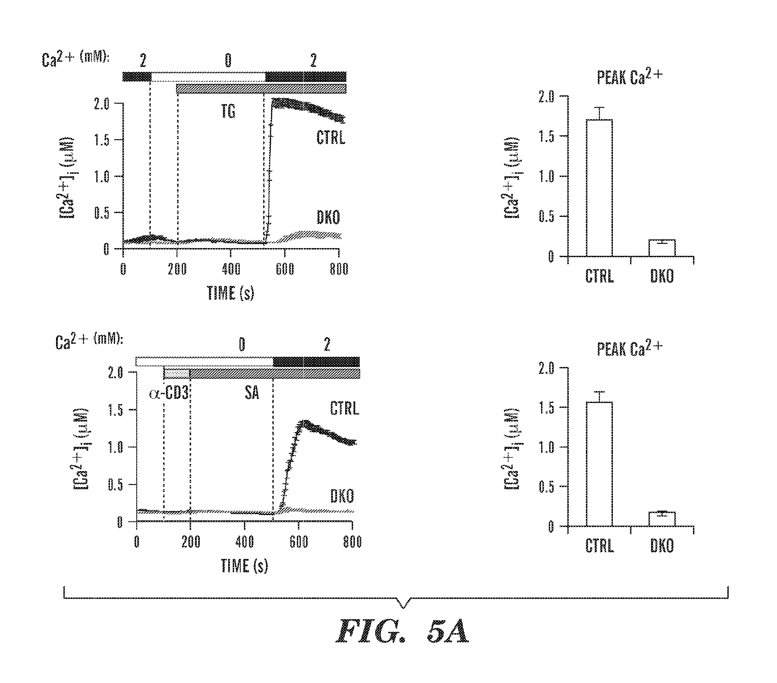

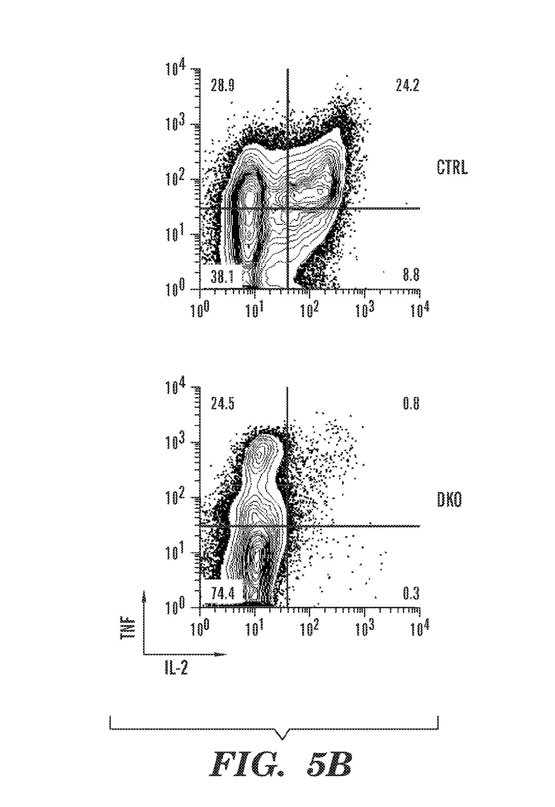

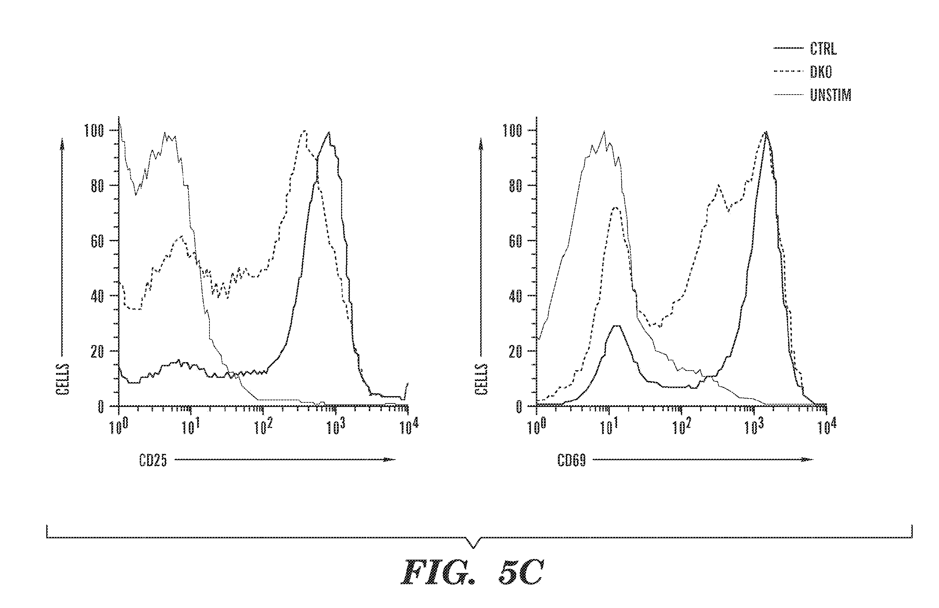

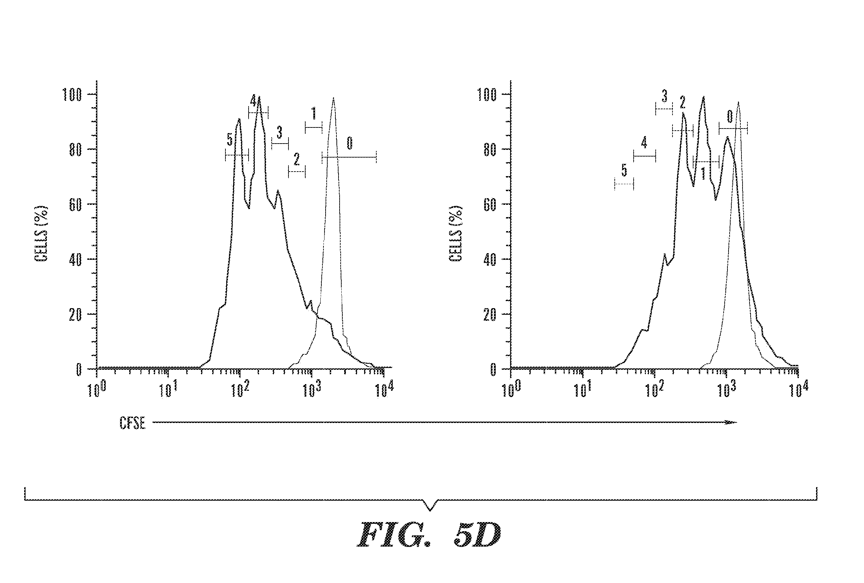

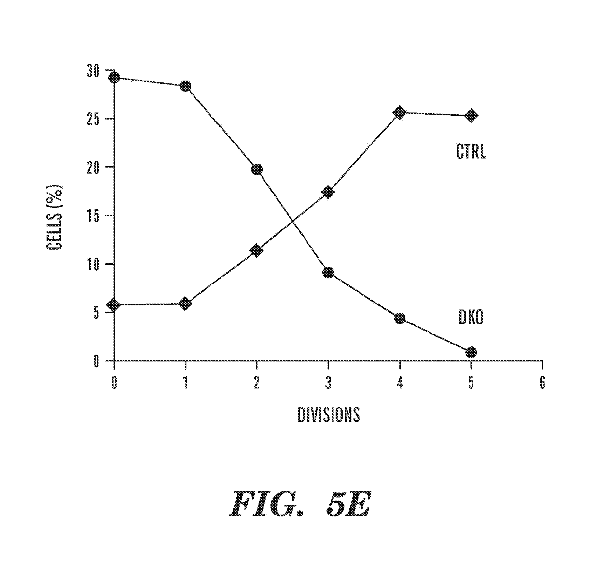

As expected from the severe deleterious phenotype of STIM1-deficient T cells, peripheral CD4+ T cells lacking both STIM proteins showed essentially no $Ca^{2+}$ influx in response to stimulation with thapsigargin or anti-CD3 (Fig. 5a, Fig. 13). The small amount of residual influx seen in the averaged curves of $Ca^{2+}$ influx and the small residual $I_{CRAC}$ stemmed from a small number of individual cells that displayed normal $Ca^{2+}$ influx (Fig. 5a) and $I_{CRAC}$ (data not shown). Of the 15 double-knockout cells examined, two had normal $I_{CRAC}$ in the recordin and the remaining cells has no $I_{CRAc}$. These represent either contaminating non-CD4+ cells that came through the purification procedure, or a small number of CD4+ T cells that escaped Cre-mediated deletion of STIM1 or STIM2 (see below, Fig. 15). DKO T cells produced almost no IL-2, although they did produce low amounts of tumor necrosis factor in response to primary stimulation (Fig. 5b), possibly due to PMA-induced NF-κB activation, which proceeded normally in the DKO cells (M.O., unpublished data). DKO T cells did upregulate expression of the activation markers CD69 and CD25 (Fig. 5c), and they underwent proliferation-albeit to a significantly reduced extent-after TCR stimulation (Fig. 5d, e).

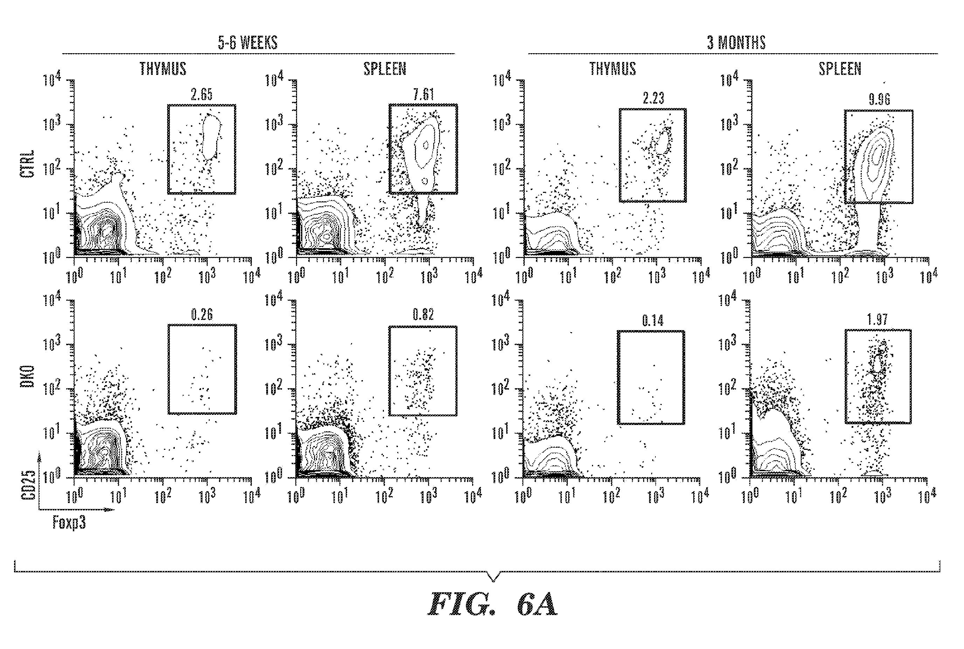

Unexpectedly, DKO mice older than 8 weeks of age developed a pronounced phenotype of splenomegaly, lymphadenopathy, dermatitis and blepharitis (Fig. 14a and data not shown). Histological analysis showed leukocyte infiltration into multiple organs including lung and liver (Fig. 6a and data not shown). Mice lacking STIM1 alone in CD4+ T cells displayed a milder version of the lymphoproliferative phenotype (Fig. 14a and data not shown). DKO mice also contained increased numbers of CD4+ T cells expressing a surface phenotype characteristic of memory or effector status (CD62L$^{lo}$CD44$^{hi}$CD69$^{hi}$CD45RB$^{lo}$CD5$^{hi}$), increased numbers of germinal centers (GC) in the spleen, increased numbers of B cells with a GC phenotype (CD95$^{hi}$CD38$^{lo}$) and increased numbers of differentiated IgE+ B cells, large numbers of CD11b+IL-5R+ eosinophils or basophils, and massively elevated serum concentrations of IgG1 (Fig. 14b-d, online and data not shown). CD4+ T cells from DKO mice produced IL-5 but not IL-4 in response to stimulation with PMA and ionomycin (data not shown). Notably, the phenotype of DKO mice is similar but not identical to those of mice with a mutation (Y136F) in the T cell transmembrane adapter LAT, which eliminates the docking site for PLC-γl and results in lowered $Ca^{2+}$ influx in response to TCR crosslinking (Sommers, C.L. et al., Science 296, 2040-2043 (2002); Aguado, E. et al., Science 296, 2036-2040 (2002)).

Example 6. STIM in Treg cell differentiation and function.

The autoreactive phenotype of LAT (Y136F) mutant mice was attributed to impaired negative selection allowing escape of autoreactive T cells into the periphery (Sommers, C.L. et al. , J Exp Med 201, 1125-1134 (2005)), and to a decrease in the number of $T_{reg}$ cells (Koonpaew, S., Shen, S., Flowers, L. & Zhang, W. LAT-mediated signaling in CD4+CD25+ regulatory T cell development. J Exp Med 203, 119-129 (2006)). Direct examination the role of STIM proteins in positive and negative selection of thymocytes, will require mice in which *Stim1* and *Stim2* are ablated at an earlier stage of T cell development using Lck-Cre, and breeding these mice with HY-TCR transgenic mice. Meanwhile, a clear reduction of $T_{reg}$ cell numbers in the thymus, spleen and lymph nodes of 5-6 week old *Stim1*$^{fl/fl}$ *Stim2*$^{fl/fl}$ CD4-Cre mice (Fig. 6c,d and data not shown) were documented. The proportion of $T_{reg}$ cells in spleen and lymph nodes of DKO mice increased with age, but nevertheless remained between 10-20% of the that in control mice (Fig. 6c); these increases were likely the result of age-dependent increases in the size of peripheral lymphoid organs. Mice lacking either STIM1 or STIM2 contained normal numbers of CD4+CD25+Foxp3+ $T_{reg}$ cells (data not shown). The number of cells expressing GITR, another marker of $T_{reg}$ cells, was also decreased in DKO mice (data not shown). Also noted was the complete deletion of *Stim1* and *Stim2* in CD25- and CD25+ T cells from older (8 week) DKO mice (Fig. 15a). Like CD4+CD25- T cells, CD4+CD25+ $T_{reg}$ cells from DKO mice showed impaired $Ca^{2+}$ influx in response to treatment with thapsigargin or anti-CD3 (Fig. 6e and Fig. 15b).

The marked decrease in the percentage and absolute numbers of $T_{reg}$ cells in DKO mice could reflect defective $T_{reg}$ cell development, survival in the periphery, or both. Given the fact that DKO T cells make very little IL-2, one possibility was that $T_{reg}$ cell development was defective in part due to lack of IL-2 produced by "bystander" T cells (Setoguchi, R., et. al., J Exp Med 201, 723-735 (2005); Fontenot, J.D.,et. al., Nat Immunol 6, 1142-1151 (2005); D'Cruz, L.M. & Klein, L. Nat Immunol 6, 1152-1159 (2005)). To address these possibilities, mixed bone marrow chimeras were generated. Sublethally irradiated Rag1$^{-/-}$ recipient mice deficient in recombination-activating gene 1 (*Rag1$^{-/-}$* mice) were reconstituted with T-cell depleted bone marrow from Thy1.2+ contol mice alone or with Thy1.2+ DKO mice or bone marrow from Thy1.2+ DKO mice or Thy1.1+ wild-type mice mixed at a 2:1 ratio. As expected, mice given only DKO bone marrow contained significantly fewer $T_{reg}$ cells in both thymus and lymph nodes compared to mice reconstituted with control bone marrow (Fig. 7) and developed the same severe lymphoproliferative phenotype as unmanipulated DKO mice (Fig. 16a). In contrast, mixed chimeras reconstituted with both DKO and wild-type bone marrow did not show any signs of lymphoproliferative disease and remained as healthy as control chimeric mice (Fig. 16a,). In these chimeric mice, the wild-type bone marrow gave rise to normal numbers of peripheral $T_{reg}$ cells, whereas the DKO precursors yielded far fewer $T_{reg}$ cells both in thymus and in the periphery (Fig. 7). Collectively, these results indicate that DKO mice have a cell-intrinsic defect in $T_{reg}$ cell development not restored by IL-2 produced by "bystander" T cells derived from the wild-type bone marrow (Setoguchi, R., et. al., J Exp Med 201, 723-735 (2005); Fontenot, J.D.,et. al., Nat Immunol 6, 1142-1151 (2005); D'Cruz, L.M. & Klein, L. Nat Immunol 6, 1152-1159 (2005)).

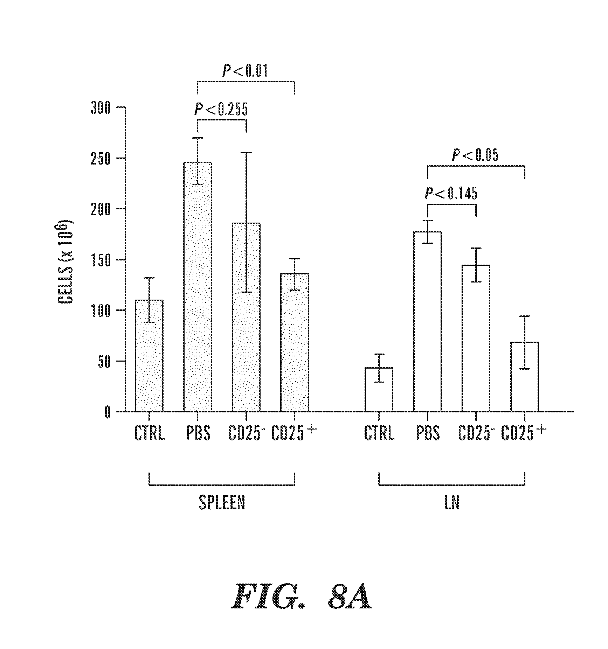

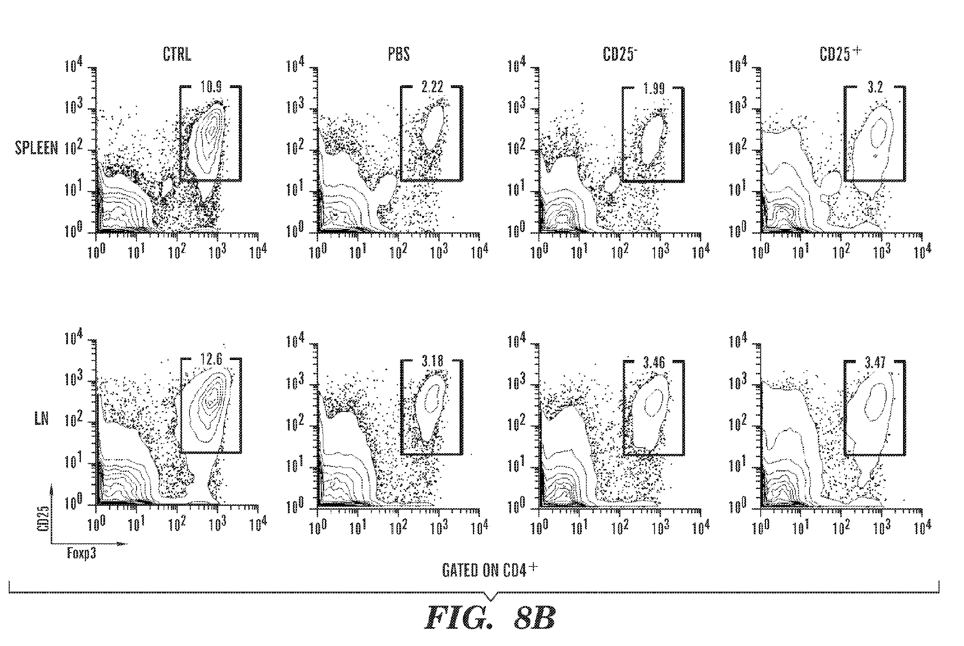

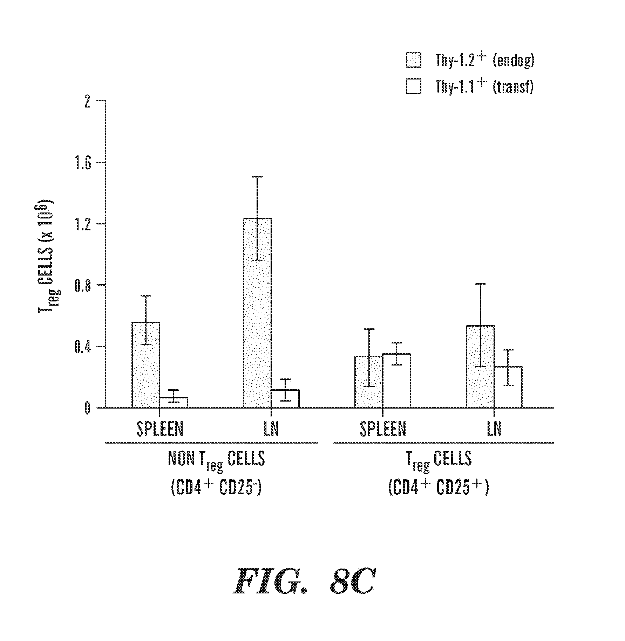

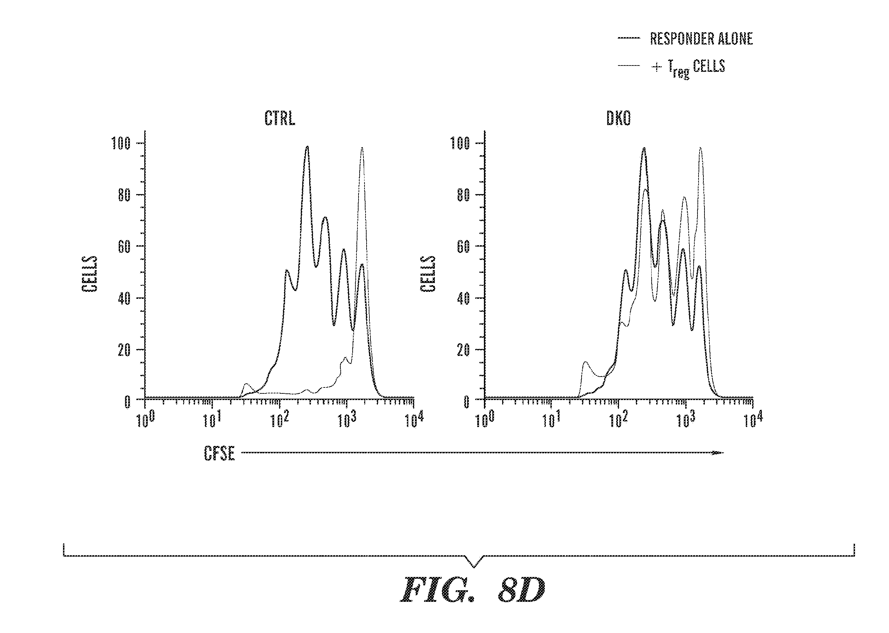

Next, the onset of the lymphoproliferative phenotype in DKO mice was prevent by injecting young DKO mice with $T_{reg}$ cells from wild-type mice. Injection of 2 week old DKO mice with wild-type $T_{reg}$ cells prevented the development of lymphoadenopathy and splenomegaly 8 weeks later, whereas injection with phosphate-buffered saline or with non-$T_{reg}$ cells did not (Fig. 8a and Fig. 16b). Because DKO mice contained very few endogenous $T_{reg}$ cells and only $3 \times 10^5$ wild-type CD4+CD25$^+$ T cells were transferred into each DKO mouse, the injected mice continued to contain very few $T_{reg}$ cells 8 weeks after injection (Fig. 8b). Thy1.1$^+$ $T_{reg}$ cells derived from the transferred wild-type Treg population were distinguished from endogenous Thy1.2$^+$ $T_{reg}$ cells by flow cytometry (Fig. 8b, c). As expected, endogenous Thy1.2$^+$ $T_{reg}$ cells accounted for the vast majority of CD4+CD25$^+$Foxp3$^+$ cells in DKO mice that were injected with wild-type CD4$^+$CD25$^-$ T cells, which displayed lymphadenopathy and splenomegaly at 10 weeks of age; in contrast, mice injected with wild-type CD4$^+$CD25$^+$ $T_{reg}$ cells and cured of the lymphoproliferative phenotype, contained a $T_{reg}$ cell population derived approximately equally from Thy1.1$^+$ donor and Thy1.2$^+$ endogenous cells (Fig. 8c). These data indicate that even though some $T_{re}$g cells develop in DKO mice, they function poorly (if at all) in comparison with $T_{reg}$ cells from wild-type mice. Moreover the numbers of endogenous $T_{reg}$ cells decreased in the presence of transferred wild-type $T_{reg}$ cells (Fig. 8c, compare left and right panels), suggesting that $T_{reg}$ cells from the DKO mice were at a competitive disadvantage in vivo. The confirmation of the defective function of the residual $T_{reg}$ cells in DKO mice was by performed in in vitro suppression assays (Fig. 8d and Fig. 17). Together the data indicate strongly that loss of both STIM1 and STIM2 impaired development and the function of Foxp3$^+$ regulatory T cells.

In summary, it has been established that both Stim1 and Stim2 are positive regulators of Ca$^{2+}$ signaling. T cells and embryonic fibroblasts lacking one of the two Stim proteins, but expressing the other Stim protein at physiological levels, exhibit major deficits in store-operated Ca$^{2+}$ entry and Ca$^{2+}$-dependent signaling; and these deficits can be partially or fully reconstituted by either Stim1 or Stim2. In T cells, however, the relative importance of Stim1 and Stim2 depends on the differentiation state. In naive T cells, Stim1 is the predominant effector of store-operated Ca$^{2+}$ entry and cytokine expression; in contrast, Stim2 is present only at low levels, and does not contribute to Ca$^{2+}$ signaling under the assay conditions described herein. In differentiated T cells, Stim1 remains essential for Ca$^{2+}$-dependent cytokine expression, but Stim2 is upregulated and complements Stim1 in promoting store-operated Ca$^{2+}$ entry and cytokine expression.

The prominent function of Stim2 in differentiated helper T cells is not simply a consequence of an increase in levels of either Stim2 or total Stim protein. Rather, Stim2 has an effect on overall Ca$^{2+}$ signaling that is disproportionate to its levels in differentiated helper T cells. In differentiated Stim1-/- differentiated helper T cells, Stim2 alone does not support store-operated Ca$^{2+}$ entry, and even after upregulation, Stim2 levels remain low relative to total Stim protein. In the NFAT pathway, the function of Stim2 is manifest in the sustained NFAT nuclear localization needed for effective cytokine production. In the NFκB pathway, its presence contributes to a Ca$^{2+}$-dependent component of p65 nuclear import that is prominent in the first hour of stimulation. The simplest explanation of these results is that at physiological levels in T cells, Stim2 enhances a signal in the Ca$^{2+}$ pathway initiated by activation of Stim1. The availability of conditionally Stim1-and Stim2-deficient mice will allow detailed evaluation of the role of these essential proteins in store-operated Ca$^{2+}$ entry in a variety of different cell types.

Table 1. STIM-deficient mouse is lethal. a, Viability of Stim1$^{fl/fl}$CMV-Cre mice on the C57BL/6 (B6) background at both embryonic and postnatal stages. b, Viability of Stim2$^{fl/fl}$CMV-Cre mice on the B6 background. c, Viability of Stim1$^{fl/fl}$CMV-Cre mice of mixed background after crossing to the outbred ICR mouse strain. A fraction of newborn STIM1-null mice did not survive past postnatal day 1; the numbers in parentheses are the numbers of mice that were alive upon visual inspection.

**a**

*Stim1* ꜰˡ/ꜰˡ CMV-Cre (B6 background)

| Day | Genotype | Total (alive) | | |
|---|---|---|---|---|
| | | +/+ | +/- | -/- |
| Embryonic | E14.5 | 16 | 18 | 8 |
| | E18.5 | 9 | 19 | 7 |
| | | | | |
| Postnatal | P0 | 43(42) | 83(79) | 37(9) |
| | P2 | 37 | 72 | 1 |
| | P14 | 37 | 72 | 0 |

**b**

*Stim2* ꜰˡ/ꜰˡ CMV-Cre (B6 background)

| Day | Genotype | Total (alive) | | |
|---|---|---|---|---|
| | | +/+ | +/- | -/- |
| Postnatal | P28 | 38 | 90 | 25 |
| | P35 | 38 | 90 | 1 |

**c**

*Stim1* ꜰˡ/ꜰˡ CMV-Cre (ICR mixed background)

| Day | Genotype | Total (alive) | | |
|---|---|---|---|---|
| | | +/+ | +/- | -/- |
| Postnatal | P0 | 19(18) | 49(48) | 13(8) |
| | P2 | 18 | 47 | 7 |
| | P14 | 18 | 47 | 0 |

The references cited herein and throughout the specification are incorporated herein by reference in their entirety.

## Claims

1. An isolated Stim-deficient cell, wherein the cell comprises a disruption of a STIM gene.

2. The cell of claim 1, wherein the cell is deficient in Stim1 protein, Stim2 protein, or both Stim1 and Stim2 proteins.

3. A method of screening for agents that modulate intracellular calcium flux comprising the use of the isolated Stim-deficient cell of claim 1.

4. The method of claim 3 further comprising: (a) contacting the cell with an agent; (b) assessing the effects of the agent on $Ca^{2+}$ influx in the cell; and (c) identifying the agent as an agent that modulates intracellular $Ca^+$ if the agent has an effect on the intracellular $Ca^{2+}$.

5. A method for evaluating the mode of action of an agent that modulates intracellular calcium flux comprising the use of the isolated Stim-deficient cell of claim 1.

6. The method of claim 5 further comprising: (a) contacting the Stim-deficient cell and a non Stim-deficient cell with an agent; (b) assessing the effects of the agent on $Ca^{2+}$ influx in the non Stim-deficient cell and Stim-deficient cell; (c) comparing the effects of the agent on the $Ca^+$ influx in the non Stim-deficient cell and Stim-deficient cell; and (d) determining the agent as an agent that modulates intracellular $Ca^{2+}$ via a Stim-dependent pathway if the agent reduces the intracellular $Ca^+$ in the non Stim-deficient cell as compared to the Stim-deficient cell.

7. The method of any of claims 4 - 6, wherein the agent is a biomolecule.

8. The method of claim 7, wherein the biomolecule is a nucleic acid or polypeptide.

9. A method for studying the cellular functions of a STIM protein or a mutant STIM protein in the absence of any other STIM homologues comprising the use of the isolated Stim-deficient cell of claim 1.

10. The method of claim 9, further comprising introducing a STIM protein or a mutant STIM protein into the cell.

11. The method of claim 10, wherein the STIM protein or mutant STIM protein is STIM1 or STIM2.

12. A method for studying the effects and/or efficacy of a STIM inhibitor or a STIM modulator comprising the use of the isolated Stim-deficient cell of claim 1.

13. The method of Claim 12, further comprising (a) contacting a STIM inhibitor with a STIM deficient cell; (b) assessing the $Ca^{2+}$ influx in said cell; and (c) determining that said STIM inhibitor is effective when there is a reduction in $Ca^{2+}$ influx in the treated cell.

14. A method for screening a STIM inhibitor for side effects or toxicity resulting from the inhibitor's action on a target(s) other than STIM comprising the use of a transgenic mouse whose genome comprises a heterozygous or homozygous disruption of a STIM gene.

**15.** The method of claim 14 further comprising (a) administering a STIM inhibitor to the transgenic mouse; and (b) monitoring the transgenic mouse to evaluate side effects or toxicity of the inhibitor.

*FIG. 1A*

*FIG. 1B*

*FIG. 1C*

FIG. 1D

*FIG. 1E*

EP 2 472 262 A2

FIG. 1F

EP 2 472 262 A2

FIG. 2A

*FIG. 2B*

FIG. 2C

FIG. 2C(cont'd)

FIG. 3A

*FIG. 3B*

*FIG. 3C*

*FIG. 3D*

FIG. 3E

EP 2 472 262 A2

**FIG. 4A**

EP 2 472 262 A2

FIG. 4B

EP 2 472 262 A2

## FIG. 4C

FIG. 5A

*FIG. 5B*

FIG. 5C

**FIG. 5D**

FIG. 5E

FIG. 6A

LUNG

*FIG. 6B*

*FIG. 6C*

FIG. 7A

FIG. 7B

FIG. 8A

FIG. 8B

EP 2 472 262 A2

*FIG. 8C*

FIG. 8D

*FIG. 9A*

*FIG. 9B*

FIG. 9C

EP 2 472 262 A2

FIG. 9D

RESTING

FIG. 10A

*FIG. 10B*

FIG. 10C

FIG. 10D

FIG. 10E

FIG. 11A

FIG. 11B

**FIG. 12A**

**FIG. 12B**

**FIG. 12C**

FIG. 12D

FIG. 13A

FIG. 13B

CTRL
STIM1-KO
STIM2-KO
DKO

SPLEEN

LN
CERVICAL
BRACHIAL
AXILLARY
INGUINAL

4 MONTHS

*FIG. 14A*

GATED ON CD4+ CELLS

*FIG. 14B*

FIG. 14C

FIG. 14D

FIG. 15A

FIG. 15B

FIG. 16B

FIG. 16A

FIG. 17

## REFERENCES CITED IN THE DESCRIPTION

### Patent documents cited in the description

- US 95902307 P
- US 4950598 A
- US 6103523 A
- US 6458593 B
- WO 2002072768 A
- US 20070031814 A
- US 6100445 A
- US 6060642 A
- US 6365796 B
- US 6747187 B
- US 7166764 B

### Non-patent literature cited in the description

- **CAROL, A.** *Frontiers in BioSciences,* 1997, vol. 2, 12-26
- **LI ; COHEN.** *Cell,* 1996, vol. 85, 319-329
- Computational Molecular Biology. Oxford University Press, 1988
- Biocomputing: Informatics and - 14 Genome Projects. Academic Press, 1993
- Computer Analysis of Sequence Data. Humana Press, 1994
- **HEINJE, G.** Sequence Analysis in Molecular Biology. Academic Press, 1987
- Sequence Analysis Primer, Gribskov. M Stockton Press, 1991
- **CARILLO, H. ; LIPMAN, D.** *SIAM J. Applied Math.,* 1988, vol. 48, 1073
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat'1. Acad. Sci. USA,* 1988, vol. 85, 2444
- Current Protocols in Molecular Biology. 1995
- **ALTSCHUL et al.** *Nuc. Acids Res.,* 1977, vol. 25, 3389-3402
- **ALTSCHUL et al.** *J. Mol. Biol.,* 1990, vol. 215, 403-410
- **HENIKOFF ; HENIKOFF.** *Proc. Natl. Acad. Sci. USA,* 1989, vol. 89, 10915
- The Merck Manual of Diagnosis and Therapy. Merck Research Laboratories, 2006
- The Encyclopedia of Molecular Biology. Blackwell Science Ltd, 1994
- Molecular Biology and Biotechnology: a Comprehensive Desk Reference. VCH Publishers, Inc, 1995
- **CROWTHER J. R.** *Methods in molecular biology,* 2000, 149
- **JEFFREY TRAVIS.** Fundamentals of RIA and Other Ligand Assays. Scientific Newsletters, 1979
- **WERNER LUTTMANN.** Immunology. Elsevier, 2006
- **BENJAMIN LEWIN.** Genes IX. Jones & Bartlett Publishing, 2007
- **MANIATIS et al.** Molecular Cloning: A Laboratory Manual. Cold Spring Harbor Laboratory Press, 1982
- **SAMBROOK et al.** Molecular Cloning: A Laboratory Manual. Cold Spring Harbor Laboratory Press, 1989
- **DAVIS et al.** Basic Methods in Molecular Biology. Elsevier Science Publishing, Inc, 1986
- Methods in Enzymology: Guide to Molecular Cloning Techniques. Academic Press Inc, 1987, vol. 152
- Current Protocols in Molecular Biology. John Wiley and Sons, Inc,
- Current Protocols in Protein Science. John Wiley and Sons, Inc,
- Current Protocols in Immunology. John Wiley and Sons, Inc,
- Current Protocols in Cell Biology. John Wiley and Sons, Inc,
- **R. IAN FRESHNEY.** Culture of Animal Cells: A Manual of Basic Technique. Wiley-Liss, 2005
- Animal Cell Culture Methods. Methods in Cell Biology. Academic Press, 1998, vol. 57
- **A CUSHING.** *J Neurosci Methods,* 1999, vol. 90, 33-6
- **S VERNINO.** *Journal of Neuroscience,* 1994, vol. 14, 5514-5524
- **LAJOS ; GERGELY.** *Clin. Diag. Lab. Immunol.,* 1997, vol. 4, 70-74
- **RANDRIAMAM-PITA ; TSIEN.** *Nature,* 1993, vol. 364, 809-814
- **PAREKH et al.** *Nature,* 1993, vol. 364, 814-818
- **CSUTORA et al.** *Proc. Natl. Acad. Sci. U.S.A.,* 1999, vol. 96, 121-126

- **WILLIAMS et al.** *Biochem. J.,* 2001, vol. 357, 673-685
- **HOGAN, B. ; BEDDINGTON, R. ; COSTANTINI, F. ; LACY, E.** Manipulating the Mouse Embryo: A Laboratory Manual. Cold Spring Harbor Laboratory, 1994
- **PORTER et al.** *Eur. J. Biochem.,* 1993, vol. 218, 273-281
- **BRADLEY, A.** Modifying the mammalian genome by gene targeting. *Current Opinion in Biotechnology,* 1991, vol. 2, 823-829
- **CAPECCHI, M.** The New Mouse Genetics: Altering the Genome by Gene Targeting. *Trends in Genetics,* 1989, vol. 5 (3), 70-76
- **HERTZOG PJ.** *Methods Mol Biol.,* 2001, vol. 158, 205-15
- **LINDA C. SAMUELSON ; JOSEPH M. METZGER.** *CSH Protocols,* 2006
- **WEISS et al.** *International Journal of Cancer,* 2001, vol. 92 (6), 877-882
- **PAREKH, A.B. ; PUTNEY, J.W.** *Physiol. Rev.,* 2005, vol. 85, 757-810
- **LEWIS, R.S.** *Annu. Rev. Immunol.,* 2001, vol. 19, 497-521
- **FESKE, S.** *Biochem. Biophys. Res. Commun.,* 2003, vol. 311, 1117-1132
- **GALLO, E.M.** *Nature Immunol.,* 2006, vol. 7, 25-32
- **ROOS, J. et al.** *J. Cell Biol.,* 2005, vol. 169, 435-445
- **LIOU, J. et al.** *Curr. Biol.,* 2005, vol. 15, 1235-1241
- **FESKE, S. et al.** *Nature,* 2006, vol. 441, 179-185
- **VIG, M. et al.** *Science,* 2006, vol. 312, 1220-1223
- **ZHANG, S.L. et al.** *Proc. Natl Acad. Sci. U S A,* vol. 103, 9357-9362
- **LIOU, J. et al.** *Curr. Biol,* 2005, vol. 15, 1235-1241
- **TAYLOR, C.W.** *Trends Biochem. Sci.,* 2006, vol. 31, 597-601
- **LEWIS, R.S.** *Nature,* 2007, vol. 446, 284-287
- **PUTNEY, J.W., JR.** *Cell Calcium,* 2007
- **ZHANG, S.L. et al.** *Nature,* 2005, vol. 437, 902-905
- **SPASSOVA, M.A. et al.** *Proc. Natl Acad. Sci. U S A,* 2006, vol. 103, 4040-4045
- **LUIK, R.M.** *J. Cell Biol.,* 2006, vol. 174, 815-825
- **WU, M.M.** *J. Cell Biol.,* 2006, vol. 174, 803-813
- **BABA, Y. et al.** *Proc. Natl Acad. Sci. U S A,* 2006, vol. 103, 16704-16709
- **SOBOLOFF, J. et al.** *Curr. Biol.,* 2006, vol. 16, 1465-1470
- **SOBOLOFF, J. et al.** *J. Biol. Chem.,* 2006, vol. 281, 20661-20665
- **MULJO, S.A. et al.** *J. Exp. Med.,* 2005, vol. 202, 261-269
- **SCHWENK, F.** *Nucleic Acids Res,* 1995, vol. 23, 5080-5081
- **RODRIGUEZ, C.I. et al.** *Nat Genet,* 2000, vol. 25, 139-140
- **LEE, P.P. et al.** *Immunity,* 2001, vol. 15, 763-774
- **ANSEL, K.M. et al.** *Nature Immunol.,* 2004, vol. 5, 1251-1259
- **WU, Y. et al.** *Cell,* 2006, vol. 126, 375-387
- **HO, A.M.** *J. Biol. Chem.,* 1994, vol. 269, 28181-28186
- **WILLIAMS, R.T. et al.** *Biochem. J.,* 2001, vol. 357, 673-685
- **SCHWENK, F. ; BARON, U. ; RAJEWSKY, K.** *Nucleic Acids Res,* 1995, vol. 23, 5080-5081
- **VORNDRAN, C.** *Biophys J,* 1995, vol. 69, 2112-2124
- **HOGAN, P.G.** *Genes Dev.,* 2003, vol. 17, 2205-2232
- **PRAKRIYA, M. ; LEWIS, R.S.** *J. Physiol.,* 2001, vol. 536, 3-19
- **SOMMERS, C.L. et al.** *Science,* 2002, vol. 296, 2040-2043
- **AGUADO, E. et al.** *Science,* 2002, vol. 296, 2036-2040
- **SOMMERS, C.L. et al.** *J Exp Med,* 2005, vol. 201, 1125-1134
- **KOONPAEW, S. ; SHEN, S. ; FLOWERS, L. ; ZHANG, W.** LAT-mediated signaling in CD4+CD25+ regulatory T cell development. *J Exp Med,* 2006, vol. 203, 119-129
- **SETOGUCHI, R.** *J Exp Med,* 2005, vol. 201, 723-735
- **FONTENOT, J.D.** *Nat Immunol,* 2005, vol. 6, 1142-1151
- **D'CRUZ, L.M. ; KLEIN, L.** *Nat Immunol,* 2005, vol. 6, 1152-1159

SEQUENCE LISTING

<110> IMMUNE DISEASE INSTITUTE, INC.

<120> STROMAL INTERACTING MOLECULE KNOCKOUT MOUSE AND USES
      THEREOF

<130> 033393-060111-PCT

<140>
<141>

<150> 60/959,023
<151> 2007-07-10

<160> 5

<170> PatentIn Ver. 3.3

<210> 1
<211> 30
<212> PRT
<213> Homo sapiens

<400> 1
Cys Asp Asn Gly Ser Ile Gly Glu Glu Thr Asp Ser Ser Pro Gly Arg
  1               5                  10                  15

Lys Lys Phe Pro Leu Lys Ile Phe Lys Lys Pro Leu Lys Lys
            20                  25                  30


<210> 2
<211> 15
<212> PRT
<213> Homo sapiens

<400> 2
Cys Lys Pro Ser Lys Ile Lys Ser Leu Phe Lys Lys Lys Ser Lys
  1               5                  10                  15



<210> 3
<211> 19
<212> RNA
<213> Artificial Sequence

<220>
<223> Description of Artificial Sequence: Synthetic
      oligonucleotide

<400> 3
agguggaggu gcaauauua                                              19


<210> 4
<211> 19
<212> RNA
<213> Artificial Sequence

<220>
<223> Description of Artificial Sequence: Synthetic
      oligonucleotide

```
<400> 4
uaaaccuccu ggaucauua                                          19


<210> 5
<211> 19
<212> RNA
<213> Artificial Sequence

<220>
<223> Description of Artificial Sequence: Synthetic
      oligonucleotide

<400> 5
cuuuaagccu cgagauaua                                          19
```